(19) Europäisches Patentamt
European Patent Office
Office européen des brevets

(11) Veröffentlichungsnummer: **0 258 183 B1**

(12) **EUROPÄISCHE PATENTSCHRIFT**

(45) Veröffentlichungstag der Patentschrift: **17.03.93**

(21) Anmeldenummer: **87810450.4**

(22) Anmeldetag: **07.08.87**

(51) Int. Cl.5: **C07C 227/00**, C07C 227/06, C07C 231/02, C07C 67/343, C07C 51/353, C07D 307/32, C07C 57/00, C07C 69/533, C07C 69/608, C07C 233/01, C07C 247/02

# (54) Verfahren zur Herstellung von 5-Amino-4-hydroxyvaleriansäure-Derivaten.

(30) Priorität: **13.08.86 CH 3248/86**

(43) Veröffentlichungstag der Anmeldung: **02.03.88 Patentblatt 88/09**

(45) Bekanntmachung des Hinweises auf die Patenterteilung: **17.03.93 Patentblatt 93/11**

(84) Benannte Vertragsstaaten: **AT BE CH DE ES FR GB IT LI NL SE**

(56) Entgegenhaltungen:
**EP-A- 0 143 746**
**EP-A- 0 173 481**
**EP-A- 0 184 550**
**EP-A- 0 206 090**
**EP-A- 0 270 234**
**DE-A- 1 914 380**
**US-A- 4 031 115**

(73) Patentinhaber: **CIBA-GEIGY AG**
**Klybeckstrasse 141**
**CH-4002 Basel(CH)**

(72) Erfinder: **Herold, Peter, Dr.**
**Eichenstrasse 53**
**CH-4054 Basel(CH)**
Erfinder: **Angst, Christof, Dr.**
**13 Lowell Avenue**
**Summit New Jersey(US)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

**JOURNAL OF ORGANIC CHEMISTRY, Band 50, 15. November 1985, Seiten 4615-4625, American Chemical Society; B.E. EVANS et al.: "A stereocontrolled synthesis of hydroxyethylene dipeptide isosteres using novel, chiral aminoalkyl epoxides and gamma-(aminoalkyl) gamma-lactones"**

**CHEMICAL SOCIETY REVIEWS, Band 8, 1979, Seiten 171-197, The Chemical Society, London, GB; M.D. DOWLE et al.: "Synthesis and synthetic utility of halolactones"**

**JOURNAL OF ORGANIC CHEMISTRY, Band 54, 2. März 1989, Seiten 1178-1185, American Chemical Society; P. HEROLD et al.: "A versatile and stereocontrolled synthesis of hydroxyethylene dipeptide isosteres"**

**Beschreibung**

Die Erfindung betrifft neue Verfahren zur Herstellung von 5-Amino-4-hydroxyvaleriansäure-Derivaten, die als Ausgangsstoffe zur Herstellung von anti-hypertensiv wirksamen Reninhemmern und anderer Pharmazeutika verwendet werden können. Neue Zwischenprodukte sind ebenfalls Gegenstand der Erfindung.

Die nach den erfindungsgemässen Verfahren herstellbaren 5-Amino-4-hydroxyvaleriansäure-Derivate sind Mimetika für eine Dipeptid-Einheit. Anstelle der zentralen Amidfunktion enthalten die Mimetika eine Hydroxyethylengruppe. Diese Mimetika können in Polypeptide oder Polypeptid-ähnliche Verbindungen eingebaut werden. Solchermassen abgewandelte Polypeptide oder Polypeptid-ähnliche Verbindungen weisen meist physiologische Eigenschaften auf, die denen von entsprechenden nicht abgewandelten Polypeptiden gleichen. Da aber eine zentrale Amidbindung durch eine Hydroxyethylengruppe ersetzt ist, sind sie gegen hydrolytische Spaltung an dieser zentralen Bindung stabil und erwerben damit zusätzliche, gegebenenfalls vorteilhafte Eigenschaften, wie längere Wirksamkeit in natürlicher physiologischer Umgebung dank Stabilität gegenüber Proteasen und/oder irreversible Bindung an Enzyme, die die zentrale Amidbindung spalten würden.

Die Herstellung von 5-Amino-4-hydroxyvaleriansäure-Derivaten ist bereits in der Europäischen Patentanmeldung 143 746 beschrieben. Gegenüber dem vorbekannten Verfahren bedeuten die neuen Verfahren eine wesentliche Verbesserung, können doch damit 5-Amino-4-hydroxyvaleriansäure-Derivate mit höherer Gesamtausbeute, höherer Stereoselektivität und weniger aufwendigen Reinigungsmethoden hergestellt werden.

Die Erfindung betrifft Verfahren zur Herstellung von Verbindungen der Formel

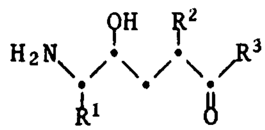 (I),

worin $R^1$ Wasserstoff, gegebenenfalls substituiertes Alkyl, Cycloalkyl, Cycloalkylniederalkyl, Aryl, Arylniederalkyl oder den Rest einer natürlichen Aminosäure, $R^2$ Wasserstoff, gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Hydroxy oder Amino darstellen, und von Salzen dieser Verbindungen, dadurch gekennzeichnet, dass man
einen Allylalkohol der Formel

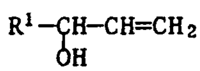 (II)

mit einer Säure oder Ester der Formel

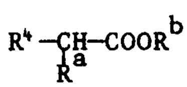 (III),

worin $R^a$ Wasserstoff oder eine Gruppe $COOR^b$ und $R^b$ Wasserstoff, einen Kohlenwasserstoffrest oder einen Silylrest bedeuten und $R^4$ die Bedeutungen von $R^2$ hat oder Halogen ist, oder mit einem Derivat davon in Gegenwart eines Katalysators verestert, die erhältliche Verbindung in situ und/oder nach Behandlung mit einer Base und einem Silylierungsmittel durch Erwärmen umlagert und gegebenenfalls decarboxyliert, die erhältliche Verbindung der Formel

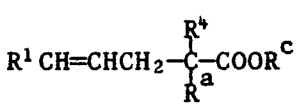 (IV),

worin $R^c$ Wasserstoff, einen Kohlenwasserstoffrest oder einen Silylrest bedeutet, falls $R^c$ verschieden von Wasserstoff ist und/oder $R^a$ eine Gruppe $COOR^b$ bedeutet und $R^b$ verschieden von Wasserstoff ist,

hydrolysiert und, falls $R^a$ eine Gruppe $COOR^b$ bedeutet, decarboxyliert und, falls $R^4$ Halogen bedeutet, Halogen durch Amino, Hydroxy oder Mercapto austauscht,
eine erhältliche Verbindung der Formel

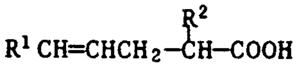

(V),

gewünschtenfalls nach Trennung der Enantiomeren und/oder Umwandlung der Carboxygruppe in eine Carboxamidfunktion oder in eine Hydroxamsäureestergruppe, mit einem Halogenierungsmittel halolactonisiert,
in einer erhältlichen Verbindung der Formel

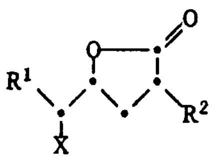

(VI),

worin X Halogen bedeutet, Halogen durch einen über Stickstoff gebundenen Rest austauscht und in beliebiger Reihenfolge den Lactonring mit einer den Rest $R^3$ einführenden Verbindung öffnet und den stickstoffhaltigen Rest X in eine Aminogruppe umwandelt und diese gegebenenfalls schützt,
und in einer erhältlichen Verbindung gegebenenfalls vorhandene Schutzgruppen abspaltet und/oder gewünschtenfalls eine erhältliche Verbindung der Formel I in ihr Salz überführt oder ein erhältliches Salz in die freie Verbindung überführt und/oder gewünschtenfalls erhältliche Isomerengemische auftrennt.

In der Beschreibung der vorliegenden Erfindung bedeutet der bei der Definition von Gruppen und Resten verwendete Ausdruck "Nieder", z.B. Niederalkyl, Niederalkoxy, Niederalkanoyl etc., dass die so bezeichneten Gruppen oder Reste, falls nicht ausdrücklich anders definiert, 1 bis 7 C-Atome und bevorzugt 1 bis 4 C-Atome enthalten.

Die durch vier verschiedene Reste substituierten C-Atome, beispielsweise die die Reste $R^1$, OH und $R^2$ tragenden C-Atome einer Verbindung der Formel I, das den Rest $R^1$ tragende C-Atom einer Verbindung der Formel II, die den Rest $R^2$ oder $R^4$ tragenden C-Atome von Verbindungen der Formel IV und V und die die Reste $R^1$, -OCO und $R^2$ tragenden C-Atome einer Verbindung der Formel VI können die R-, S-oder R,S-Konfiguration aufweisen.

Die in der Definition von Substituenten verwendeten allgemeinen Ausdrücke und Bezeichnungen haben vorzugsweise die folgenden Bedeutungen:

Alkyl $R^1$, $R^2$ oder $R^4$ hat vorzugsweise bis zu 12 C-Atome und bedeutet Niederalkyl mit 1 bis 7 C-Atomen oder z.B. n-Octyl, n-Nonyl, n-Decyl oder n-Dodecyl. Niederalkyl $R^1$, $R^2$ oder $R^4$ ist z.B. Methyl, Ethyl, n-Propyl, Isopropyl, n-Butyl, sec-Butyl, Isobutyl, n-Pentyl, Isopentyl oder n-Hexyl. Alkyl $R^1$, $R^2$ oder $R^4$ kann durch eine oder mehrere funktionelle Gruppen substituiert sein, beispielsweise durch Hydroxy, verethertes Hydroxy, z.B. Niederalkoxy, wie Methoxy oder Ethoxy, verestertes Hydroxy, z.B. Niederalkanoyloxy, wie Acetoxy, Halogen, z.B. Fluor oder Chlor, Carboxy, verestertes Carboxy, z.B. Niederalkoxycarbonyl, wie Methoxy- der Ethoxycarbonyl, amidiertes Carboxy, z.B. Carbamoyl oder Mono- oder Diniederalkylcarbamoyl, wie Mono- oder Dimethylcarbamoyl, Cyano, Amino, Mono-oder Diniederalkylamino, z.B. Methylamino oder Dimethylamino, cyclisches substituiertes Amino, z.B. Pyrrolidino, Piperidino oder Morpholino, oder Oxo.

Cycloalkyl $R^1$, $R^2$ oder $R^4$ ist mono-, bi- oder tricyclisch und ist gegebenenfalls durch eine oder mehrere der unter Alkyl $R^1$ genannten funktionellen Gruppen substituiert. Monocyclisches Cycloalkyl enthält z.B. 3 bis 8, insbesondere 5 bis 7, C-Atome und ist z.B. Cyclopropyl, Cyclopentyl, Cyclohexyl oder Cycloheptyl. Bicyclisches Cycloalkyl enthält z.B. 6 bis 10 C-Atome und ist. z.B. endo- oder exo-Norbornyl oder $\alpha$- oder $\beta$-Decahydronaphthyl. Tricyclisches Cycloalkyl enthält z.B. 8 bis 10 C-Atome und ist z.B. 1-Adamantyl.

Cycloalkylniederalkyl $R^1$, $R^2$ oder $R^4$ bedeutet einen wie oben unter Alkyl $R^1$ definierten Niederalkylrest, der eine oder mehrere mono-, bi- oder tricyclische Cycloalkylgruppen trägt und gegebenenfalls durch weitere der oben genannten Reste substituiert ist. Insbesondere enthält Cycloalkylniederalkyl 6 bis 10 C-Atome und ist beispielsweise Niederalkyl mit 1 bis 4 C-Atomen, das eine Cyclopentyl-, Cyclohexyl- oder

Cycloheptylgruppe trägt, z.B. Cyclopentylmethyl, Cyclohexylmethyl, 2-Cyclohexylethyl oder Cycloheptylmethyl.

Aryl $R^1$, $R^2$ oder $R^4$ enthält vorzugsweise 6 bis 14 C-Atome und ist beispielsweise Phenyl oder 1- oder 2-Naphthyl, unsubstituiert oder substituiert durch eine oder mehrere funktionelle Gruppen, beispielsweise durch Niederalkyl, z.B. Methyl, Hydroxy, verethertes Hydroxy, z.B. Niederalkoxy, wie Methoxy, verestertes Hydroxy, z.B. Niederalkanoyloxy, wie Acetoxy, Amino, Niederalkylamino, z.B. Methylamino, Diniederalkylamino, z.B. Dimethylamino, Acylamino, z.B. tert-Butoxycarbonylamino, oder Halo, z.B. Fluor, Chlor, Brom oder Iod, wobei der oder die Substituenten in irgendeiner Stellung des Arylrestes, z.B. in o-, m- oder p-Stellung des Phenylrestes, stehen können.

Arylniederalkyl $R^1$, $R^2$ oder $R^4$ enthält z.B. 7 bis 20 C-Atome und bedeutet z.B. einen wie oben unter Alkyl $R^1$ definierten Niederalkylrest, der einen oder mehrere der oben definierten Arylreste trägt und gegebenenfalls durch weitere der oben genannten funktionellen Gruppen substituiert ist. Beispielsweise enthält Arylniederalkyl 7 bis 11 C-Atome und ist z.B. Niederalkyl mit 1 bis 4 C-Atomen, das eine unsubstituierte oder durch Niederalkyl, Hydroxy, Niederalkoxy oder Halo substituierte Phenylgruppe oder eine $\alpha$- oder $\beta$-Naphthylgruppe trägt, z.B. Benzyl, 4-Methylbenzyl, 4-Hydroxybenzyl, 2-Phenylethyl oder $\alpha$- oder $\beta$-Naphthylmethyl.

Ein Rest einer natürlichen Aminosäure $R^1$, $R^2$ oder $R^4$ ist ein Rest, wie er in natürlichen $\alpha$-Aminosäuren mit dem $\alpha$-C-Atom verknüpft ist. Natürliche $\alpha$-Aminosäuren sind solche, die üblicherweise in Proteinen vorkommen, beispielsweise Glycin, Alanin, Valin, Norvalin, Leucin, Isoleucin, Norleucin, Serin, Homoserin, Threonin, Methionin, Cystein, Phenylalanin, Tyrosin, Tryptophan, Asparaginsäure, Asparagin, Glutaminsäure, Glutamin, Histidin, Arginin, Lysin, Ornithin, $\alpha,\gamma$-Diaminobuttersäure und $\alpha,\beta$-Diaminopropionsäure. Reste solcher Aminosäuren sind teilweise schon durch eine der weiter oben stehenden Definitionen umfasst sind insbesondere Wasserstoff, Methyl, Isopropyl, n-Propyl, Isobutyl, sec-Butyl, n-Butyl, Hydroxymethyl, 2-Hydroxyethyl, 1-Hydroxyethyl, 2-Methylthioethyl, Mercaptomethyl, Benzyl, 4-Hydroxybenzyl, 3-Indolylmethyl, Carboxymethyl, Carbamoylmethyl, 2-Carboxyethyl, 2-Carbamoylethyl, 4-Imidazolylmethyl, 3-Guanidinopropyl, 4-Aminobutyl, 2-Aminoethyl und Aminomethyl.

Amino $R^2$ oder $R^4$ ist gegebenenfalls substituiert, z.B. durch eine oder zwei Niederalkylgruppen, durch Arylniederalkyl, Niederalkanoyl, Niederalkoxycarbonyl oder durch Arylniederalkoxycarbonyl, oder ist Teil eines Ringes und ist beispielsweise Amino, Methylamino, Ethylamino, n-Butylamino, Dimethylamino, Benzylamino, Acetylamino, Pivaloylamino, Methoxy-, Ethoxy- oder tert-Butoxycarbonylamino, Benzyloxycarbonylamino, Pyrrolidino, Piperidino oder Morpholino.

Hydroxy $R^2$ oder $R^4$ ist gegebenenfalls substituiert, z.B. durch unsubstituiertes oder substituiertes Niederalkyl, z.B. Niederalkanoyloxyniederalkyl oder Niederalkoxycarbonyloxyniederalkyl, Aryl, Arylniederalkyl, Niederalkanoyl, Cycloalkylcarbonyl, Arylcarbonyl oder Silyl, z.B. Triniederalkylsilyl, und ist beispielsweise Hydroxy, Methoxy, Ethoxy, Acetoxymethoxy, tert-Butoxycarbonyloxymethoxy, Phenoxy, Benzyloxy, Acetoxy, Pivaloyloxy, Cyclohexylcarbonyloxy, Benzoyloxy, Trimethylsilyloxy oder tert-Butyldimethylsilyloxy.

Mercapto $R^2$ oder $R^4$ ist gegebenenfalls substituiert, z.B. durch unsubstituiertes oder substituiertes Niederalkyl, z.B. Hydroxyniederalkyl, Aryl, Arylniederalkyl oder Niederalkanoyl, und ist beispielsweise Mercapto, Methylthio, Ethylthio, Isopropylthio, tert-Butylthio, 2-Hydroxyethylthio, Phenylthio, Benzylthio oder Acetylthio.

Sulfinyl $R^2$ oder $R^4$ ist z.B. durch unsubstituiertes oder substituiertes Niederalkyl, z.B. Hydroxyniederalkyl, Aryl oder Arylnideralkyl substituiert und ist z.B. Methylsulfinyl, Ethylsulfinyl, Isopropylsulfinyl, tert-Butylsulfinyl, 2-Hydroxyethylsulfinyl, Phenylsulfinyl oder Benzylsulfinyl. Das Schwefelatom in Sulfinyl kann in R-, S-oder R,S-Konfiguration vorliegen.

Sulfonyl $R^2$ oder $R^4$ ist z.B. durch unsubstituiertes oder substituiertes Niederalkyl, z.B. Hydroxyniederalkyl, Aryl oder Arylniederalkyl substituiert und ist z.B. Methylsulfonyl, Ethylsulfonyl, Isopropylsulfonyl, tert-Butylsulfonyl, 2-Hydroxyethylsulfonyl, Phenylsulfonyl oder Benzylsulfonyl.

Gegebenenfalls substituiertes Hydroxy $R^3$ ist unsubstituiert oder substituiert durch einen unsubstituierten oder substituierten, gesättigten oder ungesättigten aliphatischen, aromatischen, heteroaromatischen, aromatisch-aliphatischen oder heteroaromatisch-aliphatischen Kohlenwasserstoffrest mit bis zu 18, bevorzugt mit bis zu 10 C-Atomen.

Gegebenenfalls substituiertes Amino $R^3$ ist unsubstituiert oder substituiert durch einen oder zwei unsubstituierte oder substituierte, gesättigte oder ungesättigte aliphatische, aromatische, heteroaromatische, aromatisch-aliphatische oder heteroaromatisch-aliphatische Kohlenwasserstoffreste mit bis zu 18, bevorzugt mit bis zu 10 C-Atomen, oder Teil eines stickstoffhaltigen Ringes.

Ein unsubstituierter oder substituierter, gesättigter oder ungesättigter aliphatischer Kohlenwasserstoffrest ist beispielsweise gegebenenfalls substituiertes Alkyl mit bis zu 12 C-Atomen, mono-, bi- oder tricyclisches Cycloalkyl oder Cycloalkylniederalkyl, wie oben für die Reste $R^1$, $R^2$ und $R^4$ definiert, ferner

Niederalkenyl mit 2 bis 7 C-Atomen oder Niederalkinyl mit 2 bis 7 C-Atomen, wobei diese Reste durch die unter Alkyl $R^1$ genannten funktionellen Gruppen substituiert sein können, beispielsweise Vinyl, Allyl, 2- oder 3-Butenyl, 3-Carboxy-2-propenyl oder 2-Propinyl.

Ein unsubstituierter oder substituierter aromatischer oder aromatisch-aliphatischerKohlenwasserstoffrest ist beispielsweise gegebenenfalls substituiertes Aryl mit 6 bis 14 C-Atomen oder Arylniederalkyl mit 7 bis 20 C-Atomen, wie oben für die Reste $R^1$, $R^2$ und $R^4$ definiert.

Ein unsubstituierter oder substituierter heteroaromatischer Kohlenwasserstoffrest ist beispielsweise ein mono-, bi- oder tricyclischer Heterocyclus enthaltend ein bis zwei Stickstoffatome und/oder ein Sauerstoff- oder Schwefelatom, z.B. Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl oder $\beta$-Carbolinyl. Dieser Heterocyclus kann an einem Stickstoffatom durch Niederalkyl, z.B. Methyl, Phenyl, Phenylniederalkyl, z.B. Benzyl, und/oder an einem oder mehreren Kohlenstoffatomen durch Niederalkyl, z.B. Methyl, Phenyl, Halogen, z.B. Chlor, Hydroxy, Niederalkoxy, z.B. Methoxy, Phenylniederalkoxy, z.B. Benzyloxy, oder Oxo substituiert und teilweise gesättigt sein und ist beispielsweise 2- oder 3-Pyrrolyl, 5-Phenyl-2-pyrrolyl, 2-Furyl, 2-Thienyl, 4-Imidazolyl, 2-, 3- oder 4-Pyridyl, 2-, 3- oder 5-Indolyl, 1-Methyl-, 5-Methyl-, 5-Methoxy- oder 5-Chlor-2-indolyl, 1-Benzyl-2- oder 3-indolyl, 2-, 3- oder 4-Chinolyl, 4-Hydroxy-2-chinolyl, 1-Oxo-1,2-dihydro-3-isochinolyl oder 2-Chinoxalinyl.

Ein unsubstituierter oder substituierter heteroaromatisch-aliphatischer Kohlenwasserstoffrest enthält beispielsweise einen wie oben unter Alkyl $R^1$ definierten Niederalkylrest, der einen oder zwei der oben definierten Heterocyclen trägt und gegebenenfalls am Niederalkylrest durch weitere der oben genannten funktionellen Gruppen substituiert ist, beispielsweise Niederalkyl mit 1 bis 4 C-Atomen, das einen mono- oder bicyclischen Heterocyclus mit einem oder zwei Stickstoffatomen trägt, z.B. 2- oder 3-Pyrrolylmethyl, 2-, 3- oder 4-Pyridylmethyl, 2-(2-, 3- oder 4-Pyridyl)-ethyl, 4-Imidazolylmethyl, 2-(4-Imidazolyl)-ethyl, 2- oder 3-Indolylmethyl, 2-(3-Indolyl)-ethyl oder 2-Chinolylmethyl.

Substituiertes Amino $R^3$ als Teil eines stickstoffhaltigen Ringes ist beispielsweise Teil eines fünf-, sechs- oder siebengliedrigen Ringes, der neben C-Atomen gegebenenfalls ein weiteres unsubstituiertes oder substituiertes Stickstoffatom, ein Sauerstoffatom oder ein Schwefelatom enthält. Beispiele für Substituenten dieses weiteren Stickstoffatoms sind Niederalkyl, z.B. Methyl, Phenyl, Phenylniederalkyl, z.B. Benzyl, Acyl, z.B. Niederalkanoyl, wie Acetyl oder Pivaloyl, Niederalkoxycarbonyl, wie tert-Butyloxycarbonyl, oder Phenylniederalkoxycarbonyl, wie Benzyloxycarbonyl. Solchermassen substituiertes Amino $R^3$ ist beispielsweise Pyrrolidino, Piperidino, Hexamethylenamino, Morpholino, Thiomorpholino, 4-Methyl-1-piperazinyl oder 4-Acetyl-1-piperazinyl.

Halogen $R^4$ ist Fluor, Chlor, Brom oder Jod, vorzugsweise Chlor oder Brom.

Die in den Substituenten $R^1$, $R^2$, $R^3$ oder $R^4$ vorhandenen funktionellen Gruppen, beispielsweise Carboxy, Amino, Hydroxy oder Mercapto, können statt in freier auch in geschützter Form vorliegen. Geeignete Schutzgruppen und ihre Einführung und Abspaltung sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist. Beispiele für eine geschützte Carboxygruppe sind tert-Niederalkoxycarbonyl, z.B. tert-Butoxycarbonyl, Arylmethoxycarbonyl mit einem oder zwei Arylresten, z.B. Benzyloxycarbonyl, 4-Nitrobenzyloxycarbonyl oder Diphenylmethoxycarbonyl, 1-Niederalkoxyniederalkoxycarbonyl, z.B. Methoxymethoxycarbonyl, 1-Methoxyethoxycarbonyl oder 1-Ethoxyethoxycarbonyl, Aroylmethoxycarbonyl, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, sowie 2-Triniederalkylsilylniederalkoxycarbonyl, z.B. 2-Trimethylsilylethoxycarbonyl.

Eine Aminogruppe kann z.B. in Form einer Acylamino- oder Arylmethylaminogruppe geschützt sein. In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure, einer gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Iod-, 2,2,2-Trifluor- oder 2,2,2-Trichloracetyl, gegebenenfalls z.B.

durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls z.B. durch Niederalkyl, z.B. tert-Niederalkyl, wie tert-Butyl, Niederalkoxy, wie Methoxy, Hydroxy, Halogen, wie Chlor, und/oder Nitro mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, 2-Triniederalkylsilylniederalkoxycarbonyl, z.B. 2-Trimethylsilylethoxycarbonyl, oder 2-Triarylsilylniederalkoxycarbonyl, z.B. 2-Triphenylsilylethoxycarbonyl. Eine Arylmethylaminogruppe ist z.B. Mono-, Di- oder insbesondere Triphenylmethylamino, z.B. Benzyl-, Diphenylmethyl- oder Tritylamino.

Eine Hydroxygruppe kann beispielsweise durch eine Acylgruppe, z.B. durch Halogen, z.B. Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl oder Dimethyl-tert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyloder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, z.B. 2-Tetrahydrofuryl oder 2-Tetrahydropyranyl oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Eine Mercaptogruppe kann beispielsweise durch eine gegebenenfalls substituierte Alkylgruppe, eine Acylgruppe, eine Silylgruppe, als Thioacetal oder als Disulfid geschützt sein. Bevorzugte Schutzgruppen für Mercapto sind gegebenenfalls im Phenylrest, z.B. durch Methoxy oder Nitro, substituiertes 1-Phenylniederalkyl, beispielsweise Benzyl, 4-Methoxybenzyl, 2-Nitrobenzyl, Bis(4-methoxyphenyl)methyl oder Trityl, die für Aminogruppen genannten Acylreste eines Kohlensäurehalbesters, z.B. Benzyloxycarbonyl, ferner Niederalkylaminocarbonyl, z.B. Ethylaminocarbonyl, Triniederalkylsilyl, z.B. Trimethylsilyl, Benzylthiomethyl, Tetrahydropyranyl oder Niederalkylthio, z.B. Methylthio, Ethylthio oder Benzylthio.

Ein Kohlenwasserstoffrest $R^b$ oder $R^c$ ist beispielsweise ein unter Substituenten von Hydroxy oder Amino $R^3$ genannter Kohlenwasserstoffrest, insbesondere ein Niederalkylrest mit 1 bis 7 C-Atomen, z.B. Methyl, Ethyl oder tert-Butyl, oder ein Arylniederalkylrest mit 7 bis 15 C-Atomen, z.B. Benzyl.

Ein Silylrest $R^b$ oder $R^c$ ist durch drei gleiche oder verschiedene Kohlenwasserstoffreste substituiert, wie sie als Substituenten von Hydroxy oder Amino $R^3$ oben genannt sind. Ein solcher Kohlenwasserstoffrest ist beispielsweise Niederalkyl mit 1 bis 7 C-Atomen, z.B. Methyl, Ethyl, Isopropyl, tert-Butyl oder tert-Hexyl, monocyclisches Cycloalkyl mit 5 bis 7 C-Atomen, z.B. Cyclohexyl, Aryl mit 6 bis 14 C-Atomen, z.B. Phenyl, oder Arylniederalkyl mit 7 bis 20 C-Atomen, z.B. Benzyl oder Trityl. Ein Silylrest $R^b$ oder $R^c$ ist beispielsweise Trimethylsilyl, Triethylsilyl, Tripropylsilyl, Triisopropylsilyl, Tributylsilyl, Triphenylsilyl, Dimethyl-tertbutylsilyl, Dimethylisopropylsilyl, Dimethylphenylsilyl, Diphenylmethylsilyl, Diphenylisopropylsilyl, Diphenyl-tert-butylsilyl oder Dimethyltritylsilyl.

Salze von Verbindungen der Formel I sind beispielsweise Säureadditionssalze der Aminogruppe, z.B. mit anorganischen Säuren, z.B. Salzsäure, Schwefelsäure, Salpetersäure oder Phosphorsäure, oder mit organischen Carbon- oder Sulfonsäuren, z.B. mit Essigsäure, Chloressigsäure, Trichloressigsäure, Trifluoressigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, mit Methansulfonsäure, Trifluormethansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren Gruppen, z.B. Carboxy, können auch innere Salze bilden.

Verbindungen der Formel I, in denen $R^3$ Hydroxy bedeutet, können auch Alkalimetall-, z.B. Natrium- oder Kaliumsalze bilden, ferner Erdalkalimetallsalze, z.B. Magnesium- oder Calciumsalze, Zinksalze, Ammoniumsalze, Salze mit organischen Aminen, z.B. mit gegebenenfalls substituierten Mono-, Di- oder Trialkylaminen, z.B. mit Cyclohexylamin, Diethylamin, Cyclohexylethylamin, Dibutylamin, Trimethylamin, Triethylamin oder Tri-(2-hydroxyethyl)-amin, oder mit tetrasubstituierten organischen Ammoniumionen, z.B. mit Tetramethylammonium, Tetraethylammonium oder Tetrabutylammonium.

Verbindungen der Formel I, in denen $R^3$ Hydroxy bedeutet, können auch innere Salze bilden.

Die Verbindungen der vorliegenden Erfindung können auf bekannte Art und Weise in Polypeptidähnliche Verbindungen mit nützlichen physiologischen Eigenschaften umgewandelt werden. Beispielsweise können sie in der in den Europäischen Patentanmeldung 143 746 und 184 550 beschriebenen Art und Weise mit Carbonsäuren oder Carbonsäure-Derivaten kondensiert werden und dabei blutdrucksenkende Reninhemmer ergeben.

Renin gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Antiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Renin-Hemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Die aus den Verbindungen der vorliegenden Erfindung herstellbaren Reninhemmer zeigen in den in vitro-Systemen Hemmwirkungen schon bei Konzentrationen von etwa $10^{-6}$ bis etwa $10^{-9}$ Mol/l.

Die Verbindungen der vorliegenden Erfindung können auch auf bekannte Art und Weise in Polypeptidähnliche Verbindungen mit analgetischer Wirkung umgewandelt werden, beispielsweise in Verbindungen, die die Enkephalin-abbauende Aminopeptidase hemmen.

Die Erfindung betrifft insbesondere Verfahren zur Herstellung von Verbindungen der Formel I, bevorzugt in diastereomerenreiner Form, worin $R^1$ Niederalkyl, Hydroxyniederalkyl, Cycloalkyl, Cycloalkylniederalkyl, Aryl oder Arylniederalkyl, $R^2$ Niederalkyl, Hydroxyniederalkyl, Cycloalkyl, Cycloalkylniederalkyl, Aryl, Arylniederalkyl, gegebenenfalls substituiertes Amino, gegebenenfalls substituiertes Hydroxy, gegebenenfalls substituiertes Mercapto, substituiertes Sulfinyl oder substituiertes Sulfonyl und $R^3$ gegebenenfalls substituiertes Amino darstellen, ferner von Salzen dieser Verbindungen.

Hauptsächlich betrifft die Erfindung Verfahren zur Herstellung von Verbindungen der Formel I, bevorzugt in diastereomerenreiner Form, worin $R^1$ Niederalkyl, z.B. Isopropyl oder Isobutyl, Cycloalkyl, z.B. Cyclohexyl, Cycloalkylniederalkyl, z.B. Cyclopentylmethyl, Cyclohexylmethyl, 2-Cyclohexylethyl oder Cycloheptylmethyl, Phenyl oder Phenylniederalkyl, z.B. Benzyl, $R^2$ Niederalkyl, z.B. Methyl, Ethyl, n-Propyl, Isopropyl, n-Butyl, sec-Butyl oder Isobutyl, Hydroxyniederalkyl, z.B. Hydroxymethyl oder 2-Hydroxyethyl, Cycloalkyl, z.B. Cyclopentyl oder Cyclohexyl, Cycloalkylniederalkyl, z.B. Cyclohexylmethyl, Phenyl, Phenylniederalkyl, z.B. Benzyl, Amino, Niederalkylamino, z.B. Methylamino oder Ethylamino, Diniederalkylamino, z.B. Dimethylamino, Niederalkanoylamino, z.B. Acetylamino oder Pivaloylamino, substituiertes Amino als Teil eines Ringes, z.B. Pyrrolidino, Piperidino oder Morpholino, Hydroxy, Niederalkoxy, z.B. Methoxy oder Ethoxy, Niederalkanoyloxy, z.B. Acetoxy, Mercapto, Niederalkylthio, z.B. Methylthio, Ethylthio oder tert-Butylthio, oder Niederalkylsulfonyl, z.B. Methylsulfonyl, Ethylsulfonyl oder tert-Butylsulfonyl, und $R^3$ substituiertes Amino, worin der Substituent gegebenenfalls substituiertes Alkyl mit bis zu 12 C-Atomen, beispielsweise Niederalkyl, z.B. Methyl, Ethyl, n-Butyl oder n-Pentyl, n-Alkyl, z.B. n-Octyl oder n-Decyl, Hydroxyniederalkyl, z.B. 2-Hydroxyethyl, Carboxyniederalkyl, z.B. Carboxymethyl, 1-Carboxyethyl, 1-Carboxyisobutyl oder 3-Carboxypropyl, gegebenenfalls substituiertes Carbamoylniederalkyl, z.B. Carbamoylmethyl, 1-Carbamoylethyl, 1-Carbamoylisobutyl oder 1-Carboxyniederalkyl, worin die Carboxygruppe durch die Aminofunktion einer natürlichen Aminosäure, deren Niederalkylester oder deren Amid amidiert ist, z.B. 1-(Histidinylcarbonyl)-2-methyl-butyl, 1-(Methoxyhistidinylcarbonyl)-2-methyl-butyl oder 1-(Amidohistidinylcarbonyl)-2-methyl-butyl, Cycloalkyl, z.B. Cyclohexyl, Cycloalkylniederalkyl, z.B. Cyclopropylmethyl oder Cyclohexylmethyl, Niederalkenyl, z.B. Allyl, Niederalkinyl, z.B. 2-Propinyl, Aryl, z.B. Phenyl, Arylniederalkyl, z.B. Benzyl, Heteroaryl, z.B. 2-Pyridyl oder 3-Indolyl, oder Heteroarylniederalkyl enthaltend einen mono- oder bicyclischen Heterocyclus mit einem oder zwei Stickstoffatomen, z.B. 2- oder 4-Pyridylmethyl, 2-(2- oder 4-Pyridyl)-ethyl, 4-Imidazolylmethyl, 2-(4-Imidazolyl)-ethyl, 3-Indolylmethyl oder 2-(3-Indolyl)-ethyl, bedeutet, ferner Diniederalkylamino, z.B. Dimethylamino oder Diethylamino, oder substituiertes Amino als Teil eines Ringes, z.B. Pyrrolidino, Piperidino oder Morpholino, darstellen, ferner von Salzen von solchen Verbindungen.

Ganz besonders betrifft die Erfindung Verfahren zur Herstellung von Verbindungen der Formel I, worin $R^1$ Niederalkyl, z.B. Isopropyl oder Isobutyl, Cycloalkylniederalkyl, z.B. Cyclopentylmethyl, Cyclohexylmethyl, 2-Cyclohexylethyl oder Cycloheptylmethyl, oder Phenylniederalkyl, z.B. Benzyl, $R^2$ Niederalkyl, z.B. Methyl, Ethyl, Isopropyl oder sec-Butyl, Cycloalkyl, z.B. Cyclopentyl oder Cyclohexyl, Phenyl, Diniederalkylamino, z.B. Dimethylamino, substituiertes Amino als Teil eines fünf- oder sechsgliederigen Ringes, z.B. Pyrrolidino, Piperidino oder Morpholino, Niederalkoxy, z.B. Methoxy oder Ethoxy, Niederalkylthio, z.B. Methylthio oder Ethylthio, oder Niederalkylsulfonyl, z.B. Methylsulfonyl oder Ethylsulfonyl, und $R^3$ Niederalkylamino, z.B. Methyl-, Ethyl-, n-Butyl- oder n-Pentylamino, Hydroxyniederalkylamino, z.B. 2-Hydroxyethylamino, Carboxyniederalkylamino, z.B. Carboxymethyl-, 1-Carboxyethyl-, 1-Carboxyisobutyl-oder 3-Carboxypropylamino, Carbamoylniederalkylamino, z.B. Carbamoylmethyl-, 1-Carbamoylethyl- oder 1-Carbamoylisobutylamino, Cycloalkylniederalkylamino, z.B. Cyclopropylmethylamino, Arylniederalkylamino, z.B. Benzylamino, Heteroarylniederalkylamino enthaltend einen mono- oder bicyclischen Heterocyclus mit einem oder zwei Stickstoffatomen, z.B. 2- oder 4-Pyridylmethylamino, 2-(2- oder 4-Pyridyl)-ethylamino, 4-Imidazolylmethylamino, 2-(4-Imidazolyl)-ethylamino, 3-Indolylmethylamino oder 2-(3-Indolyl)-ethylamino, Diniederalkylamino, z.B. Dimethylamino oder Diethylamino, oder substituiertes Amino als Teil eines fünf- oder sechsgliedrigen Ringes, z.B. Pyrrolidino, Piperidino oder Morpholino, bedeuten und die die Reste $R^1$ und OH tragenden C-Atome beide die R-Konfiguration oder beide die S-Konfiguration, bevorzugt die S-Konfiguration, und das den Rest $R^2$ tragende C-Atom die R- oder S-Konfiguration aufweisen, ferner von Salzen von solchen Verbindungen.

In erster Linie betrifft die Erfindung Verfahren zur Herstellung von Verbindungen der Formel I, worin $R^1$ Niederalkyl, z.B. Isobutyl, oder Cycloalkylniederalkyl, z.B. Cyclopentylmethyl, Cyclohexylmethyl, 2-Cyclohexylethyl oder Cycloheptylmethyl, $R^2$ Niederalkyl, z.B. Isopropyl, Diniederalkylamino, z.B. Dimethylamino, oder Niederalkoxy, z.B. Methoxy, und $R^3$ Niederalkylamino, z.B. Methylamino oder n-Butylamino, darstellen und die die Reste $R^1$ und OH tragenden C-Atome die S-Konfiguration und das den Rest $R^2$ tragende C-Atom die R- oder S-Konfiguration aufweist, ferner von Salzen von solchen Verbindungen.

Zuallererst betrifft die Erfindung ein Verfahren zur Herstellung der in den Beispielen genannten Verbindungen.

Verfahren

Umwandlung einer Verbindung der Formel II in eine Verbindung der Formel V:

Die Veresterung eines Allylalkohols der Formel II mit einer Säure der Formel III, worin $R^b$ Wasserstoff bedeutet, zu einem Allylester der Formel

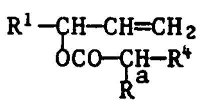

(VII),

worin $R^1$, $R^4$ und $R^a$ die genannten Bedeutungen haben, kann nach einer der üblichen Veresterungsmethoden erfolgen, beispielsweise in Gegenwart eines sauren Katalysators, z.B. einer Protonen-haltigen Säure, beispielsweise Schwefelsäure, Chlor- oder Bromwasserstoff, Phosphorsäure oder einer starken organischen Säure, wie Arylsulfonsäure, z.B. p-Toluolsulfonsäure, oder Alkylsulfonsäure, z.B. Methan-oder Trifluormethansulfonsäure, eines Proton-freien Lewissäure-Katalysators, beispielsweise Bortrifluorid-etherat oder wasserfreie Zinksalze, z.B. Zinkchlorid, oder stark saure Ionenaustauscher, beispielsweise Ionenaustauscher mit Sulfonsäuregruppen, ohne Lösungsmittel, z.B. in einem Ueberschuss des Alkohols der Formel II, oder in Gegenwart eines inerten Lösungsmittels, z.B. Toluol, Chlorbenzol, Cyclohexan oder dergleichen, bei Temperaturen zwischen 0° und 200°C, bevorzugt zwischen 50° und 150°C, z.B. beim Siedepunkt des Lösungsmittels, Bevorzugt wird das bei der Veresterung entstehende Reaktionswasser entfernt, beispielsweise durch azeotrope Destillation mit geeigneten Schlepper-Lösungsmitteln, z.B. Toluol, durch Adsorption an Molekularsieb, z.B. Molekularsieb 3A, oder durch Reaktion mit Thionylchlorid, oder dergleichen.

Als Säurederivate geeignet für die Veresterung eines Allylalkohols sind beispielsweise Anhydride, z.B. das Anhydrid mit Chlorwasserstoffsäure (COCl anstelle von $COOR^b$), Anhydride mit starken organischen Säuren, z.B. mit Trifluoressigsäure, mit Ameisensäure oder mit einer Sulfonsäure, z.B. p-Toluolsulfonsäure, oder Anhydride mit sich selbst. Solche Anhydride können nach bekannten Methoden hergestellt werden, beispielsweise auch in situ durch Zugabe von Trifluoressigsäureanhydrid oder p-Toluolsulfonsäurechlorid in Ueberschuss von Pyridin. Säureanhydride werden mit Allylalkoholen in Gegenwart eines der oben genannten sauren Katalysatoren oder in Gegenwart einer Base umgesetzt, beispielsweise mit einem organischen

tertiären Amin, z.B. Triethylamin, Dimethylanilin, Pyridin oder 4-Dimethylaminopyridin. Anstelle einer Base kann auch der Allylalkohol der Formel II als Alkoholat, z.B. hergestellt mit Natrium- oder Kaliumhydrid, eingesetzt werden. Geeignet sind die oben genannten Reaktionsbedingungen und Lösungsmittel, ferner auch dipolar aprotische Lösungsmittel, z.B. Acetonitril oder Dimethylformamid, und Temperaturen von -30° bis 50° C, z.B. um 0° C.

Die Veresterung eines Allylalkohols der Formel II mit einem Ester der Formel III, worin $R^b$ einen Kohlenwasserstoffrest oder Silylrest bedeutet, erfolgt nach einer der üblichen Umesterungsmethoden, beispielsweise in Gegenwart eines sauren Katalysators, wie er oben bei der Veresterung der Säure genannt ist oder in Gegenwart einer Base, z.B. in Gegenwart einer katalytischen Menge des Alkoholats, das aus dem Allylalkohol der Formel II und Natrium- oder Kaliumhydrid oder Natrium in metallischer Form hergestellt worden ist. Bevorzugt wird die Reaktion in einem Ueberschuss des einzuführenden Alkohols der Formel II durchgeführt und/oder der austretende Alkohol der Formel $R^bOH$ z.B. durch Destillation oder Adsorption an Molekularsieb, z.B. Molekularsieb 4A, entfernt.

Ganz besonders bevorzugt ist die Umesterung in Gegenwart eines Tetraalkoxy-Derivats von Titan oder Zirkon, z.B. in Gegenwart von Titan-tetraethoxid, Titan-tetrabutoxid oder Titan-tetraisopropoxid, bevorzugt Titan-tetraethoxid. Der Katalysator wird in Mengen zwischen 0,01 % und 50 %, z.B. 1 % bis 30 %, zugesetzt und die Umesterung bei Temperaturen zwischen 50° und 200° C, z.B. beim Siedepunkt des Alkohols $R^bOH$ oder eines zugesetzten inerten Lösungsmittels, z.B. Toluol oder Cyclohexan, durchgeführt.

Die Veresterung des Allylalkohols der Formel II mit einem Ester-Derivat der Formel III umfasst z.B. die Reaktion mit einem Orthoester der Formel III, worin die Gruppe $COOR^b$ durch einen Rest $C(OR^b)_3$ ersetzt ist und $R^b$ Niederalkyl, z.B. Methyl oder Ethyl, bedeutet, die Reaktion mit einem Dialkoxyethylen der Formel III, worin die Gruppe $CH\text{-}COOR^b$ durch einen Rest $C=C(OR^b)_2$ ersetzt ist und $R^b$ Niederalkyl, z.B. Ethyl, bedeutet, ferner die Reaktion mit den entsprechenden Dialkylamino-Derivaten, in denen jeweils ein Rest $OR^b$ durch $NR^b_2$ ersetzt ist. Die Veresterung mit einem Orthoester oder einem Dialkoxyethylen erfolgt vorteilhafterweise in Gegenwart eines sauren Katalysators, wie er oben genannt ist, z.B. Bortrifluoridetherat, oder mit einem schwach sauren Katalysator, z.B. einer Niederalkancarbonsäure, z.B. Essigsäure oder Propionsäure, oder einer Arylcarbonsäure, z.B. Benzoesäure oder 2,4,6-Trimethylbenzoesäure, ohne Lösungsmittel oder in einem der genannten inerten Lösungsmittel bei Temperaturen zwischen 0° und 200° C, z.B. zwischen 0° und 100° C.

Enthalten die Reste $R^1$ und $R^4$ empfindliche funktionelle Gruppen, z.B. Carboxy-, Amino-, Hydroxy- oder Mercaptogruppen, so werden diese Gruppen in Verbindungen der Formel II und/oder III vorzugsweise vor der Veresterung durch eine der oben genannten Schutzgruppen geschützt.

Die Umlagerung des aus den Verbindungen der Formeln II und III hergestellten Allylesters der Formel VII in eine Verbindung der Formel IV erfolgt je nach Art der Substituenten $R^1$, $R^4$ und $R^a$ in situ bei den für die Veresterung oder Umesterung gewählten Reaktionsbedingungen.

Ist beispielsweise $R^1$ Phenyl, $R^4$ Wasserstoff, $R^a$ ein Rest $COOR^b$ und $R^b$ Ethyl und wird als Umesterungskatalysator Natriumacetat eingesetzt, so ist bekannt, dass durch Erhitzen auf 200°-230° C die Umlagerungsreaktion und Decarboxylierung zu der Verbindung der Formel IV eintritt. Das entstehende Produkt fällt allerdings nach Hydrolyse der Esterfunktion in nur 51 % Ausbeute in unreiner Form an. Ueberraschenderweise wurde nun gefunden, dass, wenn die Umesterung von Verbindungen der Formel III, worin $R^a$ eine Gruppe $COOR^b$ ist und $R^4$ auch verschieden von Wasserstoff sein kann, mit einem Allylalkohol der Formel II in Gegenwart eines Titan- oder Zirkontetraalkoholats durchgeführt wird, der Ester der Formel VII unter milden Bedingungen und mit wesentlich höheren Ausbeuten als bisher direkt in eine Verbindung der Formel IV, worin $R^a$ Wasserstoff bedeutet und $R^c$ die gleiche Bedeutung wie $R^b$ hat, umgewandelt werden kann. Beispielsweise entstehen bei der Reaktion von Verbindungen der Formel II mit Verbindungen der Formel III, worin $R^a$ eine Gruppe $COOR^b$ und $R^b$ Niederalkyl, z.B. Ethyl, ist, in Gegenwart von zwischen 1 % und 30 % eines Titan-tetraniederalkoxids, z.B. Titantetraethoxid, bei Temperaturen von 150° bis 220° C ohne Lösungsmittel oder in einem inerten Lösungsmittel, z.B. Mesitylen, Decahydronaphthalin oder Dichlorbenzol, in Ausbeuten von 50 % bis gegen 100 % Verbindungen der Formel IV, worin $R^a$ Wasserstoff und $R^c$ Niederalkyl wie in $R^b$ ist.

Erfolgt die Veresterung des Allylalkohols der Formel II mit einem Orthoester-Derivat der Formel III, worin die Gruppe $COOR^b$ durch einen Rest $C(OR^b)_3$ ersetzt ist, $R^b$ Niederalkyl, z.B. Methyl oder Ethyl, und $R^a$ Wasserstoff bedeuten, in Gegenwart einer schwach sauren organischen Verbindung, z.B. einer Niederalkancarbonsäure, wie Propionsäure, so erfolgt die Umlagerung zu einer Verbindung der Formel IV, worin $R^a$ Wasserstoff und $R^c$ Niederalkyl wie in $R^b$ sind, schon unter den Reaktionsbedingungen der Veresterung, z.B. bei Temperaturen zwischen 100° und 150° C ohne Lösungsmittel oder in Gegenwart eines inerten Lösungsmittels.

Gleichermassen erfolgt die Umlagerung in eine Verbindung der Formel IV, worin $R^a$ eine Gruppe $COOR^b$ und $R^c$ Niederalkyl wie in $R^b$ sind, wenn die Veresterung mit einem Dialkoxyethylen der Formel III, worin die Gruppe $CH\text{-}COOR^b$ durch einen Rest $C=C(OR^b)_2$ ersetzt ist und $R^a$ eine Gruppe $COOR^b$ und $R^b$ Niederalkyl bedeuten, in Gegenwart einer der oben genannten schwach sauren organischen Katalysatoren bei Temperaturen zwischen 100° und 160°C durchgeführt wird.

Erfolgt die Veresterung des Allylalkohols der Formel II mit einem Ester-Derivat der Formel III, worin die Gruppe $CH\text{-}COOR^b$ durch einen Rest $C=C(OR^b)(NR^b_2)$ ersetzt ist, so entsteht ohne Zusatz eines Katalysators durch Reaktion bei Temperaturen zwischen 100° und 200°C, bevorzugt 130° bis 170°C, beispielsweise in einem inerten Lösungsmittel, z.B. Xylol oder Dimethylformamid, eine Verbindung der Formel IV, worin der Rest $COOR^c$ durch eine Gruppe $CONR^b_2$ ersetzt ist.

Die Umlagerung von aus Verbindungen der Formeln II und III vorgeformten Allylestern der Formel VII in einem getrennten Reaktionsschritt ist dann der Regelfall, wenn nicht eines der oben genannten Esterderivate der Formel III zur Veresterung eingesetzt wird und $R^a$ Wasserstoff ist. Zur Umlagerung wird der Allylester der Formel VII bei tiefen Temperaturen, z.B. zwischen -100° und 0°C, bevorzugt zwischen -80° und -30°C, in einem polaren aprotischen Lösungsmittel oder Lösungsmittelgemisch, z.B. einem polaren Ether, z.B. Tetrahydrofuran, Dimethoxyethan oder Diethylenglykol-dimethylether, gegebenenfalls gemischt mit einem Amid, z.B. Dimethylformamid, N-Methylpyrrolidon oder Hexamethylphosphorsäuretriamid, oder einem Harnstoff, z.B. N,N'-Dimethyl-N,N'-propylenharnstoff, und/oder einem inerten Kohlenwasserstoff, z.B. Hexan oder Toluol, mit einer starken, nicht-nukleophilen Base deprotoniert, gewünschtenfalls mit einem Silylierungsmittel behandelt und durch Erwärmen auf Temperaturen von -20° bis 80°C, bevorzugt zwischen 0° und 30°C, in ein Salz der Verbindung der Formel IV, worin $R^c$ Wasserstoff, oder in eine Verbindung der Formel IV, worin $R^c$ den in der Silylierung verwendeten Silylrest darstellt, umgelagert.

Nicht-nukleophile Basen sind Verbindungen, die aus Estern ein Wasserstoff aus der $\alpha$-Position entfernen, ohne an die Carbonylgruppe der Esterfunktion zu addieren. Beispiele für solche starke, nicht-nukleophile Basen sind Alkalimetallsalze, z.B. Lithium-, Kalium-oder Natriumsalze, von sekundären Aminen mit sperrigen Kohlenwasserstoff- oder Silylresten, z.B. Lithiumdiisopropylamid, Lithiumdicyclohexylamid, Lithiumisopropylcyclohexylamid, Lithiumbis(trimethylsilyl)amid, Kaliumbis(trimethylsilyl)amid, Lithium-2,2,6,6-tetramethylpiperid oder dergleichen.

Bevorzugte Silylierungsmittel sind Triniederalkylsilylhalogenide mit gleichen oder verschiedenen Silylresten, z.B. Trimethylsilylchlorid, tert-Butyldimethylsilylchlorid oder Triisopropylsilylchlorid.

Die durch eine der oben aufgeführten Reaktionsschritte hergestellten Verbindungen der Formel IV weisen üblicherweise eine E-konfigurierte C=C-Doppelbindung auf.

In Estern der Formel VII, in denen $R^1$ verschieden von Wasserstoff und verschieden von Vinyl ist, ist das den Rest $R^1$ tragende Kohlenstoffatom chiral. Wird nun eine enantiomere Form des Esters der Formel VII in die Umlagerungsreaktion eingesetzt, so kann die Chiralität bei der Wahl geeigneter Reaktionsbedingungen von diesem Kohlenstoffatom auf das den Rest $R^4$ tragende Kohlenstoffatom im Produkt der Formel IV übertragen werden. Dabei ist es möglich, aus der einen enantiomeren Form des Esters der Formel VII durch Wahl geeigneter Lösungsmittel oder Lösungsmittelgemische in der Umlagerungsreaktion die eine oder die andere enantiomere Form des Produkts der Formel IV herzustellen.

Verbindungen der Formel IV, worin $R^c$ einen Silylrest darstellt, werden durch Wasser und/oder Niederalkanol, bevorzugt Methanol, bei Temperaturen zwischen 0° und 50°C hydrolysiert. Stellt $R^c$ einen Kohlenwasserstoffrest dar, so wird der Ester durch eine der üblichen Standardmethoden der Esterhydrolyse, beispielsweise in Gegenwart von wässriger Säure oder wässriger Base, z.B. wässrig-alkoholischer Kalilauge oder Natronlauge, in die Säure der Formel V umgewandelt. Ist $R^a$ eine Gruppe $COOR^b$ und $R^b$ verschieden von Wasserstoff, so wird im Anschluss an die obengenannte Esterhydrolyse das Reaktionsprodukt auf Temperaturen von 50° bis 200°C, bevorzugt um 150°C, erwärmt, um die Abspaltung von Kohlendioxid zu erreichen. Enthält die Verbindung der Formel IV in den Resten $R^1$ und/oder $R^4$ gegebenenfalls geschützte funktionelle Gruppen, so werden je nach Art der Schutzgruppe diese unter den Bedingungen der Esterhydrolyse wieder freigesetzt.

Bedeutet $R^4$ Halogen, so wird dieser Rest nach oder vorzugsweise vor einer allfälligen Hydrolyse und/oder Decarboxylierung durch einen Rest $R^2$ mit der Bedeutung Amino, Hydroxy oder Mercapto ausgetauscht. Der Austausch erfolgt unter den üblichen Bedingungen einer nukleophilen Substitution eines Halogenids. Die Einführung einer Aminogruppe, z.B. von Dimethylamino, Diethylamino oder Pyrrolidino, kann durch Reaktion mit dem entsprechenden freien Amin in einem inerten, vorzugsweise polaren Lösungsmittel, beispielsweise einem Alkohol, z.B. Methanol oder Ethanol, Acetonitril, Tetrahydrofuran, Dimethylformamid, Dimethylsulfoxid oder dergleichen bei Temperaturen zwischen 0° und 100°C erreicht werden. Zur Einführung einer unsubstituierten Aminogruppe verwendet man vorzugsweise die weiter unten bei der Umwandlung einer Verbindung der Formel VI in eine Verbindung der Formel I genannten

Reagentien und Bedingungen. Halogen kann durch unsubstituiertes Hydroxy unter den obengenannten Bedingungen der Esterhydrolyse ausgetauscht werden. Verethertes Hydroxy, z.B. Methoxy, wird durch Reaktion in einem Ueberschuss des entsprechenden Alkohols, acyliertes Hydroxy, z.B. Acetoxy, durch Reaktion in einem Ueberschuss der entsprechenden Carbonsäure eingeführt, wobei vorzugsweise in Gegenwart einer nicht-nukleophilen Base gearbeitet wird, um den freigesetzten Halogenwasserstoff zu binden. Eine substituierte, z.B. veretherte, Mercaptogruppe wird durch Reaktion mit dem entsprechenden Mercaptan oder Alkalimetallmercaptid in einem der obengenannten, inerten, polaren Lösungsmittel und bei den genannten Temperaturen eingeführt.

Der Austausch von Halogen durch einen der genannten Reste $R^2$ kann im allgemeinen so geführt werden, dass sich die Konfiguration des den Rest $R^4$ bzw. $R^2$ tragenden Kohlenstoffatoms in Verbindungen der Formel IV bzw. V umkehrt.

Ist $R^2$ verschieden von Wasserstoff und verschieden vom Rest der Formel $R^1CH=CHCH_2$-, so ist die Verbindung der Formel V chiral. Soll in einem Endprodukt der Formel I das den Rest $R^2$ tragende Kohlenstoffatom nur eine der beiden möglichen Konfigurationen aufweisen, so wird eine racemische Verbindung der Formel V bevorzugt auf dieser Stufe einer Enantiomerentrennung unterworfen. Enantiomerentrennungen racemischer Carbonsäuren der Formel V werden analog bekannten Methoden beispielsweise durch fraktionierte Kristallisation oder chromatographische Auftrennung diastereomerer Carbonsäuresalze oder Carbonsäureamide mit chiralen, gewünschtenfalls enantiomerenreinen organischen Aminen erreicht. Beispielsweise werden Carbonsäuren der Formel V in einem Lösungsmittel mit äquimolaren Mengen eines enantiomerenreinen Amins, z.B. (R)- oder (S)-$\alpha$-Phenylethylamin, (R)- oder (S)-1-$\alpha$- oder $\beta$-Naphthylethylamin, Chinin, Chinchonidin, Dehydroabietylamin oder d- oder 1-Ephedrin, umgesetzt, die diastereomeren Salze durch fraktionierte Kristallisation getrennt und gereinigt, die diastereomerenreinen Salze durch Zugabe einer wässrigen Säure gespalten, das als Hilfsstoff eingesetzte Amin abgetrennt und die enantiomerenreine Säure der Formel V isoliert.

Umwandlung einer Verbindung der Formel V in eine Verbindung der Formel VI:

Die $\gamma$,$\delta$-ungesättigten Carbonsäuren der Formel V werden durch Halogenierungsmittel in Halolactone der Formel VI übergeführt. Verbindungen der Formel VI, worin X Iod bedeutet, werden beispielsweise erhalten, indem man Säuren der Formel V mit Iod in wässrigen oder organischen Lösungsmitteln umsetzt, z.B. in Wasser, wässrigem Niederalkanol, z.B. wässrigem Methanol oder Ethanol, in Wasser-Ether-Mischungen,z.B. Wasser-Diethylether, in Acetonitril, Amiden, z.B. Dimethylform-amid oder N-Methylpyrrolidon, polaren Ethern, z.B. Tetrahydrofuran, Dioxan oder Dimethoxyethan, oder in Halogenkohlenwasserstoffen,z.B. Methylenchlorid oder Chloroform, bei Temperaturen zwischen -80° und 50°C, bevorzugt zwischen 0° und 30°C, gewünschtenfalls in Gegenwart einer Base, die freigesetzte Iodwasserstoffsäure bindet, beispielsweise Alkalihydrogencarbonat, z.B. Natriumhydrogencarbonat, oder Alkali- oder Erdalkalicarbonat, z.B. Magnesium- oder Calciumcarbonat, und/oder in Gegenwart von Kaliumiodid, das die Löslichkeit von Iod in wässrigen Lösungsmitteln erhöht. Anstelle von Iod kann auch ein anderes, positives Iod abgebendes Reagens verwendet werden, beispielsweise N-Iodsuccinimid oder N-Iodacetamid.

Verbindungen der Formel VI, worin X Brom bedeutet, werden erhalten, indem man Säuren der Formel V mit Brom, N-Bromsuccinimid oder andern positives Brom abgebenden Reagentien in den obengenannten Lösungsmitteln und bei den genannten Reaktionsbedingungen umsetzt, beispielsweise mit Brom in wässriger Natriumhydrogencarbonat-Lösung enthaltend gewünschtenfalls Kaliumbromid, mit Brom in Acetonitril oder mit N-Bromsuccinimid in Dimethylformamid, Tetrahydrofuran oder dergleichen. Zu Verbindungen der Formel VI, worin X Chlor bedeutet, gelangt man auf entsprechende Art und Weise, indem als Halogenierungsmittel N-Chlorsuccinimid eingesetzt wird.

Die Konfiguration des den Rest $R^2$ tragenden Kohlenstoffatoms beeinflusst die räumliche Anordnung der Substituenten -OCO und -X in Verbindungen der Formel VI. Unter den üblichen Reaktionsbedingungen der Halolactonisierung werden bevorzugt jene zwei Diastereomeren gebildet, die durch antiperiplanare Anlagerung der Substituenten -OCO und -X an die Doppelbindung der ungesättigten Säure der Formel V entstehen. Durch Addition an eine E-konfigurierte C=C-Doppelbindung der Verbindung der Formel V weisen die neugebildeten chiralen Kohlenstoffatome somit die R,S- oder die S,R-Konfiguration auf. Abhängig von der Art des Substituenten $R^2$ und den gewählten Reaktionsbedingungen ist es möglich, die Reaktion so zu führen, dass eines dieser beiden Diastereomeren klar überwiegt und leicht in reiner Form abgetrennt werden kann.

Bevorzugt wird die Carbonsäure der Formel V vor der Behandlung mit dem Halogenierungsmittel in ein Amid oder einen Hydroxamsäureester der Formel

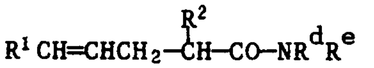 (VIII)

übergeführt.

Geeignet sind beispielsweise Amide der Formel VIII, worin $R^d$ und $R^e$ unabhängig voneinander Niederalkyl, z.B. Methyl oder Ethyl, Cycloalkyl, z.B. Cyclohexyl, oder Arylniederalkyl, z.B. Benzyl, oder $R^d$ und $R^e$ zusammen Niederalkylen, Oxaniederalkylen oder Niederalkylazaniederalkylen mit 4 bis 6 Atomen in der Kette, wobei jedoch Oxa oder Aza nicht in 1-Stellung stehen, z.B. 1,4-Butylen, 1,5-Pentylen, 1,6-Hexylen, 3-Oxa-1,5-pentylen oder 3-Methyl-3-aza-1,5-pentylen, darstellen.

Solche Amide werden nach den üblichen Methoden aus den entsprechenden Carbonsäuren der Formel V hergestellt, wie es z.B. in "Methoden der Organischen Chemie (Houben-Weyl)", Band E5, Thieme Verlag, Stuttgart 1985, Seiten 941-982, beschrieben ist. Beispielsweise werden die Carbonsäuren mit einem üblichen Halogenierungsmittel, z.B. Thionylchlorid, Phosphortri- oder -pentachlorid, Phosgen oder Oxalychlorid, gegebenenfalls in Gegenwart eines Katalysators, z.B. Zinkchlorid, Pyridin, Dimethylformamid oder Hexamethylphosphorsäuretriamid, ohne Lösungsmittel oder in einem inerten Lösungsmittel, beispielsweise einem Kohlenwasserstoff, z.B. Toluol oder Hexan, oder einem Ether, z.B. Diethylether, bei Temperaturen zwischen 20° und 120°C in das entsprechende Säurehalogenid übergeführt und mit dem entsprechenden Amin, beispielsweise dem Diniederalkylamin oder dem cyclischen sekundären Amin, gegebenenfalls in einem inerten organischen Lösungsmittel, beispielsweise einem Halogenkohlenwasserstoff, z.B. Methylenchlorid, oder einem Ether, z.B. Diethylether und in Gegenwart einer Base, beispielsweise eines Trialkylamins, z.B. von Triethylamin oder Triisopropylamin, Pyridin und/oder 4-Dimethylaminopyridin, bei Temperaturen zwischen -30° und 100°C, z.B. um 0°C, umgesetzt.

Die Amide der Formel VIII, worin $R^d$ und $R^e$ die genannten Bedeutungen haben, werden unter den für Carbonsäuren der Formel V genannten Reaktionsbedingungen der Halolactonisierung unterworfen. Als Halogenierungsmittel können dafür beispielsweise Iod, N-Iodsuccinimit, Brom, N-Bromsuccinimid oder N-Chlorsuccinimid in den genannten Lösungsmitteln oder Lösungsmittelgemischen bei Temperaturen zwischen -30° und 80°C, bevorzugt zwischen 0° und 30°C, eingesetzt werden.

Besonders bevorzugt ist die Halolactonisierung von Amiden der Formel VIII mit N-Bromsuccinimid in Gegenwart einer schwachen Säure, beispielsweise einer Niederalkancarbonsäure, z.B. Essigsäure, in einem wässrig-organischen Lösungsmittelgemisch, z.B. wässrigem Tetrahydrofuran, bei Temperaturen um 0°C. Unter diesen bevorzugten Reaktionsbedingungen werden von den möglichen Diastereomeren überwiegend nur jene gebildet, in welchen der Rest $R^2$ und die die Reste $R^1$ und X tragende Methylgruppe trans (bezüglich des Lactonringes) zueinander angeordnet und die Substituenten -X und -OCO durch antiperiplane Addition an die Doppelbindung eingeführt sind.

Für die Halolactonisierung geeignete Hydroxamsäureester sind Verbindungen der Formel VIII, worin $R^d$ Niederalkyl, z.B. Methyl oder Ethyl, und $R^e$ Niederalkoxy, z.B. Methoxy oder Ethoxy, oder $R^d$ und $R^e$ zusammen 1-Oxaniederalkylen mit 4 bis 6 Atomen in der Kette, z.B. 1-Oxa-1,4-butylen oder 1-Oxa-1,5-pentylen, bedeuten. Solche Hydroxamsäureester werden nach den üblichen Methoden aus Carbonsäuren der Formel V hergestellt, wie z.B. in "Houben-Weyl", Band E5, Seiten 1144-1149, beschrieben, beispielsweise durch Umsetzung eines Carbonsäurehalogenids, z.B. des Carbonsäurechlorids, mit einem N,O-Diniederalkylhydroxylamin oder dem entsprechenden cyclischen Hydroxylamin, gegebenenfalls in einem inerten organischen Lösungsmittel und in Gegenwart einer Base, beispielsweise unter den für die Herstellung von Diniederalkylamiden aus Carbonsäurechloriden üblichen Reaktionsbedingungen.

Hydroxamsäureester der Formel VIII, worin $R^d$ Niederalkyl und $R^e$ Niederalkoxy oder $R^d$ und $R^e$ zusammen 1-Oxaniederalkylen bedeuten, werden bevorzugt mit N-Bromsuccinimid in einem der obengenannten Lösungsmittel oder Lösungmittelgemisch bei Temperaturen zwischen -20° und 80°C, bevorzugt um 0°C, umgesetzt. Dabei wird bevorzugt das gleiche Diastereomere der Formel VI gebildet, das auch bei der bevorzugten Cyclisierung von Carbonsäureamiden entsteht.

Enthalten die Reste $R^1$ und $R^2$ empfindliche funktionelle Gruppen, z.B. Carboxy-, Amino-, Hydroxy- oder Mercaptogruppen, in ungeschützter Form, so werden diese Gruppen in Verbindungen der Formel V bevorzugt vor der Halolactonisierung und gegebenenfalls vor der Umwandlung in ein Amid oder einen Hydroxamsäureester der Formel VIII durch eine der weiter oben genannten Schutzgruppen geschützt.

Umwandlung einer Verbindung der Formel VI in eine Verbindung der Formel I:

In Verbindungen der Formel VI wird X mit der Bedeutung Halogen, insbesondere Chlor, Brom oder Iod, durch einen über Stickstoff gebundenen Rest ausgetauscht. Dieser Austausch erfolgt beispielsweise durch

eine nukleophile Substitution mit einem geeigneten stickstoffhaltigen Reagens, beispielsweise Azid, Cyanat, leicht spaltbaren sekundären Aminen, z.B. Dibenzylamin, Bis(phenylthio)amin oder Bis(trimethylsilyl)amin, leicht spaltbaren tertiären Aminen, z.B. Hexamethylentetramin, Imiden, z.B. Phthalimid oder N-Ethoxycarbonyl-p-toluolsulfonamid, Hydrazinen, z.B. N,N-Dimethylhydrazin, Cyanamid, Guanidin oder dergleichen.

Beispielsweise wird in Verbindungen der Formel VI, worin X Chlor, Brom oder Iod bedeutet, nach den üblichen Methoden einer nukleophilen Substitution X durch Azid ausgetauscht, beispielsweise mit Alkalimetallazid, z.B. Natriumazid, oder Ammoniumazid, z.B. unsubstituiertem Ammoniumazid oder Tetrabutylammoniumazid, in einem polaren organischen Lösungsmittel, beispielsweise in einem Niederalkanol, z.B. Methanol oder Ethanol, in einem Diniederalkylketon, z.B. Aceton, einem Nitril, z.B. Acetonitril, einem Amid, z.B. Dimethylformamid, N,N-Dimethylacetamid, N-Methylpyrrolidon oder Hexamethylphosphorsäuretriamid, einem Harnstoff, z.B. N,N′-Dimethyl-N,N′-propylenharnstoff, einem polaren Ether, z.B. Tetrahydrofuran, Dioxan, Dimethoxyethan oder Diethylenglykoldimethylether, einem Niederalkoxyniederalkanol, z.B. Diethylenglykolmonomethyl- oder monobutylether, Dimethylsulfoxid oder Mischungen der genannten Lösungsmittel untereinander oder mit Wasser, bei Temperaturen zwischen 0° und 200°C, bevorzugt zwischen 20° und 50°C. Der Austausch von Halogen X durch Azid kann auch in zweiphasigen Systemen, bevorzugt in Gegenwart eines Phasentransferkatalysators, erreicht werden, beispielsweise in Wasser-Halogenkohlenwasserstoff-Mischungen, z.B. Wasser-Chloroform oder Wasser-Methylenchlorid, oder in Wasser-Kohlenwasserstoff-Mischungen, z.B. Wasser-Toluol, beispielsweise unter Zusatz eines Benzyltriniederalkylammoniumsalzes, z.B. Benzyltrimethylammonium-chlorid oder -hydrogensulfat, oder eines langkettigen Alkyltriniederalkylammonium- oder phosphoniumsalzes, z.B. Hexadecyltrimethylammonium- oder -phosphoniumchlorid, bei Temperaturen zwischen 0° und 100°C, bevorzugt zwischen 20° und 80°C.

Für den Austausch des Restes X mit der Bedeutung Halogen durch Cyanat verwendet man beispielsweise Alkalimetallcyanat, z.B. Kaliumcyanat oder Natriumcyanat, oder Ammoniumcyanat, z.B. Tetrabutylammoniumcyanat, in einem der obengenannten polaren aprotischen Lösungsmittel, z.B. in Acetonitril, Dimethylformamid, N-Methylpyrrolidon, Tetrahydrofuran, Dimethoxyethan, Dimethylsulfoxid oder dergleichen bei den genannten Temperaturen. Der Austausch ist auch möglich mit Silbercyanat in unpolaren Lösungsmitteln, z.B. Benzol oder Toluol, bei Temperaturen zwischen -20° und 50°C, z.B. um 0°C.

Unter den gleichen Bedingungen wie Azid oder Cyanat können auch andere salzartige Stickstoff-Nukleophile eingeführt werden, beispielsweise das Kaliumsalz von Phthalimid oder Natrium- oder Kalium-N-ethoxycarbonyl-p-toluolsulfonamid.

Die genannten polaren Lösungsmittel und Reaktionstemperaturen sind ebenfalls geeignet für die Einführung der übrigen genannten Stickstoff-Nukleophile, z.B. von Dibenzylamin oder N,N-Dimethylhydrazin. Dabei ist jedoch je nach Art der Substituenten $R^1$ und $R^2$ der Austausch unter derart kontrollierten Bedingungen durchzuführen, dass nicht gleichzeitig die Carbonylgruppe des Lactonringes mit dem Stickstoff-haltigen nukleophilen Reagens umgesetzt und ein offenkettiges Säurederivat gebildet wird.

Durch die oben beschriebene Umwandlung der Verbindung der Formel VI, in der X Halogenid bedeutet, in eine Verbindung der Formel VI, in der X ein stickstoffhaltiger Rest, z.B. Azid, bedeutet, kehrt sich die Konfiguration am den Substituenten X tragenden Kohlenstoffatom um. Wird beispielsweise von einem Diastereomeren ausgegangen, in dem das den Rest X tragende Kohlenstoffatom die R-Konfiguration aufweist, so zeigt z.B. das gebildete Azid oder Cyanat an diesem Kohlenstoffatom S-Konfiguration, wobei die Konfiguration an den übrigen chiralen Zentren, insbesondere am Kohlenstoffatom, das den Rest -OCO, und am Kohlenstoffatom, das den Rest $R^2$ trägt, unverändert bleibt.

Ein auf die oben beschriebene Art und Weise hergestelltes Lacton der Formel VI, worin X Azid bedeutet, wird entweder zuerst mit einer den Rest $R^3$ einführenden Verbindung umgesetzt, wobei eine Verbindung der Formel

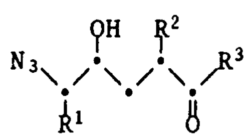 (IX)

gebildet wird, oder zuerst mit einem Reduktionsmittel behandelt, wobei eine Verbindung der Formel VI, worin X Amino bedeutet, entsteht.

Hat $R^3$ die Bedeutung unsubstituiertes oder substituiertes Amino, so wird bevorzugt das Lacton der Formel VI, worin X Azid bedeutet, in eine offenkettige Verbindung der Formel IX umgewandelt.

Ist $R^3$ Amino, so ist das Reagens beispielsweise Ammoniak, z.B. gasförmiges Ammoniak oder Ammoniak in konzentrierter oder verdünnter wässriger Lösung. Die Umsetzung mit Ammoniak wird bevorzugt bei Temperaturen zwischen -30° und 30°C, bevorzugt um 0°C, durchgeführt.

Ist $R^3$ monosubstituiertes Amino, so ist das Reagens bevorzugt das entsprechende primäre Amin. Die Reaktion kann ohne Lösungsmittel oder in einem polaren Lösungsmittel durchgeführt werden, beispielsweise in einem Niederalkanol, z.B. Methanol oder Ethanol, einem polaren Ether, z.B. Tetrahydrofuran oder Dioxan, einem Nitril, z.B. Acetonitril, oder in einem anderen der oben bei der Umwandlung in ein Azid genannten polaren Lösungsmittel, auch in Mischungen der Lösungsmittel untereinander oder mit Wasser. Die Lactonringöffnung mit einem primären Amin wird bei Temperaturen zwischen -30° und 100°C, bevorzugt zwischen 20° und 80°C, durchgeführt.

Ist $R^3$ disubstituiertes oder cyclisches Amino, so ist das Reagens das entsprechende sekundäre Amin. Dabei werden die gleichen Reaktionsbedingungen wie bei primären Aminen angewendet.

Ist $R^3$ gegebenenfalls substituiertes Hydroxy, so ist die Oeffnung des Lactonringes der Formel VI, worin X Azid bedeutet, zwar mit den weiter unten genannten, den Rest $R^3$ einführenden Reagentien möglich, aber von geringem praktischem Nutzen, weil gleichzeitig die Azidgruppe teilweise oder vollständig eliminiert wird.

Enthält der Rest $R^3$ empfindliche funktionelle Gruppen, z.B. Carboxy-, Amino-, Hydroxy- oder Mercaptogruppen, so werden diese im Reagens vor der Umsetzung mit Verbindungen der Formel VI vorteilhafterweise durch eine der weiter vorn genannten Schutzgruppen geschützt.

Im nächsten Reaktionsschritt wird der Azidrest in einer Verbindung der Formel IX reduktiv in eine Aminogruppe umgewandelt. Bevorzugt wird die Reduktion durch katalytische Hydrierung mit Wasserstoff erreicht, beispielsweise in Gegenwart eines Edelmetallkatalysators, wie Platin, z.B. als Platinoxid, feinverteiltes metallisches Platin oder auf einen Träger aufgebrachtes metallisches Platin, Palladium, z.B. in Form von metallischem Palladium auf einem Träger, z.B. auf Aktivkohle, oder auch in Gegenwart von aktiviertem Nickel, z.B. Raney-Nickel. Die katalytische Hydrierung wird vorzugsweise in einem inerten Lösungsmittel oder Lösungsmittelgemisch, beispielsweise in einem halogenierten Kohlenwasserstoff, z.B. Methylenchlorid, einem Ether, z.B. Tetrahydrofuran oder Dioxan, einem Ester, z.B. Essigsäureethylester, einem Alkohol, z.B. Methanol oder Ethanol, oder Mischungen der genannten Lösungsmittel untereinander oder mit Wasser bei Temperaturen zwischen 0° und 50°C, beispielsweise zwischen 20° und 30°C, durchgeführt. Die Azidgruppe kann auch mit anderen Reduktionsmitteln, z.B. mit metallischem Zink in Gegenwart einer Säure, mit Lithiumaluminiumhydrid, ferner mit Triphenylphosphin oder Triethylphosphit und anschliessender Säurebehandlung, in die Aminogruppe umgewandelt werden.

Enthält eine Verbindung der Formel IX funktionelle Gruppen in durch Schutzgruppen geschützter Form, so können diese je nach Art der Schutzgruppe und je nach Reaktionsbedingungen während der Reduktion der Azidgruppe der Formel IX freigesetzt werden.

Das Lacton der Formel VI, worin X Azid bedeutet, kann erfindungsgemäss auch in ein Lacton der Formel VI, worin X Amino bedeutet, umgewandelt und nach Schutz der Aminofunktion mit einem den Rest $R^3$ einführenden Reagens geöffnet werden.

Zur Reduktion des Azidrestes in einem Lacton der Formel VI sind die oben für die Reduktion des Azidrestes in einer Verbindung der Formel IX genannten Bedingungen geeignet.

Die Aminogruppe in einem solchermassen reduzierten Lacton der Formel VI wird vor der Einführung des Restes $R^3$ vorzugsweise mit einer Schutzgruppe geschützt. Geeignete Amino-Schutzgruppen und Bedingungen für ihre Einführung sind weiter oben genannt. Vorzugsweise werden Schutzgruppen verwendet, die unter den Bedingungen der Lactonring-Oeffnung, insbesondere in Gegenwart einer Base, stabil sind, beispielsweise gegebenenfalls substituiertes Niederalkanoyl, gegebenenfalls substituiertes Benzoyl oder Arylmethyl, z.B. Trityl.

Ist $R^3$ unsubstituiertes oder substituiertes Amino, so wird das Lacton der Formel VI, worin X gegebenenfalls geschütztes Amino bedeutet, mit einem der oben genannten Reagentien unter den erwähnten Reaktionsbedingungen in eine gegebenenfalls an der Aminofunktion geschützte Verbindung der Formel I übergeführt.

Ist $R^3$ Hydroxy, so wird die Lactonringöffnung mit einer wässrigen Base, beispielsweise einem Alkali- oder Erdalkalihydroxid, z.B. Lithium-, Natrium- oder Kaliumhydroxid oder Calciumhydroxid, oder einem tetrasubstituierten Ammoniumhydroxid, z.B. Benzyltrimethylammoniumhydroxid, in wässriger Lösung bei Temperaturen zwischen 0° und 100°C, beispielsweise zwischen 20° und 50°C, durchgeführt.

Ist $R^3$ substituiertes Hydroxy, so ist das Reagens beispielsweise der entsprechende Alkohol. Die Ringöffnung wird dabei in einem Ueberschuss des entsprechenden Alkohols ohne Lösungsmittel oder in Gegenwart eines der bei der Umwandlung des Halolactons ins Azid genannten polaren organischen Lösungsmittels mit Ausnahme der genannten Niederalkanole bei den angegebenen Temperaturen umgesetzt, bevorzugt in Gegenwart einer katalytischen oder äquimolaren Menge des dem Reagens entsprechenden Alkoholats, z.B. des Natrium-, Kalium- oder Titantetra-alkoholats.

Ein auf die weiter oben beschriebene Art und Weise hergestelltes Lacton der Formel VI, worin X Cyanat bedeutet, wird vorzugsweise mit einem Alkohol, z.B. Benzylalkohol, umgesetzt, wobei der Alkohol an die Carbonylgruppe der Cyanat-Gruppe addiert und ein Kohlensäurehalbester-geschütztes Derivat der Verbindung der Formel VI, worin X Amino bedeutet, entsteht. Eine solche geschützte Verbindung wird wie oben beschrieben mit einem den Rest $R^3$ einführenden Reagens umgesetzt und dabei in eine an der Aminofunktion durch den Acylrest eines Kohlensäurehalbesters geschützte Verbindung der Formel I übergeführt.

Lactone der Formel VI, worin X ein anderer stickstoffhaltiger Rest als Azid oder Cyanat bedeutet, werden vorzugsweise zuerst mit einem den Rest $R^3$ einführenden Reagens in der oben beschriebenen Art und Weise umgesetzt.

Im letzten Schritt wird dann aus dem stickstoffhaltigen Rest die Aminogruppe freigesetzt. Ist dieser Rest Dibenzylamino, Hydrazino oder N,N-Dimethylhydrazino, so erfolgt die Freisetzung durch katalytische Hydrierung, beispielsweise unter Bedingungen, wie sie oben für die Reduktion des Azidrestes genannt sind. Aus Phthalimido wird die Aminogruppe vorzugsweise durch Hydrazinolyse freigesetzt. Die Spaltung der Bis-(phenylthio)aminogruppe, der Bis(trimethylsilyl)aminogruppe oder des mit Hexamethylentetramin gebildeten Ammoniumsalzes erfolgt beispielsweise mit Säure, z.B. mit wässriger Mineralsäure, wie Salzsäure oder Schwefelsäure. Mit wässriger Säure und/oder Base werden die entsprechenden Cyanamino-, Guanidino- und Ethoxycarbonyl-p-toluolsulfonamido-Gruppen hydrolysiert.

Nachoperationen:

In einer nach dem oben beschriebenen Verfahren hergestellten Verbindung der Formel I oder irgendeinem der genannten Zwischenprodukte der Formeln IV, V, VI, VIII oder IX, worin eine oder mehrere funktionelle Gruppen geschützt sind, können diese Gruppen, z.B. Carboxy-, Amino-, Hydroxy- und/oder Mercaptogruppen, in an sich bekannter Weise mittels Solvolyse, insbesondere Hydrolyse, gegebenenfalls enzymatischer Hydrolyse, Alkoholyse oder Acidolyse, oder mittels Reduktion, insbesondere Hydrogenolyse oder chemischer Reduktion, gegebenenfalls stufenweise oder gleichzeitig, freigesetzt werden. Die Abspaltung der Schutzgruppen ist in den weiter vorn genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Gegebenenfalls substituiertes Benzyloxycarbonyl kann beispielsweise mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch durch Reduktion, beispielsweise durch Behandeln mit einem Alkalimetall-, z.B. Natrium-dithionit, mit einem reduzierenden Metall, z.B. Zink, oder einem reduzierenden Metallsalz, wie einem Chrom-II-salz, z.B. Chrom-II-chlorid, üblicherweise in Gegenwart eines Wasserstoff-abgebenden Mittels, das zusammen mit dem Metall naszierenden Wasserstoff zu erzeugen vermag, wie einer Säure, in erster Linie einer geeigneten Carbonsäure, z.B. einer gewünschtenfalls substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Benzyltriniederalkylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino können z.B. durch Behandeln mit einem geeigneten Reduktionsmittel, wie Zink in Gegenwart einer geeigneten Carbonsäure, z.B. wässriger Essigsäure, gespalten werden. Aroylmethoxycarbonylamino kann auch durch Behandeln mit einem nukleophilen, vorzugsweise salzbildenden Reagens, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert-Niederalkoxycarbonylamino oder 2-Triniederalkylsilylniederalkoxycarbonylamino können durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, und gegebenenfalls substituiertes Triarylmethylamino oder Formylamino z.B. durch Behandeln mit einer Säure, wie Mineralsäure, z.B. Salzsäure, oder einer organischen Säure, z.B. Ameisen-, Essig-oder Trifluoressigsäure, gegebenenfalls in Gegenwart von Wasser, freigesetzt werden. Eine durch 2-Halogenacetyl, z.B. 2-Chloracetyl, geschützte Aminogruppe kann durch Behandeln mit Thioharnstoff in Gegenwart einer Base oder mit einem Thiolatsalz, z.B. einem Alkalimetallthiolat des Thioharnstoffs, und anschliessende Solvolyse, z.B. Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden.

Eine durch eine geeignete Acylgruppe oder durch gegebenenfalls substituiertes 1-Phenylniederalkyl geschützte Hydroxy- oder Mercaptogruppe wird analog einer entsprechend geschüzten Aminogruppe freigesetzt. Eine durch 2,2-Dichloracetyl geschützte Hydroxygruppe wird z.B. durch basische Hydrolyse, eine durch tert-Niederalkyl, Silyl oder durch einen 2-oxa- oder 2-thia-aliphatischen oder -cycloaliphatischen Kohlenwasserstoffrest geschützte Hydroxy- oder Mercaptogruppe durch Acidolyse, z.B. durch Behandeln mit einer Mineralsäure oder einer starken Carbonsäure, z.B. Trifluoressigsäure, freigesetzt. Eine durch eine organische Silylgruppe geschützte Hydroxy- oder Mercaptogruppe lässt sich auch mit einem Fluoridsalz freisetzen, wie oben für die Freisetzung einer entsprechend geschützten Carboxygruppe angegeben ist. Eine als Disulfid, z.B. durch Niederalkylthio, geschützte Mercaptogruppe wird reduktiv freigesetzt, beispielsweise mit Lithiumaluminiumhydrid oder Natriumborhydrid. Thioacetale lassen sich mit Quecksilbersalzen, z.B. wässrigem Quecksilberacetat, spalten.

Eine nach dem oben beschriebenen Verfahren hergestellte Verbindung der Formel I oder irgendeines der genannten Zwischenprodukte der Formeln IV, V, VI, VIII oder IX kann in eine andere Verbindung der Formel I oder in ein anderes entsprechende Zwischenprodukt der Formeln IV, V, VI, VIII oder IX umgewandelt werden.

Beispielsweise kann man eine Amino-, Hydroxy- oder Mercaptogruppe $R^2$ oder auch eine Carboxygruppe in einem Carboxyalkylrest, eine Aminogruppe in einem Aminoalkylrest oder eine Hydroxygruppe in einem Hydroxyalkylrest $R^1$, $R^2$, $R^3$ oder $R^4$ mit einem Alkylierungsmittel alkylieren. Geeignete Alkylierungsmittel sind beispielsweise Alkylhalogenide, Sulfonsäureester, Meerweinsalze oder 1-substituierte 3-Aryltriazene, für Methylierungen auch Diazomethan. Eine Amino-, Hydroxy- oder Mercaptogruppe kann auch acyliert werden, beispielsweise unter den bei der Einführung von Acyl-Schutzgruppen beschriebenen Reaktionsbedingungen.

Ferner kann man eine substituierte Mercaptogruppe $R^2$ oder $R^4$ zu einer Sulfinyl- oder Sulfonylgruppe oder eine Sulfinylgruppe $R^2$ oder $R^4$ zu einer Sulfonylgruppe oxidieren. Für diese Oxidationen verwendet man vorzugsweise selektive Oxidationsmittel, z.B. aromatische oder aliphatische Peroxycarbonsäuren, z.B. m-Chlorperbenzoesäure oder Peressigsäure, Wasserstoffperoxid, Kaliumperoxomonosulfat oder tert-Butylhypochlorit. Gleichermassen ist es möglich, eine Sulfonylgruppe oder Sulfinylgruppe $R^2$ oder $R^4$ in eine substituierte Mercaptogruppe umzuwandeln, beispielsweise mit Hydriden, z.B. Diisobutylaluminiumhydrid oder Natriumborhydrid, katalytischem Wasserstoff, Boranen, z.B. Dichlorboran, oder dergleichen.

Salze von Verbindungen der Formel I oder von den genannten Zwischenprodukten erhält man in üblicher Weise, z.B. Säureadditionssalze durch Behandeln mit einer Säure oder einem geeigneten Anionenaustauscherreagens, wobei man vorzugsweise stöchiometrische Mengen oder nur einen kleinen Ueberschuss des salzbildenden Mittels verwendet. Innere Salze von Verbindungen der Formel I, welche z.B. eine freie Carboxygruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden. Salze von Verbindungen der Formeln IV oder V mit Carboxygruppen kann man z.B. durch Behandeln mit Metallverbindungen, wie Alkalimetallsalzen von geeigneten organischen Carbonsäuren, z.B. dem Natriumsalz der 2-Ethylhexansäure, oder mit anorganischen Alkali- oder Erdalkalimetallsalzen, z.B. Natriumhydrogencarbonat, oder mit Ammoniak oder einem geeigneten organische Amin bilden.

Salze können in üblicher Weise in die freien Verbindungen übergeführt werden, Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise gespalten werden, z.B. nach Ueberführen der optischen Antipoden in diastereomere Salze, beispielsweise durch Umsetzung mit chiralen, gewünschtenfalls enantiomerenreinen Carbon- oder Sulfonsäuren, analog dem bei der Enantiomerentrennung von Verbindungen der Formel V oben beschriebenen Verfahren.

Das Verfahren der Erfindung umfasst auch diejenigen Ausführungsformen, bei denen Zwischenprodukte isoliert und die restlichen Verfahrensschritte mit diesen durchgeführt werden, Ausgangsstoffe und Reagentien in situ hergestellt werden und/oder Zwischen- und Endprodukte ohne Isolierung weiterverarbeitet werden.

Insbesondere betrifft die Erfindung auch die im folgenden genannten neuen Verfahrensschritte unter den genannten bevorzugten Reaktionsbedingungen als solche oder als Teil eines gesamten Verfahrens zur Herstellung der Verbindung der Formel I.

Bevorzugt ist ein Verfahren zur Herstellung von Verbindungen der Formel IV, worin $R^1$ und $R^4$ die oben genannten Bedeutungen haben und $R^a$ Wasserstoff und $R^c$ Wasserstoff oder Niederalkyl bedeuten, dadurch gekennzeichnet, dass man einen Allylalkohol der Formel II mit einem Malonester der Formel III, worin $R^a$ eine Gruppe $COOR^b$ und $R^b$ Niederalkyl, z.B. Ethyl, bedeuten, in Gegenwart eines Tetraalkoxy-Derivats von Titan oder Zirkon bei Temperaturen zwischen 150° und 250°C umsetzt, und gewünschtenfalls die erhältliche Verbindung der Formel IV, worin $R^a$ Wasserstoff und $R^c$ Niederalkyl bedeuten, mit einer wässrigen Base hydrolysiert.

Ganz besonders bevorzugt ist das genannte Verfahren unter Verwendung von 1 % bis 30 % eines Tetraniederalkoxy-Derivats von Titan, z.B. von Titan-tetraethoxid.

Ebenfalls bevorzugt ist ein Verfahren zur Herstellung von Verbindungen der Formel VI, worin X Chlor, Brom oder Iod bedeutet, dadurch gekennzeichnet, dass eine Verbindung der Formel VIII, worin $R^1$ und $R^2$ die obengenannten Bedeutungen haben, $R^d$ und $R^e$ unabhängig voneinander Niederalkyl, Cycloalkyl, Arylniederalkyl oder zusammen Niederalkylen, Oxaniederalkylen oder Niederalkylazaniederalkylen mit 4 bis 6 C-Atomen in der Kette, wobei jedoch Aza nicht in 1-Stellung steht, oder $R^d$ Niederalkyl und $R^e$ Niederalkoxy bedeuten, mit einem Halogenierungsmittel, z.B. N-Chlorsuccinimid, N-Bromsuccinimid, Iod oder N-Iodsuccinimid, und, wenn $R^e$ verschieden von Niederalkoxy und $R^d$ und $R^e$ zusammen verschieden von 1-Oxaniederalkylen sind, in Gegenwart einer schwachen Säure, z.B. einer Niederalkancarbonsäure, in einem inerten organischen Lösungsmittel oder einem organischen oder wässrig-organischen Lösungsmittelgemisch bei Temperaturen zwischen -20° und 80°C umgesetzt wird.

Ganz besonders bevorzugt ist ein Verfahren zur Herstellung von Verbindungen der Formel VI, worin X Brom bedeutet, dadurch gekennzeichnet, dass eine Verbindung der Formel VIII, worin $R^1$ und $R^2$ die obengenannten Bedeutungen haben und $R^d$ und $R^e$ gleiches oder verschiedenes Niederalkyl oder zusammen Niederalkylen oder Oxaniederalkylen mit 4 bis 6 Atomen in der Kette darstellen, mit N-Bromsuccinimid in Gegenwart einer schwachen Säure, z.B. einer Niederalkancarbonsäure, z.B. Essigsäure, in einem wässrig-organischen Lösungsmittelgemisch, z.B. wässrigem Tetrahydrofuran, bei Temperaturen um 0°C umgesetzt wird.

Gleichermassen bevorzugt ist ein Verfahren zur Herstellung von Verbindungen der Formel VI, worin X Brom bedeutet, dadurch gekennzeichnet, dass eine Verbindung der Formel VIII, worin $R^1$ und $R^2$ die obengenannten Bedeutungen haben, $R^d$ Niederalkyl und $R^e$ Niederalkoxy oder $R^d$ und $R^e$ zusammen 1-Oxaniederalkylen mit 4 bis 6 Atomen in der Kette bedeuten, mit N-Bromsuccinimid in einem wässrig-organischen Lösungsmittelgemisch, z.B. wässrigem Tetrahydrofuran, bei Temperaturen um 0°C umgesetzt wird.

In allen genannten Verfahren werden vorzugsweise solche Ausgangsstoffe verwendet und die Reaktionsbedingungen so gewählt, dass man zu den eingangs als besonders bevorzugt aufgeführten Verbindungen gelangt.

Ausgangsstoffe der Formel II sind bekannt oder können, wenn sie neu sind, analog bekannten Vefahren hergestellt werden, beispielsweise durch Grignardaddition von Vinylmagnesiumbromid an einen Aldehyden der Formel $R^1CHO$ oder durch Reduktion eines Ethinylketons $R^1COC{\equiv}CH$.

Säuren und Ester der Formel III sind ebenfalls bekannt oder können nach bekannten Methoden hergestellt werden. Ist $R^a$ eine Gruppe $COOR^b$, so erhält man die entsprechenden Verbindungen der Formel III durch Reaktion des unsubstituierten Malonesters mit einem den Rest $R^4$ einführenden Alkylierungsmittel oder, wenn $R^4$ Halogen, gegebenenfalls substituiertes Hydroxy oder Amino bedeutet, mit dem

entsprechenden Halogenierungs-, Oxidations- oder Aminierungsmittel. Durch Hydrolyse und Decarboxylierung erhält man auf bekannte Art und Weise aus Malonestern der Formel III, worin $R^a$ eine Gruppe $COOR^b$ ist, die entsprechenden Säuren oder Ester der Formel III, worin $R^a$ Wasserstoff bedeutet. Ist $R^4$ Halogen, gegebenenfalls substituiertes Hydroxy oder Amino, so werden die entsprechenden Verbindungen der Formel III, worin $R^a$ Wasserstoff ist, nach den üblichen Methoden zur Herstellung von $\alpha$-Halo-, $\alpha$-Hydroxy- und $\alpha$-Amino-Carbonsäuren und Derivaten davon erhalten.

Die Erfindung betrifft ausserdem die im beschriebenen Verfahren verwendeten neuen Zwischenprodukte, insbesondere die neuen Verbindungen der Formeln IV, V, VI, VIII und IX.

Beispielsweise betrifft die Erfindung Verbindungen der Formel

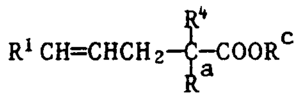 (IV),

worin $R^1$ Cycloalkylniederalkyl, $R^4$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl, Halogen oder den Rest einer natürlichen Aminosäure ausser Glycin, $R^a$ Wasserstoff und $R^c$ Wasserstoff, einen Kohlenwasserstoffrest oder einen Silylrest bedeuten, und Salze von solchen Verbindungen mit salzbildenden Gruppen.

Bevorzugt sind Verbindungen der Formel IV, worin $R^1$ die unter Formel I als bevorzugt genannten Reste und $R^4$ die unter Formel I für $R^2$ als bevorzugt genannten Reste oder Halogen bedeuten. Besonders bevorzugt sind Verbindungen der Formel IV, worin $R^c$ Wasserstoff bedeutet, Verbindungen der Formel V, ferner Salze davon, reine Enantiomere und Salze reiner Enantiomere.

Salze von Verbindungen der Formeln IV und V sind beispielsweise die weiter vorn unter Formel I genannten Säureadditionssalze, falls $R^2$ oder $R^4$ gegebenenfalls substituiertes Amino bedeutet, ferner Salze der Carboxygruppe in Verbindungen der Formel IV, in denen $R^b$ und/oder $R^c$ Wasserstoff bedeuten, z.B. wie in Verbindungen der Formel V, in erster Linie Alkalimetall-, z.B. Natrium- oder Kaliumsalze, Erdalkalimetall-, z.B. Magnesium- oder Calciumsalze, ferner Zinksalze oder gegebenenfalls substituierte Ammoniumsalze, beispielsweise Salze mit organischen Aminen, z.B. mit den weiter oben genannten, für die Racematspaltung geeigneten chiralen Aminen, oder mit achiralen, gegebenenfalls durch Hydroxy substituierten Mono-, Di- oder Trialkylaminen, z.B. mit Diethylamin, D-(2-hydroxyethyl)-amin, Triethylamin, Tris-(hydroxymethyl)methylamin oder Tri-(2-hydroxyethyl)-amin,mit Cycloalkylaminen, z.B. Cyclohexylamin, Dicyclohexylamin oder Ethylcyclohexylamin, oder mit Arylniederalkylaminen, z.B. mit Benzylamin, ferner tetrasubstituierte organische Ammoniumsalze, z.B. Tetramethyl-, Tetrabutyl- oder Benzyltrimethylammoniumsalze.

Weiter betrifft die Erfindung Verbindungen der Formel

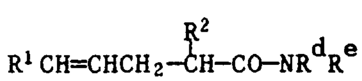 (VIII),

worin $R^1$ gegebenenfalls substituiertes Alkyl mit 2 und mehr Kohlenstoffatomen, Cycloalkyl, Cycloalkylniederalkyl, Aryl, Arylniederalkyl oder den Rest einer natürlichen Aminosäure ausser Glycin und Alanin, $R^2$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure ausser Glycin und $R^d$ und $R^e$ unabhängig voneinander Niederalkyl, Cycloalkyl oder Arylniederalkyl, $R^d$ und $R^e$ zusammen Niederalkylen, Oxaniederalkylen oder Niederalkylazaniederalkylen mit 4 bis 6 C-Atomen in der Kette, wobei Aza nicht in 1-Stellung steht, oder $R^d$ Niederalkyl und $R^e$ Niederalkoxy bedeuten, und Salze von solchen Verbindungen, in denen $R^2$ gegebenenfalls substituiertes Amino bedeutet.

Bevorzugt sind Verbindungen der Formel VIII, worin $R^1$ und $R^2$ die unter Formel I als bevorzugt genannten Rest sind. Ebenfalls bevorzugt sind Verbindungen, in denen $R^d$ Niederalkyl und $R^e$ Niederalkyl oder Niederalkoxy oder $R^d$ und $R^e$ zusammen Niederalkylen oder Oxaniederalkylen mit 4 bis 6 Atomen in der Kette darstellen, und reine Enantiomere davon.

Salze von Verbindungen der Formel VIII, worin $R^2$ gegebenenfalls substituiertes Amino bedeutet, sind beispielsweise die weiter vorn unter Formel I genannten Säureadditionssalze.

Die Erfindung betrifft ebenfalls Verbindungen der Formel

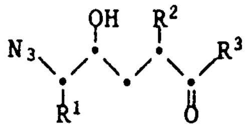

(IX),

worin $R^1$ Wasserstoff, gegebenenfalls substituiertes Alkyl, Cycloalkyl, Cycloalkylniederalkyl, Aryl, Arylniederalkyl oder den Rest einer natürliche Aminosäure, $R^2$ Wasserstoff, gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Amino darstellen, und Salze von solchen Verbindungen mit salzbildenden Gruppen.

Bevorzugt sind Verbindungen der Formel IX, worin $R^1$, $R^2$ und $R^3$ die unter Formel I als bevorzugt genannten Reste sind, ferner reine Diastereomere oder Enantiomere dieser Verbindungen. Besonders bevorzugt sind Diastereomere der genannten Verbindungen, worin die Kohlenstoffatome, die die Reste $N_3$ und OH tragen, die R- und R-Konfiguration oder die S- und S-Konfiguration aufweisen, ferner Enantiomere, worin diese Kohlenstoffatome beide die S-Konfiguration aufweisen.

Salze von Verbindungen der Formel IX mit salzbildenden Gruppen sind beispielsweise die weiter vorn unter Formel I genannten Säureadditionssalze von Verbindungen, worin $R^2$ gegebenenfalls substituiertes Amino ist und/oder worin der Rest $R^3$ eine Aminogruppe enthält.

In erster Linie betrifft die Erfindung die in den Beispielen genannten Zwischenprodukte der Formeln IV, V, VI, VIII und IX.

Die folgenden Beispiele dienen zur Illustration der Erfindung, schränken deren Umfang jedoch in keiner Weise ein.

Temperaturen werden in Celsiusgraden angegeben. Die Werte für Proton-Kernresonanzspektroskopie ($^1$H-NMR) werden im ppm (parts per million) bezogen auf Tetramethylsilan ($\delta$ = 0) als internen Standard angegeben. s = Singulett, d = Dublett, t = Triplett, q = Quartett, m = Multiplett, dd = Doppeldublett. Kopplungswerte J in Hertz (Hz). Bei Elementaranalysen sind die Summenformel, das Molekulargewicht, berechnete (ber.) und gefundene (gef.) Analysenwerte angegeben.

Optische Drehungen $[\alpha]_D$ werden bei der Natrium-D-Linie gemessen, c = Konzentration (g/100 ml).

Abkürzungen:

| | |
|---|---|
| abs. | absolut |
| ee | Enantiomerenüberschuss (enantiomeric excess) |
| ges. | gesättigt |
| RT | Raumtemperatur |
| RV | Rotationsverdampfer |
| Smp. | Schmelzpunkt |
| THF | Tetrahydrofuran |

Beispiel 1: 1-Cyclohexyl-3-buten-2-ol

Zu einer Lösung von Vinylmagnesiumbromid in abs. THF hergestellt aus 13,8 g (0,57 Mol) Magnesium und 66,3 g (0,62 Mol) Vinylbromid in 500 ml abs. THF wird bei -20° eine Lösung von 60 g (0,47 Mol) Cyclohexylacetaldehyd in 400 ml abs. THF zugetropft. Nach beendeter Zugabe wird 30 Min. bei -20° gerührt und anschliessend mit 900 ml ges. Ammoniumchlorid-Lösung hydrolysiert. Nach Verdünnen mit 250 ml $H_2O$ wird mit dreimal 1 l Ether extrahiert, dreimal mit 500 ml Sole gewaschen, mit $MgSO_4$ getrocknet und anschliessend am RV eingedampft. Der Rückstand wird bei 50°/0,1 mbar destilliert.
$C_{10}H_{18}O$ (154,25): ber. C 77,87 H 11,77 %; gef. C 77,62 H 11,54 %.

Beispiel 2:

Analog Beispiel 1 werden hergestellt:

a) 1-Cyclopentyl-3-buten-2-ol aus Cyclopentylacetaldehyd.
$C_9H_{16}O$ (140,23): ber. C 77,09 H 11,50 %; gef. C 76,97 H 11,24 %.

b) 1-Cyclohexyl-4-penten-3-ol aus 3-Cyclohexylpropionaldehyd.
$C_{11}H_{20}O$ (168,28): ber. C 78,51 H 11,98 %; gef. C 78,70 H 11,97 %.
c) 1-Cycloheptyl-3-buten-2-ol aus Cycloheptylacetaldehyd.
$C_{11}H_{20}O$ (168,28): ber. C 78,51 H 11,98 %; gef. C 78,06 H 11,92 %.
d) 5-Methyl-1-hexen-3-ol aus Isovalerianaldehyd.
$C_7H_{14}O$ (114,19): ber. C 73,63 H 12,36 %; gef. C 73,79 H 12,57 %.

Beispiel 3: 16-Cyclohexyl-2-isopropyl-4-hexensäure

23,15 g (0,15 Mol) 1-Cyclohexyl-3-buten-2-ol, 6,85 g (0,03 Mol) Tetraethylorthotitanat und 61,5 ml (0,30 Mol) Isopropylmalonsäurediethylester werden in einen Kolben vorgelegt, 1 Std. bei 170° und anschliessend 24 Std. bei 200° gerührt. Ueberschüssiger Malonester wird bei 20 mbar abdestilliert, der Rückstand in 120 ml Ethanol gelöst und nach Zugabe von 120 ml 6N KOH 7 Std. am Rückfluss gerührt. Nach dem Abkühlen wird filtriert und das Filtrat am RV weitgehend eingedampft. Die wässrige Phase wird einmal mit Ether extrahiert, unter Eiskühlung mit 6N HCl auf pH 1 angesäuert und anschliessend dreimal mit Ether extrahiert. Die vereinigten organischen Phasen werden mit Sole gewaschen, mit $MgSO_4$ getrocknet und am RV eingedampft. Der Rückstand wird bei 137-140°/0,025 mbar destilliert.
$C_{15}H_{26}O_2$ (238,37): ber. C 75,58 H 11,00 %; gef. C 75,49 H 10,91 %.
$^1$H-NMR ($CDCl_3$): 5,28-5,55 (m, 2H, HC=CH).

Die Reaktion kann auch mit einem geringeren Ueberschuss an Isopropylmalonsäureester (1,1 statt 2,0 Aequivalente) und weniger Tetraethylorthotitanat (0,1 statt 0,2 Aequivalente) ohne wesentliche Ausbeuteverminderung durchgeführt werden.

Beispiel 4:

Analog Beispiel 3 werden hergestellt:
a) 6-Cyclopentyl-2-isopropyl-4-hexensäure, aus 1-Cyclopentyl-3-buten-2-ol und Isopropylmalonsäurediethylester.
$C_{14}H_{24}O_2$ (224,34): ber. C 74,96 H 10,79 %; gef. C 74,86 H 11,13 %.
$^1$H-NMR ($CDCl_3$): 5,3-5,55 (m, 2H, HC=CH).
b) 7-Cyclohexyl-2-isopropyl-4-heptensäure, aus 1-Cyclohexyl-4-penten-3-ol und Isopropylmalonsäurediethylester.
$C_{16}H_{28}O_2$ (252,40): ber. C 76,14 H 11,18 %; gef. C 76,24 H 11,23 %.
$^1$H-NMR ($CDCl_3$): 5,3-5,5 (m, 2H, HC=CH).
c) 6-Cycloheptyl-2-isopropyl-4-hexensäure, aus 1-Cycloheptyl-3-buten-2-ol und Isopropylmalonsäurediethylester.
$C_{16}H_{28}O_2$ (252,40): ber. C 76,14 H 11,18 %; gef. C 76,24 H 11,23 %.
$^1$H-NMR ($CDCl_3$): 5,3-5,5 (m, 2H, HC=CH).
d) 6-Cyclohexyl-2-methoxy-4-hexensäure, aus 1-Cyclopentyl-3-buten-2-ol und Methoxymalonsäurediethylester.
$C_{13}H_{22}O_3$ (226,32): ber. C 68,99 H 9,80 %; gef. C 68,80 H 9,99 %.
$^1$H-NMR ($CDCl_3$): 5,33-5,61 (m, 2H, HC=CH); 3,85 (dd, 1H, CHOR); 3,47 (s, 3H, $OCH_3$).
e) 6-Cyclohexyl-2-dimethylamino-4-hexensäure-ethylester, aus 1-Cyclohexyl-3-buten-2-ol und Dimethylaminomalonsäurediethylester, isoliert als Ester vor der Behandlung mit 6N KOH.
$^1$H-NMR ($CDCl_3$): 5,26-5,55 (m, 2H, HC=CH); 5,16 (q, 2H, $OCH_2$); 3,12 (dd, 1H, $CHNR_2$); 2,33 (s, 6H, $NCH_3$).
f) 2-Isopropyl-7-methyl-4-octensäure, aus 5-Methyl-1-hexen-3-ol und Isopropylmalonsäurediethylester.
$C_{12}H_{22}O_2$ (198,31): ber. C 72,63 H 11,18 %; gef. C 72,43 H 11,02 %.
$^1$H-NMR ($CDCl_3$): 5,16-5,45 (m, 2H, HC=CH).
g) 6-Cyclohexyl-2-phenyl-4-hexensäure-ethylester, aus 1-Cyclohexyl-3-buten-2-ol und Phenylmalonsäurediethylester, isoliert als Ester vor der Behandlung mit 6N KOH.
$C_{20}H_{28}O_2$ (300,44): ber. C 79,96 H 9,40 %; gef. C 80,00 H 9,56 %.
$^1$H-NMR ($CDCl_3$): 5,3-5,5 (m, 2H, HC=CH).
h) 6-Cyclohexyl-2-methyl-4-hexensäure-ethylester, aus 1-Cyclohexyl-3-buten-2-ol und Methylmalonsäurediethylester, isoliert als Ester vor der Behandlung mit 6N KOH.
$^1$H-NMR ($CDCl_3$): 2,38-2,51 (m, 1H, CHCOO); 5,3-5,5 (m, 2H, HC=CH).
i) 2-Brom-6-cyclohexyl-4-hexensäure-ethylester, aus 1-Cyclohexyl-3-buten-2-ol und Brommalonsäurediethylester, isoliert als Ester vor der Behandlung mit 6N KOH.

$^1H$-NMR ($CDCl_3$): 4,1-4,25 (m, 3H, $OCH_2$ und CHBrCOO); 5,25-5,6 (m, 2H, HC = CH).

j) 2-Chlor-6-cyclohexyl-4-hexensäure-ethylester, aus 1-Cyclohexyl-3-buten-2-ol und Chlormalonsäurediethylester, isoliert als Ester vor der Behandlung mit 6N KOH.

$^1H$-NMR ($CDCl_3$): 4,15-4,30 (m, 3H, $OCH_2$ und CHClCOO); 5,25-5,6 (m, 2H, HC = CH).

k) 2-tert-Butyldimethylsilyloxy-6-cyclohexyl-4-hexensäure-ethylester, aus 1-Cyclohexyl-3-buten-2-ol und tert-Butyldimethylsilyloxy-malonsäurediethylester, isoliert als Ester vor der Behandlung mit 6N KOH.

$^1H$-NMR ($CDCl_3$): 5,28-5,5 (m, 2H, HC = CH).

l) 6-Cyclohexyl-2-phenylthio-4-hexensäure-ethylester, aus 1-Cyclohexyl-3-buten-2-ol und Phenylthiomalonsäurediethylester, isoliert als Ester vor der Behandlung mit 6N KOH.

$^1H$-NMR ($CDCl_3$): 3,65 (t J = 6, 1H, CHCOO); 5,3-5,6 (m, 2H, HC = CH).

Beispiel 5: Isovaleriansäure-1-cyclohexyl-3-buten-2-yl-ester

Zu einer Lösung von 2,75 g (17,76 mMol) 1-Cyclohexyl-3-buten-2-ol, 3 ml (21,3 mMol)Triethylamin und 430 mg (3,5 mMol) 4-Dimethylaminopyridin in 20 ml Methylenchlorid werden bei 0 ° C 2,4 ml (19,5 mMol) Isovaleriansäurechlorid zugetropft. Nach beendeter Zugabe wird das Gemisch noch 3 Std. bei RT gerührt. Nach Verdünnen mit Ether wird je zweimal mit 2N HCl, ges. $NaHCO_3$-Lösung und Sole gewaschen und über $MgSO_4$ getrocknet. Nach dem Eindampfen wird der Rückstand im Kugelrohr bei 150 ° /0,007 mbar destilliert.

$^1H$-NMR ($CDCl_3$): 5,70-5,86 (m, 1H); 5,30-5,40 (m, 1H); 5,24 (d, 1H); 5,14 (d, 1H).

Beispiel 6: 6-Cyclohexyl-2-isopropyl-4-hexensäure (alternative Darstellungsweisen)

a) Zu einer Lösung von 0,39 ml (2,3 mMol) Cyclohexylisopropylamin in 4 ml abs. THF werden bei -20 ° 1,4 ml (2,3 mMol) 1,6 N n-Butyllithium in Hexan zugetropft. Nach 15 Min. bei -20 ° wird auf -78 ° abgekühlt und 500 mg (2,1 mMol) Isovaleriansäure-1-cyclohexyl-3-buten-2-yl-esterin 1 ml abs. THF zugetropft. Nach beendeter Zugabe wird innerhalb von 1 Std. auf RT erwärmt und anschliessend 2 Std. bei dieser Temperatur gerührt. Das Reaktionsgemisch wird in 5 ml kalte 1N NaOH eingetragen und zweimal mit Ether extrahiert. Die wässrige Phase wird mit konz. HCl auf pH 1 angesäuert und mit Ether extrahiert. Die organischen Phasen werden mit Sole gewaschen, mit $MgSO_4$getrocknet und eingedampft. Der Rückstand wird im Kugelrohr bei 150 ° /0,01 mbar destilliert und ergibt dabei ein Produkt, das mit der Verbindung von Beispiel 3 identisch ist.

b) Zu einer wie unter a) zubereiteten Lösung von 6 mMol Lithiumcyclohexylisopropylamid in abs. THF werden bei -78 ° zuerst 2,3 ml (18 mMol) Trimethylchlorsilan und anschliessend 1,2 g (5 mMol) Isovaleriansäure-1-cyclohexyl-3-buten-2-yl-ester zugetropft. Die Lösung wird 1 Std. bei -78 ° gehalten, innerhalb von 1 1/2 Std. auf RT erwärmt und 1 Std. bei dieser Temperatur gerührt. Das Reaktionsgemisch wird auf 0 ° gekühlt, durch Zugabe von 0,5 ml Methanol hydrolysiert, mit Ether verdünnt, mit 1 N HCl und Sole gewaschen, über $MgSO_4$ getrocknet und am RV eingedampft. Der Rückstand wird wie unter a) destilliert und ergibt das gleiche Produkt.

Beispiel 7: Enantioselektive Herstellung von 6-Cyclohexyl-2(S)-isopropyl-4-hexensäure

a) 1-Cyclohexyl-3-butin-2(S)-ol: Nach einer Vorschrift von D.R.M. Walton und F. Waugh, J. Organomet. Chem. 37, 45 (1972) wird Bis(trimethylsilyl)acetylen mit Cyclohexylessigsäurechlorid acyliert und mit wässrig/methanolischer Boraxlösung zu 1-Cyclohexyl-3-butin-2-on hydrolysiert. Reduktion mit (S)-B-(3-Pinanyl)-9-borabicyclo-[3.3.1]nonan ((S)-Alpine-Borane®, Aldrich, 88 % ee) nach der Vorschrift von M.M. Midland et al., Tetrahedron 40, 1371 (1984) ergibt 1-Cyclohexyl-3-butin-2(S)-ol.

$C_{10}H_{16}O_2$ (152,24): ber. C 78,90 H 10,60 %; gef. C 78,73 H 10,83 %.

$^1H$-NMR ($CDCl_3$): 0,83-2,0 (m, 14H); 2,45 (d J = 2, 1H, HC≡C); 4,45 (dxt, J = 8 und 2, 1H, CHO).

$[\alpha]_D$ = -8,1 ° (c = 0,9, $CHCl_3$). Der Enantiomerenüberschuss wird mit $^1H$-NMR nach Derivatisierung mit dem Mosher-Reagens R( + )-α-Methoxy-α-trifluormethyl-phenylessigsäurechlorid bestimmt und beträgt 80 % ee.

b) Isovaleriansäure-1-cyclohexyl-3-buten-2(S)-yl-ester:

1-Cyclohexyl-3-butin-2(S)-ol wird durch partielle Hydrierung in Gegenwart von Lindlar-Katalysator zu 1-Cyclohexyl-3-buten-2(S)-ol reduziert und analog Beispiel 5 mit Isovaleriansäurechlorid umgesetzt.

| $C_{15}H_{26}O_2$ (283,37): | | | |
|---|---|---|---|
| ber.<br>gef. | C 75,58<br>C 75,40 | H 11,00<br>H 11,26 | O 13,43 %<br>O 13,41 %. |

$[\alpha]_D$ = -13,6° (c = 1, $CHCl_3$), 80 % ee.

c) Umlagerung des Silylenolats: Zu einer wie in Beispiel 6a zubereiteten Lösung von 6 mMol Lithiumcyclohexylisopropylamid in abs. THF werden bei -78° zuerst 2,3 ml (18 mMol) Trimethylchlorsilan und dann 1,2 g (5 mMol) Isovaleriansäure-1-cyclohexyl-3-buten-2(s)-yl-ester zugetropft. Die Lösung wird wie in Beispiel 6b weiterverarbeitet. Es wird 6-Cyclohexyl-2(S)-isopropyl-4-hexensäure von 78 % ee erhalten.

Beispiel 8: 6-Cyclohexyl-2(R)- und 2(S)-isopropyl-4-hexensäure

154,65 g (0,65 Mol) racemische 6-Cyclohexyl-2-isopropyl-4-hexensäure und 210,5 g (0.65 Mol) wasserfreies Chinin werden in 1 l Methanol gelöst. Das Gemisch wird eingedampft und der Rückstand aus Ether/Hexan umkristallisiert. Das Kristallisat wird abgenutscht und gründlich mit kaltem Hexan gewaschen. Das Salz wird mit 1 N HCl behandelt und die Säure mit Ether extrahiert. Durch Eindampfen erhält man 6-Cyclohexyl-2(R)-isopropyl-4-hexensäure von 90 % ee. Wird das Chininsalz vor der Behandlung mit HCl ein zweites Mal aus Ether/Hexan umkristallisiert, so weist die daraus erhältliche (R)-Säure eine Reinheit von über 95 % ee auf.

Das Filtrat der ersten Kristallisation des Chininsalzes wird eingedampft und ebenfalls mit 1 N HCl behandelt. Die Lösung wird mit Ether extrahiert und der Extrakt eingedampft, wobei 6-Cyclohexyl-2(S)-isopropyl-4-hexensäure von 80 % ee zurückbleibt. Zur weiteren Anreicherung der (S)-Säure wird dieses Produkt mit einer äquimolaren Menge von (+)-Dehydroabietylamin in Methanol versetzt, eingedampft und aus Methylenchlorid/Hexan umkristallisiert. Das Kristallisat wird mit 1 N HCl behandelt und die Säure mit Ether extrahiert. Durch Eindampfen des Etherlösung erhält man die (S)-Säure in einer Reinheit von 95 % ee.

Zur Bestimmung des Enantiomerenüberschusses (ee) wird jeweils eine Probe der Säure mit (+)-Phenylethylamin in Gegenwart von Dicyclohexylcarbodiimid und 1-Hydroxybenztriazol kondensiert und das Verhältnis der gebildeten diastereomeren Amide durch HPLC bestimmt.

Beispiel 9:

Analog Beispiel 8 werden hergestellt:

a) 6-Cyclopentyl-2(R)- und 2(S)-isopropyl-4-hexensäure

b) 7-Cyclohexyl-2(R)- und 2(S)-isopropyl-4-heptensäure

c) 6-Cycloheptyl-2(R)- und 2(S)-isopropyl-4-hexensäure

Beispiel 10: 6-Cyclohexyl-2(S)-isopropyl-4-hexensäure-dimethylamid

Zu einer Lösung von 58,1 g (0,244 Mol) 6-Cyclohexyl-2(S)-isopropyl-4-hexensäure in 270 ml abs. Toluol und 0,5 ml DMF werden 42,6 ml (0,49 Mol) Oxalylchlorid zudosiert. Nach beendeter Zugabe wird 90 Min. unter Rückfluss gekocht, anschliessend das Reaktionsgemisch am RV eingeengt. Der Rückstand wird in 270 ml trockenem $CH_2Cl_2$ gelöst und bei 0° zu einer Lösung von 17,2 g (0,38 Mol) Dimethylamin und 61,4 ml (0,76 Mol) Pyridin in 270 ml $CH_2Cl_2$ zugetropft. Nach 30 Min. Rühren bei 0° wird mit Ether verdünnt und je zweimal mit 2N HCl, ges. $NaHCO_3$-Lösung und Sole gewaschen. Die vereinigten organischen Phasen werden mit $MgSO_4$ getrocknet und am RV eingedampft. Der Rückstand wird bei 124-128°/0,04 mbar destilliert.

$^1$H-NMR ($CDCl_3$): 5,21-5,50 (m, 2H, HC=CH); 3,04 (s, 3H, $NCH_3$); 2,95 (s, 3H, $NCH_3$).

Beispiel 11:

Analog Beispiel 10 werden hergestellt:

a) 6-Cyclopentyl-2(S)-isopropyl-4-hexensäure-dimethylamid

$^1$H-NMR ($CDCl_3$): 5,25-5,50 (m, 2H, HC=CH); 3,03 (s, 3H, $NCH_3$); 2,96 (s, 3H, $NCH_3$).

b) 7-Cyclohexyl-2(S)-isopropyl-4-heptensäure-dimethylamid

$^1$H-NMR ($CDCl_3$): 5,25-5,50 (m, 2H, HC=CH); 3,03 (s, 3H, $NCH_3$); 2,96 (s, 3H, $NCH_3$).

c) 6-Cycloheptyl-2(S)-isopropyl-4-hexensäure-dimethylamid
$^{1}$H-NMR ($CDCl_3$): 5,25-5,50 (m, 2H, HC=CH); 3,05 (s, 3H, $NCH_3$); 2,96 (s, 3H, $NCH_3$).
d) 6-Cyclohexyl-2-methoxy-4-hexensäure-dimethylamid
$^{1}$H-NMR ($CDCl_3$): 5,33-5,58 (m, 2H, HC=CH); 5,03-5,1 (m, 1H, CHOR); 3,33 (s, 3H, $OCH_3$); 3,1 (s, 3H, $NCH_3$); 2,95 (s, 3H, $NCH_3$).
e) 6-Cyclohexyl-2-dimethylamino-4-hexensäure-dimethylamid
$^{1}$H-NMR ($CDCl_3$): 5,15-5,50 (m, 2H, HC=CH); 4,37-4,48 (m, 1H, $CHNR_2$); 3,1 (s, 3H, $NCH_3$); 2,95 (s, 3H, $NCH_3$); 2,2 (s, 6H, $NCH_3$).
f) 2-Isopropyl-7-methyl-4-octensäure-dimethylamid
$^{1}$H-NMR ($CDCl_3$): 5,25-5,50 (m, 2H, HC=CH); 3,03 (s, 3H, $NCH_3$); 2,96 (s, 3H, $NCH_3$).
g) 6-Cyclohexyl-2(S)-isopropyl-4-hexensäure-diethylamid
$^{1}$H-NMR ($CDCl_3$): 5,23-5,50 (m, 2H, HC=CH); 3,25-3,50 (m, 4H, $NCH_2$).
h) 6-Cyclohexyl-2(S)-isopropyl-4-hexensäure-pyrrolidid
$^{1}$H-NMR ($CDCl_3$): 5,23-5,50 (m, 2H, HC=CH); 3,48 (t, 2H, $NCH_2$); 3,35 (t, 2H, $NCH_2$).
i) 6-Cyclohexyl-2(S)-isopropyl-4-hexensäure-morpholid
$^{1}$H-NMR ($CDCl_3$): 5,20-5,50 (m, 2H, HC=CH); 3,50-3,72 (m, 8H, $NCH_2CH_2O$).
j) 6-Cyclohexyl-2(S)-isopropyl-4-hexensäure-N-methoxy-N-methyl-amid
$^{1}$H-NMR ($CDCl_3$): 5,25-5,50 (m, 2H, HC=CH); 3,65 (s, 3H, $OCH_3$); 3,20 (s, 3H, $NCH_3$).
k) 6-Cyclohexyl-2(S)-isopropyl-4-hexensäure-N-isopropyl-N-methoxyamid
$^{1}$H-NMR ($CDCl_3$): 5,25-5,50 (m, 2H, HC=CH); 4,55-4,70 (m, 1H, NCH); 3,75 (s, 3H, $OCH_3$).
l) 6-Cyclohexyl-2(S)-isopropyl-4-hexensäure-N-isopropoxy-N-methylamid
$^{1}$H-NMR ($CDCl_3$): 5,25-5,50 (m, 2H, HC=CH); 4,0-4,2 (m, 1H, OCH); 3,2 (s, 3H, $NCH_3$).
m) 6-Cyclohexyl-2(S)-isopropyl-4-hexensäure-tetrahydroisoxazolid
$^{1}$H-NMR ($CDCl_3$): 5,25-5,50 (m, 2H, HC=CH); 3,8-4,0 (m, 2H, $OCH_2$); 3,56-3,80 (m, 2H, $NCH_2$).

Beispiel 12: 5(R)-Brom-6-cyclohexyl-2(S)-isopropyl-4(S)-hexanolid

88,5 g (0,5 Mol) N-Bromsuccinimid und 30 g (0,5 Mol) Eisessig in 1,1 l THF werden bei 0° innerhalb von 8 Std. zu einer Lösung von 60 g (0,23 Mol) 6-Cyclohexyl-2(S)-isopropyl-4-hexensäuredimethylamid in 1,4 l THF/$H_2O$ (2:1) zugetropft. Nach beendeter Zugabe wird 30 Min. bei 0° gerührt, dann das Reaktionsgemisch auf 1,8 l eiskalte 40 % Natriumhydrogensulfit-Lösung gegossen. Die wässrige Phase wird dreimal mit Ether extrahiert und die vereinigten organischen Phasen nacheinander mit 1N HCl, ges. $NaHCO_3$-Lösung und Sole gewaschen. Nach dem Trocknen mit $MgSO_4$ und Abdampfen des Lösungsmittels wird der Rückstand über Kieselgel mit Hexan/Ether (2:1) chromatographiert und erneut eingeengt. Der Rückstand wird aus Hexan umkristallisiert, Smp. 95-95,5°.
$C_{15}H_{25}BrO_2$ (317,27): ber. C 56,79 H 7,94 Br 25,19 %; gef. C 56,94 H 8,04 Br 25,22 %.

Auf gleiche Art und Weise werden anstelle des Dimethylamids auch das Diethylamid, Pyrrolidid und Morpholid einer Bromlactonisierung unterworfen.

Werden Hydroxamsäureester, z.B. 6-Cyclohexyl-2(S)-isopropyl-4-hexensäure-N-methoxy-N-methylamid, das entsprechende -N-isopropyl-N-methoxyamid, -N-isopropoxy-N-methylamid oder -tetrahydroisoxazolid, eingesetzt, so wird die Bromlactonisierung ohne Eisessig durchgeführt.

Beispiel 13:

Analog Beispiel 12 werden hergestellt:
a) 5(R)-Brom-6-cyclopentyl-2(S)-isopropyl-4(S)-hexanolid
Smp. 66-68°.

| $C_{14}H_{23}BrO_2$ (303,24): | | | |
|---|---|---|---|
| ber.<br>gef. | C 55,46<br>C 55,81 | H 7,65<br>H 7,48 | Br 26,35 %;<br>Br 26,29 %. |

b) 5(R)-Brom-7-cyclohexyl-2(S)-isopropyl-4(S)-heptanolid
Smp. 64-65°.

| $C_{16}H_{27}BrO_2$ (331,29): | | | |
|---|---|---|---|
| ber.<br>gef. | C 58,01<br>C 58,32 | H 8,22<br>H 8,19 | Br 24,12 %;<br>Br 24,03 %. |

c) 5(R)-Brom-6-cycloheptyl-2(S)-isopropyl-4(S)-hexanolid
Smp. 57-58°.

| $C_{16}H_{27}BrO_2$ (331,29): | | | |
|---|---|---|---|
| ber.<br>gef. | C 58,01<br>C 57,99 | H 8,22<br>H 8,17 | Br 24,12 %;<br>Br 24,10 %. |

d) rel(2R,4S,5R)-5-Brom-6-cyclohexyl-2-methoxy-4-hexanolid
$^1$H-NMR ($CDCl_3$): 4,57-4,65 (m, 1H); 4,17-4,23 (m, 1H); 4,06-4,10 (m, 1H); 3,57 (s, 3H, $OCH_3$).
e) rel(2R,4S,5R)-5-Brom-6-cyclohexyl-2-dimethylamino-4-hexanolid
$^1$H-NMR ($CDCl_3$): 4,48-4,58 (m, 1H); 4,14-4,24 (m, 1H); 3,7 (t, 1H, $CHNR_2$); 2,4 (s, 6H, $NCH_3$).
f) rel(2R,4R,5S)-5-Brom-2-isopropyl-7-methyl-4-octanolid
$^1$H-NMR ($CDCl_3$): 4,40-4,50 (m, 1H); 4,08-4,17 (m, 1H); 2,63-2,75 (m, 1H, CHCO).

Beispiel 14: 5(S)-Azido-6-cyclohexyl-2(S)-isopropyl-4(S)-hexanolid

42,5 g (0,135 Mol) 5(R)-Brom-6-cyclohexyl-2(S)-isopropyl-4(S)-hexanolid und 35 g (0,54 Mol) $NaN_3$ in 480 ml N,N'-Dimethyl-N,N'-propylenharnstoff (DMPU) werden 72 Std. bei RT gerührt und anschliessend auf 1 l eiskalte 0,1 N HCl gegossen. Sodann wird dreimal mit Ether extrahiert, die vereinigten organischen Phasen zweimal mit 0,1 N HCl und dreimal mit Sole gewaschen und mit $MgSO_4$ getrocknet. Nach dem Eindampfen des Lösungsmittels wird der Rückstand aus Hexan umkristallisiert: Smp. 38,5-39°.
$C_{15}H_{25}N_3O_2$ (279,38): ber. C 64,49 H 9,02 N 15,04 %; gef. C 64,53 H 8,96 N 15,04 %.

Beispiel 15:

Analog Beispiel 14 werden hergestellt:
a) 5(S)-Azido-6-cyclopentyl-2(S)-isopropyl-4(S)-hexanolid, Oel.
$C_{14}H_{23}N_3O_2$ (265,36): ber. C 63,37 H 8,74 N 15,84 %; gef. C 63,36 H 8,92 N 15,59 %.
b) 5(S)-Azido-7-cyclohexyl-2(S)-isopropyl-4(S)-heptanolid, Oel.
$C_{16}H_{27}N_3O_2$ (293,41): ber. C 65,50 H 9,28 N 14,32 %; gef. C 65,57 H 9,40 N 14,12 %.
c) 5(S)-Azido-6-cycloheptyl-2(S)-isopropyl-4(S)-hexanolid,
Smp. 48-49°.
$C_{16}H_{27}N_3O_2$ (293,41): ber. C 65,50 H 9,28 N 14,32 %; gef. C 64,48 H 9,21 N 14,37 %.
d) rel(2R,4R,5R)-5-Azido-2-isopropyl-7-methyl-4-octanolid, Oel.
$^1$H-NMR ($CDCl_3$): 4,40-4,48 (m, 1H); 3,33-3,42 (m, 1H); 2,67-2,77 (m, 1H, CHCO).

Beispiel 16: 5(S)-Azido-6-cyclohexyl-4(S)-hydroxy-2(S)-isopropyl-hexansäure-n-butylamid

24,9 g (0,09 Mol) 5(S)-Azido-6-cyclohexyl-2(S)-isopropyl-4(S)-hexanolid in 250 ml n-Butylamin werden 10 Std. unter Rückfluss gekocht. Nach dem Abdampfen des Lösungsmittels wird der Rückstand aus Hexan umkristallisiert: Smp. 100-100,5°.
$C_{19}H_{36}N_4O_2$ (352,52): ber. C 64,74 H 10,29 N 15,89 %; gef. C 65,06 H 10,40 N 15,89 %.

Beispiel 17:

Analog Beispiel 16 werden hergestellt:
a) 5(S)-Azido-6-cyclopentyl-4(S)-hydroxy-2(S)-isopropyl-hexansäure-n-butylamid, Smp. 59-61°.
$C_{18}H_{34}N_4O_2$ (338,50): ber. C 63,87 H 10,13 N 16,55 %; gef. C 63,86 H 10,00 N 16,76 %.
b) 5(S)-Azido-7-cyclohexyl-4(S)-hydroxy-2(S)-isopropyl-heptansäure-n-butylamid, Smp. 99,5-100°.
$C_{20}H_{38}N_4O_2$ (366,55): ber. C 65,53 H 10,45 N 15,28 %; gef. C 65,72 H 10,46 N 15,28 %.

c) 5(S)-Azido-6-cycloheptyl-4(S)-hydroxy-2(S)-isopropyl-hexansäure-n-butylamid, Smp. 66,5-67°.
$C_{20}H_{38}N_4O_2$ (366,55): ber. C 65,53 H 10,45 N 15,28 %; gef. C 65,47 H 10,26 N 15,25 %.
d) 5(S)-Azido-6-cyclohexyl-4(S)-hydroxy-2(S)-isopropyl-hexansäuremethylamid aus 5(S)-Azido-6-cyclohexyl-2(S)-isopropyl-4(S)-hexanolid und Methylamin in DMF bei Raumtemperatur.
$^1$H-NMR ($CDCl_3$): 5,72 (q, 1H, NH); 3,42-3,52 (m, 1H); 3,23-3,32 (m, 1H); 2,83 (d, 3H, $NCH_3$).

Beispiel 18: 5(S)-Amino-6-cyclohexyl-4(S)-hydroxy-2(S)-isopropylhexansäure-n-butylamid

21,3 g (0,06 Mol) 5(S)-Azido-6-cyclohexyl-4(S)-hydroxy-2(S)-isopropyl-hexansäure-n-butylamid in 240 ml Methanol werden in Gegenwart von 5 g 10 % Pd/C 2 Std. hydriert. Das Reaktionsgemisch wird filtriert, am RV eingeengt und der Rückstand aus Hexan umkristallisiert: Smp. 88,5-90°; $[\alpha]_D$-27,2° ± 0,9° (c = 1,1 $CHCl_3$).
$C_{19}H_{38}N_2O_2$ (326,53): ber. C 69,89 H 11,73 N 8,58 %; gef. C 70,11 H 11,54 N 8,65 %.

Beispiel 19:

Analog Beispiel 18 werden hergestellt:
a) 5(S)-Amino-6-cyclopentyl-4(S)-hydroxy-2(S)-isopropyl-hexansäure-n-butylamid, Smp. 93-94°, $[\alpha]_D$-23,8° ± 0,9° (c = 1,1, $CHCl_3$).
$C_{18}H_{36}N_2O_2$ (312,50): ber. C 69,19 H 11,61 N 8,96 %; gef. C 69,13 H 11,54 N 8,94 %.
b) 5(S)-Amino-7-cyclohexyl-4(S)-hydroxy-2(S)-isopropyl-heptansäure-n-butylamid, Smp. 90-92°, $[\alpha]_D$-18,5° ± 0,9° (c = 1,1 $CHCl_3$).
$C_{20}H_{40}N_2O_2$ (340,55): ber. C 70,54 H 11,84 N 8,23 %; gef. C 70,54 H 11,62 N 8,07 %.
c) 5(S)-Amino-6-cycloheptyl-4(S)-hydroxy-2(S)-isopropyl-hexansäure-n-butylamid, Smp. 81-82°, $[\alpha]_D$-25,4° ± 0,9° (c = 1,1, $CHCl_3$).
$C_{20}H_{40}N_2O_2$ (340,55): ber. C 70,54 H 11,84 N 8,23 %; gef. C 70,33 H 11,72 N 8,46 %.
d) 5(S)-Amino-6-cycloheptyl-4(S)-hydroxy-2(S)-isopropyl-hexansäuremethylamid, farbloses Oel, $R_f$ - (Methylenchlorid-Methanol-Ammoniak konz. 350:50:1) = 0,11.

Beispiel 20: 5(S)-Benzyloxycarbonylamino-6-cyclohexyl-2(S)-isopropyl-4(S)-hexanolid

403 mg (1,44 mMol) 5(S)-Azido-6-cyclohexyl-2(S)-isopropyl-4(S)-hexanolid in 20 ml Methanol werden in Gegenwart von 200 mg 10 % Pd-C hydriert, wobei durch Zugabe von 1 N HCl der pH konstant bei 4 gehalten wird. Der Katalysator wird abfiltriert, mit Methanol gewaschen und das Filtrat am RV eingedampft. Der erhaltene Rückstand wird in 10 ml Essigester bei 0° vorgelegt, zuerst mit 3,60 ml 1 M $NaHCO_3$-Lösung und anschliessend mit 0,59 ml einer Lösung von Chlorameisensäurebenzylester 50 % in Toluol versetzt. Nach beendeter Zugabe wird das Gemisch noch 15 Min. bei 0° gerührt, dann zweimal mit Essigester extrahiert. Die vereinigten organischen Phasen werden mit Sole gewaschen, mit $MgSO_4$ getrocknet und am RV eingedampft. Der Rückstand wird aus Essigester/Hexan umkristallisiert:
Smp. 167,5 - 168°; $[\alpha]_D$ -31,4° ±0,9° (c = 1, $CHCl_3$).

| $C_{23}H_{33}NO_4$ (387,52): | | | |
|---|---|---|---|
| ber.<br>gef. | C 71.29<br>C 71.56 | H 8,58<br>H 8.64 | N 3.61 %;<br>N 3.52 %. |

**Patentansprüche**

**Patentansprüche für folgende Vertragsstaaten : BE, CH, DE, FR, GB, IT, LI, NL, SE**

**1.** Verfahren zur Herstellung von Verbindungen der Formel

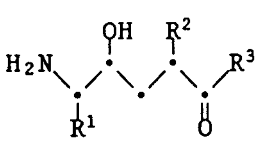 (I),

worin $R^1$ Wasserstoff, gegebenenfalls substituiertes Alkyl, Cycloalkyl, Cycloalkylniederalkyl, Aryl, Arylniederalkyl oder den Rest einer natürlichen Aminosäure, $R^2$ Wasserstoff, gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Hydroxy oder Amino darstellen, und von Salzen dieser Verbindungen, dadurch gekennzeichnet, dass man
einen Allylalkohol der Formel

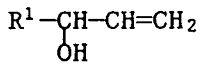 (II)

mit einer Säure oder Ester der Formel

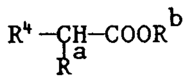 (III),

worin $R^a$ Wasserstoff oder eine Gruppe $COOR^b$ und $R^b$ Wasserstoff, einen Kohlenwasserstoffrest oder einen Silylrest bedeuten und $R^4$ die Bedeutungen von $R^2$ hat oder Halogen ist, oder mit einem Derivat davon in Gegenwart eines Katalysators verestert, die erhältliche Verbindung in situ und/oder nach Behandlung mit einer Base und einem Silylierungsmittel durch Erwärmen umlagert und gegebenenfalls decarboxyliert, die erhältliche Verbindung der Formel

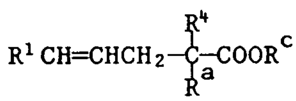 (IV),

worin $R^c$ Wasserstoff, einen Kohlenwasserstoffrest oder einen Silylrest bedeutet, falls $R^c$ verschieden von Wasserstoff ist und/oder $R^a$ eine Gruppe $COOR^b$ bedeutet und $R^b$ verschieden von Wasserstoff ist, hydrolysiert und, falls $R^a$ eine Gruppe $COOR^b$ bedeutet, decarboxyliert und, falls $R^4$ Halogen bedeutet, Halogen durch Amino, Hydroxy oder Mercapto austauscht,
eine erhältliche Verbindung der Formel

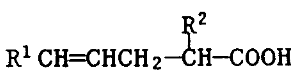 (V),

gewünschtenfalls nach Trennung der Enantiomeren und/oder Umwandlung der Carboxygruppe in eine Carboxamidfunktion oder in eine Hydroxamsäureestergruppe, mit einem Halogenierungsmittel halolactonisiert,
in einer erhältlichen Verbindung der Formel

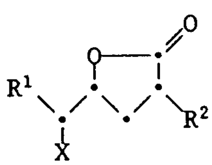 (VI),

worin X Halogen bedeutet, Halogen durch einen über Stickstoff gebundenen Rest austauscht und in beliebiger Reihenfolge den Lactonring mit einer den Rest $R^3$ einführenden Verbindung öffnet und den stickstoffhaltigen Rest X in eine Aminogruppe umwandelt und diese gegebenenfalls schützt,
und in einer erhältlichen Verbindung gegebenenfalls vorhandene Schutzgruppen abspaltet und/oder gewünschtenfalls eine erhältliche Verbindung der Formel I in ihr Salz überführt oder ein erhältliches Salz in die freie Verbindung überführt und/oder gewünschtenfalls erhältliche Isomerengemische auftrennt.

2. Verfahren gemäss Anspruch 1 zur Herstellung von Verbindungen der Formel I, worin $R^1$ Niederalkyl, Hydroxyniederalkyl, Cycloalkyl, Cycloalkylniederalkyl, Aryl oder Arylniederalkyl, $R^2$ Niederalkyl, Hydroxyniederalkyl, Cycloalkyl, Cycloalkylniederalkyl, Aryl, Arylniederalkyl, gegebenenfalls substituiertes Amino, gegebenenfalls substituiertes Hydroxy, gegebenenfalls substituiertes Mercapto, substituiertes Sulfinyl oder substituiertes Sulfonyl und $R^3$ gegebenenfalls substituiertes Amino darstellen, ferner von Salzen dieser Verbindungen.

3. Verfahren gemäss Anspruch 1 zur Herstellung von Verbindungen der Formel I, worin $R^1$ Niederalkyl, Cycloalkyl, Cycloalkylniederalkyl, Phenyl oder Phenylniederalkyl, $R^2$ Niederalkyl, Hydroxyniederalkyl, Cycloalkyl, Cycloalkylniederalkyl, Phenyl, Phenylniederalkyl, Amino, Niederalkylamino, Diniederalkylamino, Niederalkanoylamino, substituiertes Amino als Teil eines Ringes, Hydroxy, Niederalkoxy, Niederalkanoyloxy, Mercapto, Niederalkylthio oder Niederalkylsulfonyl und $R^3$ substituiertes Amino, worin der Substituent gegebenenfalls substituiertes Alkyl mit bis zu 12 C-Atomen, Hydroxyniederalkyl, Carboxyniederalkyl, gegebenenfalls substituiertes Carbamoylniederalkyl, Cycloalkyl, Cycloalkylniederalkyl, Niederalkenyl, Niederalkinyl, Aryl, Arylniederalkyl, Heteroaryl oder Heteroarylniederalkyl enthaltend einen mono- oder bicyclischen Heterocyclus mit einem oder zwei Stickstoffatomen bedeutet, ferner Diniederalkylamino oder substituiertes Amino als Teil eines Ringes darstellen, ferner von Salzen von solchen Verbindungen.

4. Verfahren gemäss Anspruch 1 zur Herstellung von Verbindungen der Formel I, worin $R^1$ Niederalkyl, Cycloalkylniederalkyl oder Phenylniederalkyl, $R^2$ Niederalkyl, Cycloalkyl, Phenyl, Diniederalkylamino, substituiertes Amino als Teil eines fünf- oder sechsgliedrigen Ringes, Niederalkoxy, Niederalkylthio oder Niederalkylsulfonyl und $R^3$ Niederalkylamino, Hydroxyniederalkylamino, Carboxyniederalkylamino, Carbamoylniederalkylamino, Cycloalkylniederalkylamino, Arylniederalkylamino, Heteroarylniederalkylamino enthaltend einen mono- oder bicyclischen Heterocyclus mit einem oder zwei Stickstoffatomen, Diniederalkylamino oder substituiertes Amino als Teil eines fünf- oder sechsgliedrigen Ringes bedeuten und die die Reste $R^1$ und OH tragenden C-Atome beide die R-Konfiguration oder beide die S-Konfiguration und das den Rest $R^2$ tragende C-Atom die R- oder S-Konfiguration aufweisen, ferner von Salzen von solchen Verbindungen.

5. Verfahren gemäss Anspruch 1 zur Herstellung von Verbindungen der Formel I, worin $R^1$ Niederalkyl oder Cycloalkylniederalkyl, $R^2$ Niederalkyl, Diniederalkylamino oder Niederalkoxy und $R^3$ Niederalkylamino darstellen und die die Reste $R^1$ und OH tragenden C-Atome die S-Konfiguration und das den Rest $R^2$ tragende C-Atom die R- oder S-Konfiguration aufweist, ferner von Salzen von solchen Verbindungen.

6. Verfahren gemäss Anspruch 1 zur Herstellung der Verbindung der Formel I, worin $R^1$ Cyclohexylmethyl, $R^2$ Isopropyl und $R^3$ n-Butylamino darstellen und die die Reste $R^1$, $R^2$ und OH tragenden C-Atome die S-Konfiguration aufweisen.

7. Verfahren zur Herstellung von Verbindungen der Formel

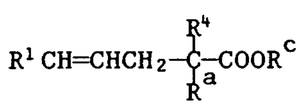 (IV),

worin $R^1$ die unter Formel I in Anspruch 1 genannten Bedeutungen hat, $R^4$ die unter Formel I in Anspruch 1 für $R^2$ genannten Bedeutungen hat oder Halogen ist und $R^a$ Wasserstoff und $R^c$ Wasserstoff oder Niederalkyl bedeuten, und von Salzen dieser Verbindungen, dadurch gekennzeichnet, dass man einen Allylalkohol der Formel

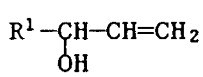 (II)

mit einem Malonester der Formel

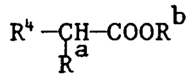

(III),

worin $R^a$ eine Gruppe $COOR^b$ und $R^b$ Niederalkyl bedeuten, in Gegenwart eines Tetraalkoxy-Derivats von Titan oder Zirkon bei Temperaturen zwischen 150° und 250°C umsetzt, und gewünschtenfalls die erhältliche Verbindung der Formel IV, worin $R^a$ Wasserstoff und $R^c$ Niederalkyl bedeuten, mit einer wässrigen Base hydrolysiert.

**8.** Verfahren gemäss Anspruch 7, dadurch gekennzeichnet, dass als Tetraalkoxy-Derivat Titan-tetraethoxid verwendet wird.

**9.** Verfahren zur Herstellung von Verbindungen der Formel

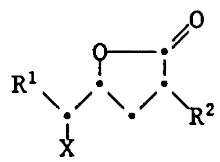

(VI),

worin $R^1$ und $R^2$ die unter Formel I in Anspruch 1 genannten Bedeutungen haben und worin X Chlor, Brom oder Iod bedeutet, dadurch gekennzeichnet, dass eine Verbindung der Formel

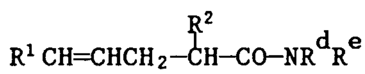

(VIII),

worin $R^1$ und $R^2$ die obengenannten Bedeutungen haben, $R^d$ und $R^e$ unabhängig voneinander Niederalkyl, Cycloalkyl, Arylniederalkyl oder zusammen Niederalkylen, Oxaniederalkylen oder Niederalkylazaniederalkylen mit 4 bis 6 C-Atomen in der Kette, wobei Aza nicht in 1-Stellung steht, oder $R^d$ Niederalkyl und $R^e$ Niederalkoxy bedeuten, mit einem Halogenierungsmittel und, wenn $R^e$ verschieden von Niederalkoxy und $R^d$ und $R^e$ zusammen verschieden von 1-Oxaniederalkylen sind, in Gegenwart einer schwachen Säure, in einem inerten organischen Lösungsmittel oder einem organischen oder wässrig-organischen Lösungsmittelgemisch bei Temperaturen zwischen -20° und 80°C umgesetzt wird.

**10.** Verfahren gemäss Anspruch 9, dadurch gekennzeichnet, dass als Halogenierungsmittel N-Bromsuccinimid verwendet wird.

**11.** Verbindungen der Formel

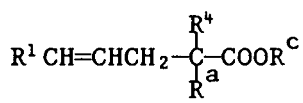

(IV),

worin $R^1$ Cycloalkylniederalkyl, $R^4$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl, Halogen oder den Rest einer natürlichen Aminosäure ausser Glycin, $R^a$ Wasserstoff und $R^c$ Wasserstoff, einen Kohlenwasserstoffrest oder einen Silylrest bedeuten, und Salze von solchen Verbindungen mit salzbildenden Gruppen.

**12.** Verbindungen gemäss Anspruch 11 der Formel IV, worin $R^c$ Wasserstoff bedeutet, ferner Salze davon, reine Enantiomere und Salze reiner Enantiomere.

**13.** Verbindungen der Formel

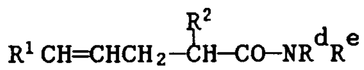 (VIII),

worin $R^1$ gegebenenfalls substituiertes Alkyl mit 2 und mehr Kohlenstoffatomen, Cycloalkyl, Cycloalkylniederalkyl, Aryl, Arylniederalkyl oder den Rest einer natürlichen Aminosäure ausser Glycin und Alanin, $R^2$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure ausser Glycin und $R^d$ und $R^e$ unabhängig voneinander Niederalkyl, Cycloalkyl oder Arylniederalkyl, $R^d$ und $R^e$ zusammen Niederalkylen, Oxaniederalkylen oder Niederalkylazaniederalkylen mit 4 bis 6 C-Atomen in der Kette, wobei Aza nicht in 1-Stellung steht, oder $R^d$ Niederalkyl und $R^e$ Niederalkoxy bedeuten, und Salze von solchen Verbindungen, in denen $R^2$ gegebenenfalls substituiertes Amino bedeutet.

**14.** Verbindungen gemäss Anspruch 13 der Formel VIII, worin $R^d$ Niederalkyl und $R^e$ Niederalkyl oder Niederalkoxy oder $R^d$ und $R^e$ zusammen Niederalkylen oder Oxaniederalkylen mit 4 bis 6 Atomen in der Kette darstellen, und reine Enantiomere davon.

**15.** Verbindungen der Formel

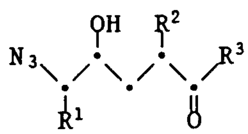 (IX),

worin $R^1$ Wasserstoff, gegebenenfalls substituiertes Alkyl, Cycloalkyl, Cycloalkylniederalkyl, Aryl, Arylniederalkyl oder den Rest einer natürliche Aminosäure, $R^2$ Wasserstoff, gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Amino darstellen, und Salze von solchen Verbindungen mit salzbildenden Gruppen.

**Patentansprüche für folgende Vertragstaaten : AT, ES**

**1.** Verfahren zur Herstellung von Verbindungen der Formel

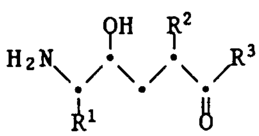 (I),

worin $R^1$ Wasserstoff, gegebenenfalls substituiertes Alkyl, Cycloalkyl, Cycloalkylniederalkyl, Aryl, Arylniederalkyl oder den Rest einer natürlichen Aminosäure, $R^2$ Wasserstoff, gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Hydroxy oder Amino darstellen, und von Salzen dieser Verbindungen, dadurch gekennzeichnet, dass man einen Allylalkohol der Formel

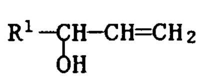 (II)

mit einer Säure oder Ester der Formel

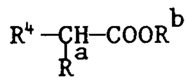 (III),

worin $R^a$ Wasserstoff oder eine Gruppe $COOR^b$ und $R^b$ Wasserstoff, einen Kohlenwasserstoffrest oder einen Silylrest bedeuten und $R^4$ die Bedeutungen von $R^2$ hat oder Halogen ist, oder mit einem Derivat davon in Gegenwart eines Katalysators verestert, die erhältliche Verbindung in situ und/oder nach Behandlung mit einer Base und einem Silylierungsmittel durch Erwärmen umlagert und gegebenenfalls decarboxyliert, die erhältliche Verbindung der Formel

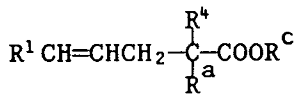 (IV),

worin $R^c$ Wasserstoff, einen Kohlenwasserstoffrest oder einen Silylrest bedeutet, falls $R^c$ verschieden von Wasserstoff ist und/oder $R^a$ eine Gruppe $COOR^b$ bedeutet und $R^b$ verschieden von Wasserstoff ist, hydrolysiert und, falls $R^a$ eine Gruppe $COOR^b$ bedeutet, decarboxyliert und, falls $R^4$ Halogen bedeutet, Halogen durch Amino, Hydroxy oder Mercapto austauscht,
eine erhältliche Verbindung der Formel

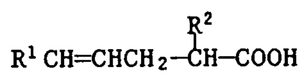 (V),

gewünschtenfalls nach Trennung der Enantiomeren und/oder Umwandlung der Carboxygruppe in eine Carboxamidfunktion oder in eine Hydroxamsäureestergruppe, mit einem Halogenierungsmittel halolactonisiert,
in einer erhältlichen Verbindung der Formel

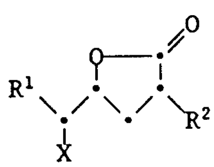 (VI),

worin X Halogen bedeutet, Halogen durch einen über Stickstoff gebundenen Rest austauscht und in beliebiger Reihenfolge den Lactonring mit einer den Rest $R^3$ einführenden Verbindung öffnet und den stickstoffhaltigen Rest X in eine Aminogruppe umwandelt und diese gegebenenfalls schützt,
und in einer erhältlichen Verbindung gegebenenfalls vorhandene Schutzgruppen abspaltet und/oder gewünschtenfalls eine erhältliche Verbindung der Formel I in ihr Salz überführt oder ein erhältliches Salz in die freie Verbindung überführt und/oder gewünschtenfalls erhältliche Isomerengemische auftrennt.

2. Verfahren gemäss Anspruch 1 zur Herstellung von Verbindungen der Formel I, worin $R^1$ Niederalkyl, Hydroxyniederalkyl, Cycloalkyl, Cycloalkylniederalkyl, Aryl oder Arylniederalkyl, $R^2$ Niederalkyl, Hydroxyniederalkyl, Cycloalkyl, Cycloalkylniederalkyl, Aryl, Arylniederalkyl, gegebenenfalls substituiertes Amino, gegebenenfalls substituiertes Hydroxy, gegebenenfalls substituiertes Mercapto, substituiertes Sulfinyl oder substituiertes Sulfonyl und $R^3$ gegebenenfalls substituiertes Amino darstellen, ferner von Salzen dieser Verbindungen.

3. Verfahren gemäss Anspruch 1 zur Herstellung von Verbindungen der Formel I, worin $R^1$ Niederalkyl, Cycloalkyl, Cycloalkylniederalkyl, Phenyl oder Phenylniederalkyl, $R^2$ Niederalkyl, Hydroxyniederalkyl, Cycloalkyl, Cycloalkylniederalkyl, Phenyl, Phenylniederalkyl, Amino, Niederalkylamino, Diniederalkylamino, Niederalkanoylamino, substituiertes Amino als Teil eines Ringes, Hydroxy, Niederalkoxy, Niederalkanoyloxy, Mercapto, Niederalkylthio oder Niederalkylsulfonyl und $R^3$ substituiertes Amino, worin der

Substituent gegebenenfalls substituiertes Alkyl mit bis zu 12 C-Atomen, Hydroxyniederalkyl, Carboxyniederalkyl, gegebenenfalls substituiertes Carbamoylniederalkyl, Cycloalkyl, Cycloalkylniederalkyl, Niederalkenyl, Niederalkinyl, Aryl, Arylniederalkyl, Heteroaryl oder Heteroarylniederalkyl enthaltend einen mono- oder bicyclischen Heterocyclus mit einem oder zwei Stickstoffatomen bedeutet, ferner Diniederalkylamino oder substituiertes Amino als Teil eines Ringes darstellen, ferner von Salzen von solchen Verbindungen.

**4.** Verfahren gemäss Anspruch 1 zur Herstellung von Verbindungen der Formel I, worin $R^1$ Niederalkyl, Cycloalkylniederalkyl oder Phenylniederalkyl, $R^2$ Niederalkyl, Cycloalkyl, Phenyl, Diniederalkylamino, substituiertes Amino als Teil eines fünf- oder sechsgliedrigen Ringes, Niederalkoxy, Niederalkylthio oder Niederalkylsulfonyl und $R^3$ Niederalkylamino, Hydroxyniederalkylamino, Carboxyniederalkylamino, Carbamoylniederalkylamino, Cycloalkylniederalkylamino, Arylniederalkylamino, Heteroarylniederalkylamino enthaltend einen mono- oder bicyclischen Heterocyclus mit einem oder zwei Stickstoffatomen, Diniederalkylamino oder substituiertes Amino als Teil eines fünf- oder sechsgliedrigen Ringes bedeuten und die die Reste $R^1$ und OH tragenden C-Atome beide die R-Konfiguration oder beide die S-Konfiguration und das den Rest $R^2$ tragende C-Atom die R- oder S-Konfiguration aufweisen, ferner von Salzen von solchen Verbindungen.

**5.** Verfahren gemäss Anspruch 1 zur Herstellung von Verbindungen der Formel I, worin $R^1$ Niederalkyl oder Cycloalkylniederalkyl, $R^2$ Niederalkyl, Diniederalkylamino oder Niederalkoxy und $R^3$ Niederalkylamino darstellen und die die Reste $R^1$ und OH tragenden C-Atome die S-Konfiguration und das den Rest $R^2$ tragende C-Atom die R- oder S-Konfiguration aufweist, ferner von Salzen von solchen Verbindungen.

**6.** Verfahren gemäss Anspruch 1 zur Herstellung der Verbindung der Formel I, worin $R^1$ Cyclohexylmethyl, $R^2$ Isopropyl und $R^3$ n-Butylamino darstellen und die die Reste $R^1$, $R^2$ und OH tragenden C-Atome die S-Konfiguration aufweisen.

**7.** Verfahren zur Herstellung von Verbindungen der Formel

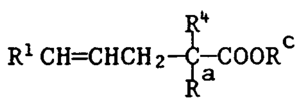 (IV),

worin $R^1$ die unter Formel I in Anspruch 1 genannten Bedeutungen hat, $R^4$ die unter Formel I in Anspruch 1 für $R^2$ genannten Bedeutungen hat oder Halogen ist und $R^a$ Wasserstoff und $R^c$ Wasserstoff, einen Kohlenwasserstoffrest oder einen Silylrest bedeuten, und von Salzen dieser Verbindungen, dadurch gekennzeichnet, dass man einen Allylalkohol der Formel

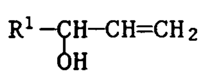 (II)

mit einer Säure oder Ester der Formel

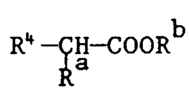 (III),

worin $R^a$ Wasserstoff oder eine Gruppe $COOR^b$ und $R^b$ Wasserstoff, einen Kohlenwasserstoffrest oder einen Silylrest bedeuten, oder mit einem Derivat davon in Gegenwart eines Katalysators verestert, die erhältliche Verbindung in situ und/oder nach Behandlung mit einer Base und einem Silylierungsmittel durch Erwärmen umlagert und gegebenenfalls decarboxyliert und gewünschtenfalls die erhältliche Verbindung der Formel IV hydrolysiert und decarboxyliert.

**8.** Verfahren gemäss Anspruch 7 zur Herstellung von Verbindungen der Formel IV, worin $R^1$, $R^4$ und $R^a$ die in Anspruch 7 genannten Bedeutungen haben und $R^c$ Wasserstoff oder Niederalkyl bedeutet, und

von Salzen dieser Verbindungen, dadurch gekennzeichnet, dass man einen Allylalkohol der Formel II mit einem Malonester der Formel III, worin $R^a$ eine Gruppe $COOR^b$ und $R^b$ Niederalkyl bedeuten, in Gegenwart eines Tetraalkoxy-Derivats von Titan oder Zirkon bei Temperaturen zwischen 150° und 250°C umsetzt, und gewünschtenfalls die erhältliche Verbindung der Formel IV, worin $R^a$ Wasserstoff und $R^c$ Niederalkyl bedeuten, mit einer wässrigen Base hydrolysiert.

**9.** Verfahren gemäss Anspruch 8, dadurch gekennzeichnet, dass als Tetraalkoxy-Derivat Titan-tetraethoxid verwendet wird.

**10.** Verfahren gemäss Anspruch 7, 8 oder 9 zur Herstellung von Verbindungen der Formel IV, worin $R^1$ Cycloalkylniederalkyl, $R^4$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl, Halogen oder den Rest einer natürlichen Aminosäure ausser Glycin, $R^a$ Wasserstoff und $R^c$ Wasserstoff, einen Kohlenwasserstoffrest oder einen Silylrest bedeuten, und von Salzen solcher Verbindungen mit salzbildenden Gruppen.

**11.** Verfahren gemäss Anspruch 7, 8 oder 9 zur Herstellung von Verbindungen der Formel IV, worin $R^1$, $R^4$ und $R^a$ die in Anspruch 10 genannten Bedeutungen haben und $R^c$ Wasserstoff bedeutet, ferner von Salzen, reinen Enantiomeren und Salzen reiner Enantiomere dieser Verbindungen.

**12.** Verfahren zur Herstellung von Verbindungen der Formel

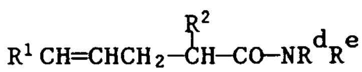 (VIII),

worin $R^1$ gegebenenfalls substituiertes Alkyl mit 2 und mehr Kohlenstoffatomen, Cycloalkyl, Cycloalkylniederalkyl, Aryl, Arylniederalkyl oder den Rest einer natürlichen Aminosäure ausser Glycin und Alanin, $R^2$ gegebenenfalls substituiertes Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure ausser Glycin und $R^d$ und $R^e$ unabhängig voneinander Niederalkyl, Cycloalkyl oder Arylniederalkyl, $R^d$ und $R^e$ zusammen Niederalkylen, Oxaniederalkylen oder Niederalkylazaniederalkylen mit 4 bis 6 C-Atomen in der Kette, wobei Aza nicht in 1-Stellung steht, oder $R^d$ Niederalkyl und $R^e$ Niederalkoxy bedeuten, und von Salzen von solchen Verbindungen, in denen $R^2$ gegebenenfalls substituiertes Amino bedeutet, dadurch gekennzeichnet, dass man eine Verbindung der Formel

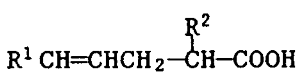 (V),

worin $R^1$ und $R^2$ die genannten Bedeutungen haben, mit einem Halogenierungsmittel in das entsprechende Säurehalogenid überführt und dieses mit einem den Rest $-NR^dR^e$ einführenden Amin oder Hydroxylamin umsetzt.

**13.** Verfahren gemäss Anspruch 12 zur Herstellung von Verbindungen der Formel VIII, worin $R^d$ Niederalkyl und $R^e$ Niederalkyl oder Niederalkoxy oder $R^d$ und $R^e$ zusammen Niederalkylen oder Oxaniederalkylen mit 4 bis 6 Atomen in der Kette darstellen, und von reinen Enantiomeren dieser Verbindungen.

**14.** Verfahren zur Herstellung von Verbindungen der Formel

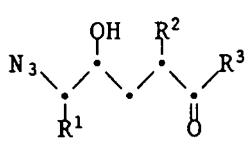 (IX),

worin $R^1$ Wasserstoff, gegebenenfalls substituiertes Alkyl, Cycloalkyl, Cycloalkylniederalkyl, Aryl, Arylniederalkyl oder den Rest einer natürliche Aminosäure, $R^2$ Wasserstoff, gegebenenfalls substituiertes

Alkyl, Cycloalkyl, Cycloalkylniederalkyl, Aryl, Arylniederalkyl, Amino, Hydroxy, Mercapto, Sulfinyl, Sulfonyl oder den Rest einer natürlichen Aminosäure und $R^3$ gegebenenfalls substituiertes Amino darstellen, und von Salzen von solchen Verbindungen mit salzbildenden Gruppen, dadurch gekennzeichnet, dass man in einer Verbindung der Formel

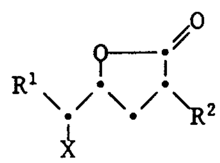

(VI),

worin X Azid bedeutet, mit einer den Rest $R^3$ einführenden Verbindung den Lactonring öffnet.

## Claims

**Claims for the following Contracting States : BE, CH, DE, FR, GB, IT, LI, NL, SE**

**1.** A process for the preparation of a compound of the formula

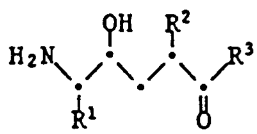

(I),

in which $R^1$ represents hydrogen, unsubstituted or substituted alkyl, cycloalkyl, cycloalkyl-lower alkyl, aryl, aryl-lower alkyl or the radical of a natural amino acid, $R^2$ represents hydrogen, unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid, and $R^3$ represents unsubstituted or substituted hydroxy or amino, or a salt of that compound, characterised in that
an allyl alcohol of the formula

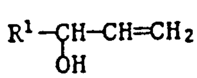

(II)

is esterified with an acid or ester of the formula

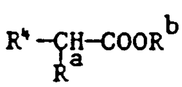

(III),

in which $R^a$ represents hydrogen or a group $COOR^b$ and $R^b$ represents hydrogen, a hydrocarbon radical or a silyl radical, and $R^4$ has the meanings of $R^2$ or is halogen, or with a derivative thereof, in the presence of a catalyst, the obtainable compound is, in situ and/or after treatment with a base and a silylating agent, rearranged by heating and optionally decarboxylated, the obtainable compound of the formula

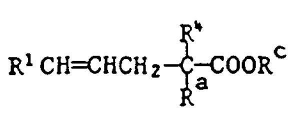

(IV),

in which $R^c$ represents hydrogen, a hydrocarbon radical or a silyl radical, is hydrolysed if $R^c$ is other than hydrogen and/or $R^a$ represents a group $COOR^b$ and $R^b$ is other than hydrogen, and decarboxylated if $R^a$ representsa group $COOR^b$, and, if $R^4$ represents halogen, halogen is replaced by amino,

hydroxy or mercapto,
an obtainable compound of the formula

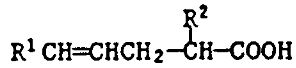

(V),

if desired after separating the enantiomers and/or converting the carboxy group into a carboxamide function or into a hydroxamic acid ester group, is halolactonised with a halogenating agent,
in an obtainable compound of the formula

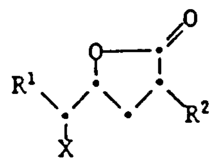

(VI),

in which X represents halogen, halogen is replaced by a radical bonded by way of nitrogen and, in any desired sequence, the lactone ring is opened with a compound that introduces the radical $R^3$, and the nitrogen-containing radical X is converted into an amino group and the latter is optionally protected,
and, in an obtainable compound, any protecting groups that may be present are removed and/or, if desired, an obtainable compound of the formula I is converted into its salt or an obtainable salt is converted into the free compound and/or, if desired, an obtainable mixture of isomers is separated.

**2.** A process according to claim 1 for the preparation of a compound of the formula I in which $R^1$ represents lower alkyl, hydroxy-lower alkyl, cycloalkyl, cycloalkyl-lower alkyl, aryl or aryl-lower alkyl, $R^2$ represents lower alkyl, hydroxy-lower alkyl, cycloalkyl, cycloalkyl-lower alkyl, aryl, aryl-lower alkyl, unsubstituted or substituted amino, unsubstituted or substituted hydroxy, unsubstituted or substituted mercapto, substituted sulphinyl or substituted sulphonyl, and $R^3$ represents unsubstituted or substituted amino, or a salt of that compound.

**3.** A process according to claim 1 for the preparation of a compound of the formula I in which $R^1$ represents lower alkyl, cycloalkyl, cycloalkyl-lower alkyl, phenyl or phenyl-lower alkyl, $R^2$ represents lower alkyl, hydroxy-lower alkyl, cycloalkyl, cycloalkyl-lower alkyl, phenyl, phenyl-lower alkyl, amino, lower alkylamino, di-lower alkylamino, lower alkanoylamino, substituted amino as part of a ring, hydroxy, lower alkoxy, lower alkanoyloxy, mercapto, lower alkylthio or lower alkylsulphonyl, and $R^3$ represents substituted amino in which the substituent is unsubstituted or substituted alkyl having up to 12 C-atoms, hydroxy-lower alkyl, carboxy-lower alkyl, unsubstituted or substituted carbamoyl-lower alkyl, cycloalkyl, cycloalkyl-lower alkyl, lower alkenyl, lower alkynyl, aryl, aryl-lower alkyl, heteroaryl or heteroaryl-lower alkyl containing a mono- or bi-cyclic heterocycle having one or two nitrogen atoms, or $R^3$ represents di-lower alkylamino or substituted amino as part of a ring, or a salt of such a compound.

**4.** A process according to claim 1 for the preparation of a compound of the formula I in which $R^1$ represents lower alkyl, cycloalkyl-lower alkyl or phenyl-lower alkyl, $R^2$ represents lower alkyl, cycloalkyl, phenyl, di-lower alkylamino, substituted amino as part of a five- or six-membered ring, lower alkoxy, lower alkylthio or lower alkylsulphonyl, and $R^3$ represents lower alkylamino, hydroxy-lower alkylamino, carboxy-lower alkylamino, carbamoyl-lower alkylamino, cycloalkyl-lower alkylamino, aryl-lower alkylamino, heteroaryl-lower alkylamino containing a mono- or bi-cyclic heterocycle having one or two nitrogen atoms, di-lower alkylamino or substituted amino as part of a five- or six-membered ring, and the C-atoms carrying the radicals $R^1$ and OH both have the R-configuration or both have the S-configuration and the C-atom carrying the radical $R^2$ has the R- or S-configuration, or a salt of such a compound.

**5.** A process according to claim 1 for the preparation of a compound of the formula I in which $R^1$ represents lower alkyl or cycloalkyl-lower alkyl, $R^2$ represents lower alkyl, di-lower alkylamino or lower alkoxy, and $R^3$ represents lower alkylamino, and the C-atoms carrying the radicals $R^1$ and OH have the

S-configuration and the C-atom carrying the radical $R^2$ has the R- or S-configuration, or a salt of such a compound.

6. A process according to claim 1 for the preparation of a compound of the formula I in which $R^1$ represents cyclohexylmethyl, $R^2$ represents isopropyl and $R^3$ represents n-butylamino, and the C-atoms carrying the radicals $R^1$, $R^2$ and OH have the S-configuration.

7. A process for the preparation of a compound of the formula

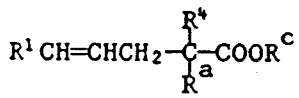

(IV)

in which $R^1$ has the meanings mentioned under formula I in claim 1, $R^4$ has the meanings mentioned for $R^2$ under formula I in claim 1 or is halogen, and $R^a$ represents hydrogen and $R^c$ represents hydrogen or lower alkyl, or a salt of that compound, characterised in that an allyl alcohol of the formula

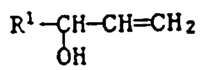

(II)

is reacted with a malonic acid ester of the formula

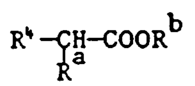

(III),

in which $R^a$ represents a group $COOR^b$ and $R^b$ represents lower alkyl, in the presence of a tetraalkoxy derivative of titanium or zirconium, at temperatures of from 150° to 250°C and, if desired, the obtainable compound of the formula IV in which $R^a$ represents hydrogen and $R^c$ represents lower alkyl is hydrolysed with an aqueous base.

8. A process according to claim 7, characterised in that titanium tetraethoxide is used as the tetraalkoxy derivative.

9. A process for the preparation of a compound of the formula

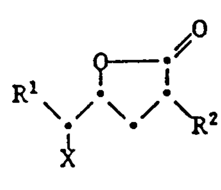

(VI)

in which $R^1$ and $R^2$ have the meanings mentioned under formula I in claim 1 and in which X represents chlorine, bromine or iodine, characterised in that a compound of the formula

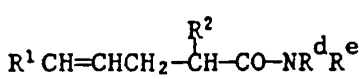

(VIII),

in which $R^1$ and $R^2$ have the meanings mentioned above, $R^d$ and $R^e$ each represents, independently of the other, lower alkyl, cycloalkyl or aryl-lower alkyl, or together represent lower alkylene, oxa-lower alkylene or lower alkylaza-lower alkylene having from 4 to 6 C-atoms in the chain, but with aza not

being in the 1-position, or $R^d$ represents lower alkyl and $R^e$ represents lower alkoxy, is reacted with a halogenating agent and, if $R^e$ is other than lower alkoxy and $R^d$ and $R^e$ together are other than 1-oxa-lower alkylene, in the presence of a weak acid, in an inert organic solvent or in an organic or aqueous-organic solvent mixture, at temperatures of from -20° to 80°C

**10.** A process according to claim 9, characterised in that N-bromosuccinimide is used as the halogenating agent.

**11.** A compound of the formula

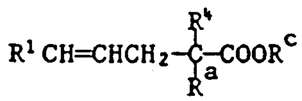

(IV)

in which $R^1$ represents cycloalkyl-lower alkyl, $R^4$ represents unsubstituted or substituted alkyl, cycloalkyl, cycloalkyl-lower alkyl, aryl, aryl-lower alkyl, amino, hydroxy, mercapto, sulphinyl, sulphonyl, halogen or the radical of a natural amino acid apart from glycine, $R^a$ represents hydrogen and $R^c$ represents hydrogen, a hydrocarbon radical or a silyl radical, or a salt of such a compound having a salt-forming group.

**12.** A compound according to claim 11 of the formula IV in which $R^c$ represents hydrogen, or a salt thereof, a pure enantiomer or a salt of a pure enantiomer.

**13.** A compound of the formula

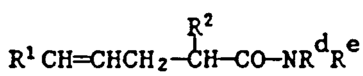

(VIII)

in which $R^1$ represents unsubstituted or substituted alkyl having 2 or more carbon atoms, cycloalkyl, cycloalkyl-lower alkyl, aryl, aryl-lower alkyl or the radical of a natural amino acid apart from glycine and alanine, $R^2$ represents unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid apart from glycine, and $R^d$ and $R^e$ each represents, independently of the other, lower alkyl, cycloalkyl or aryl-lower alkyl, $R^d$ and $R^e$ together represent lower alkylene, oxa-lower alkylene or lower alkylaza-lower alkylene having from 4 to 6 C-atoms in the chain, but with aza not being in the 1-position, or $R^d$ represents lower alkyl and $R^e$ represents lower alkoxy, or a salt of such a compound in which $R^2$ represents unsubstituted or substituted amino.

**14.** A compound according to claim 13 of the formula VIII in which $R^d$ represents lower alkyl and $R^e$ represents lower alkyl or lower alkoxy or $R^d$ and $R^e$ together represent lower alkylene or oxa-lower alkylene having from 4 to 6 atoms in the chain, or a pure enantiomer thereof.

**15.** A compound of the formula

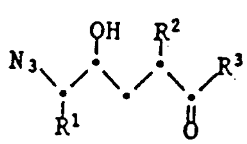

(IX)

in which $R^1$ represents hydrogen, unsubstituted or substituted alkyl, cycloalkyl, cycloalkyl-lower alkyl, aryl, aryl-lower alkyl or the radical of a natural amino acid, $R^2$ represents hydrogen, unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid, and $R^3$ represents unsubstituted or substituted amino, or a salt of such a compound having a salt-forming group.

**Claims for the following Contracting States : AT, ES**

**1.** A process for the preparation of a compound of the formula

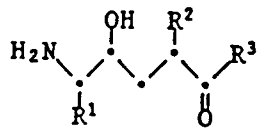

(I),

in which $R^1$ represents hydrogen, unsubstituted or substituted alkyl, cycloalkyl, cycloalkyl-lower alkyl, aryl, aryl-lower alkyl or the radical of a natural amino acid, $R^2$ represents hydrogen, unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid, and $R^3$ represents unsubstituted or substituted hydroxy or amino, or a salt of that compound, characterised in that
an allyl alcohol of the formula

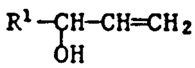

(II)

is esterified with an acid or ester of the formula

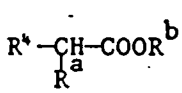

(III),

in which $R^a$ represents hydrogen or a group $COOR^b$ and $R^b$ represents hydrogen, a hydrocarbon radical or a silyl radical, and $R^4$ has the meanings of $R^2$ or is halogen, or with a derivative thereof, in the presence of a catalyst, the obtainable compound is, in situ and/or after treatment with a base and a silylating agent, rearranged by heating and optionally decarboxylated, the obtainable compound of the formula

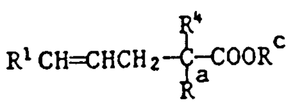

(IV),

in which $R^c$ represents hydrogen, a hydrocarbon radical or a silyl radical, is hydrolysed if $R^c$ is other than hydrogen and/or $R^a$ represents a group $COOR^b$ and $R^b$ is other than hydrogen, and decarboxylated if $R^a$ representsa group $COOR^b$, and, if $R^4$ represents halogen, halogen is replaced by amino, hydroxy or mercapto,
an obtainable compound of the formula

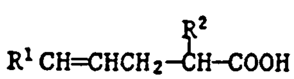

(V),

if desired after separating the enantiomers and/or converting the carboxy group into a carboxamide function or into a hydroxamic acid ester group, is halolactonised with a halogenating agent,
in an obtainable compound of the formula

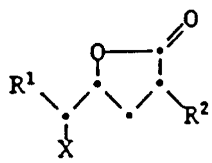 (VI),

in which X represents halogen, halogen is replaced by a radical bonded by way of nitrogen and, in any desired sequence, the lactone ring is opened with a compound that introduces the radical $R^3$, and the nitrogen-containing radical X is converted into an amino group and the latter is optionally protected, and, in an obtainable compound, any protecting groups that may be present are removed and/or, if desired, an obtainable compound of the formula I is converted into its salt or an obtainable salt is converted into the free compound and/or, if desired, an obtainable mixture of isomers is separated.

**2.** A process according to claim 1 for the preparation of a compound of the formula I in which $R^1$ represents lower alkyl, hydroxy-lower alkyl, cycloalkyl, cycloalkyl-lower alkyl, aryl or aryl-lower alkyl, $R^2$ represents lower alkyl, hydroxy-lower alkyl, cycloalkyl, cycloalkyl-lower alkyl, aryl, aryl-lower alkyl, unsubstituted or substituted amino, unsubstituted or substituted hydroxy, unsubstituted or substituted mercapto, substituted sulphinyl or substituted sulphonyl, and $R^3$ represents unsubstituted or substituted amino, or a salt of that compound.

**3.** A process according to claim 1 for the preparation of a compound of the formula I in which $R^1$ represents lower alkyl, cycloalkyl, cycloalkyl-lower alkyl, phenyl or phenyl-lower alkyl, $R^2$ represents lower alkyl, hydroxy-lower alkyl, cycloalkyl, cycloalkyl-lower alkyl, phenyl, phenyl-lower alkyl, amino, lower alkylamino, di-lower alkylamino, lower alkanoylamino, substituted amino as part of a ring, hydroxy, lower alkoxy, lower alkanoyloxy, mercapto, lower alkylthio or lower alkylsulphonyl, and $R^3$ represents substituted amino in which the substituent is unsubstituted or substituted alkyl having up to 12 C-atoms, hydroxy-lower alkyl, carboxy-lower alkyl, unsubstituted or substituted carbamoyl-lower alkyl, cycloalkyl, cycloalkyl-lower alkyl, lower alkenyl, lower alkynyl, aryl, aryl-lower alkyl, heteroaryl or heteroaryl-lower alkyl containing a mono- or bi-cyclic heterocycle having one or two nitrogen atoms, or $R^3$ represents di-lower alkylamino or substituted amino as part of a ring, or a salt of such a compound.

**4.** A process according to claim 1 for the preparation of a compound of the formula I in which $R^1$ represents lower alkyl, cycloalkyl-lower alkyl or phenyl-lower alkyl, $R^2$ represents lower alkyl, cycloalkyl, phenyl, di-lower alkylamino, substituted amino as part of a five- or six-membered ring, lower alkoxy, lower alkylthio or lower alkylsulphonyl, and $R^3$ represents lower alkylamino, hydroxy-lower alkylamino, carboxy-lower alkylamino, carbamoyl-lower alkylamino, cycloalkyl-lower alkylamino, aryl-lower alkylamino, heteroaryl-lower alkylamino containing a mono- or bi-cyclic heterocycle having one or two nitrogen atoms, di-lower alkylamino or substituted amino as part of a five- or six-membered ring, and the C-atoms carrying the radicals $R^1$ and OH both have the R-configuration or both have the S-configuration and the C-atom carrying the radical $R^2$ has the R- or S-configuration, or a salt of such a compound.

**5.** A process according to claim 1 for the preparation of a compound of the formula I in which $R^1$ represents lower alkyl or cycloalkyl-lower alkyl, $R^2$ represents lower alkyl, di-lower alkylamino or lower alkoxy, and $R^3$ represents lower alkylamino, and the C-atoms carrying the radicals $R^1$ and OH have the S-configuration and the C-atom carrying the radical $R^2$ has the R- or S-configuration, or a salt of such a compound.

**6.** A process according to claim 1 for the preparation of a compound of the formula I in which $R^1$ represents cyclohexylmethyl, $R^2$ represents isopropyl and $R^3$ represents n-butylamino, and the C-atoms carrying the radicals $R^1$, $R^2$ and OH have the S-configuration.

**7.** A process for the preparation of a compound of the formula

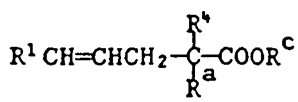

(IV)

in which $R^1$ has the meanings mentioned under formula I in claim 1, $R^4$ has the meanings mentioned for $R^2$ under formula I in claim 1 or is halogen, and $R^a$ represents hydrogen and $R^c$ represents hydrogen, a hydrocarbon radical or a silyl radical, or a salt of that compound, characterised in that an allyl alcohol of the formula

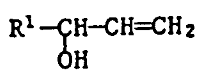

(II)

is esterified with an acid or ester of the formula

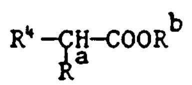

(III),

in which $R^a$ represents hydrogen or a group $COOR^b$ and $R^b$ represents hydrogen, a hydrocarbon radical or a silyl radical, or with a derivative thereof, in the presence of a catalyst, the obtainable compound is, in situ and/or after treatment with a base and a silylating agent, rearranged by heating and optionally decarboxylated and, if desired, the obtainable compound of the formula IV is hydrolysed and decarboxylated.

**8.** A process according to claim 7 for the preparation of a compound of the formula IV in which $R^1$, $R^4$ and $R^a$ have the meanings mentioned in claim 7, and $R^c$ represents hydrogen or lower alkyl, or a salt of that compound, characterised in that an allyl alcohol of the formula II is reacted with a malonic acid ester of the formula III in which $R^a$ represents a group $COOR^b$ and $R^b$ represents lower alkyl, in the presence of a tetraalkoxy derivative of titanium or zirconium at temperatures of from 150° to 250°C and, if desired, the obtainable compound of the formula IV in which $R^a$ represents hydrogen and $R^c$ represents lower alkyl is hydrolysed with an aqueous base.

**9.** A process according to claim 8, characterised in that titanium tetraethoxide is used as the tetraalkoxy derivative.

**10.** A process according to any one of claims 7, 8 and 9 for the preparation of a compound of the formula IV in which $R^1$ represents cycloalkyl-lower alkyl, $R^4$ represents unsubstituted or substituted alkyl, cycloalkyl, cycloalkyl-lower alkyl, aryl, aryl-lower alkyl, amino, hydroxy, mercapto, sulphinyl, sulphonyl, halogen or the radical of a natural amino acid apart from glycine, $R^a$ represents hydrogen and $R^c$ represents hydrogen, a hydrocarbon radical or a silyl radical, or a salt of such a compound having a salt-forming group.

**11.** A process according to any one of claims 7, 8 and 9 for the preparation of a compound of the formula IV in which $R^1$, $R^4$ and $R^a$ have the meanings mentioned in claim 10, and $R^c$ represents hydrogen, or a salt, a pure enantiomer or a salt of a pure enantiomer of that compound.

**12.** A process for the preparation of a compound of the formula

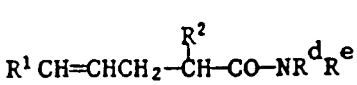

(VIII)

in which $R^1$ represents unsubstituted or substituted alkyl having 2 or more carbon atoms, cycloalkyl, cycloalkyl-lower alkyl, aryl, aryl-lower alkyl or the radical of a natural amino acid apart from glycine and alanine, $R^2$ represents unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid apart from glycine, and $R^d$ and $R^e$ each represents, independently of the other, lower alkyl, cycloalkyl or aryl-lower alkyl, $R^d$ and $R^e$ together represent lower alkylene, oxa-lower alkylene or lower alkylaza-lower alkylene having from 4 to 6 C-atoms in the chain, but with aza not being in the 1-position, or $R^d$ represents lower alkyl and $R^e$ represents lower alkoxy, or a salt of such a compound in which $R^2$ represents unsubstituted or substituted amino, characterised in that a compound of the formula

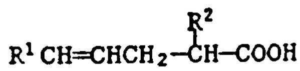 (V),

in which $R^1$ and $R^2$ have the meanings mentioned, is converted with a halogenating agent into the corresponding acid halide and the latter is reacted with an amine or a hydroxylamine that introduces the radical $-NR^dR^e$.

**13.** A process according to claim 12 for the preparation of a compound of the formula VIII in which $R^d$ represents lower alkyl and $R^e$ represents lower alkyl or lower alkoxy or $R^d$ and $R^e$ together represent lower alkylene or oxa-lower alkylene having from 4 to 6 atoms in the chain, or a pure enantiomer of that compound.

**14.** A process for the preparation of a compound of the formula

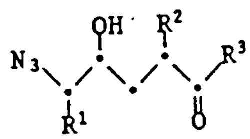 (IX)

in which $R^1$ represents hydrogen, unsubstituted or substituted alkyl, cycloalkyl, cycloalkyl-lower alkyl, aryl, aryl-lower alkyl or the radical of a natural amino acid, $R^2$ represents hydrogen, unsubstituted or substituted alkyl, cycloalkyl, cycloalkyl-lower alkyl, aryl, aryl-lower alkyl, amino, hydroxy, mercapto, sulphinyl, sulphonyl or the radical of a natural amino acid, and $R^3$ represents unsubstituted or substituted amino, or a salt of such a compound having a salt-forming group, characterised in that, in a compound of the formula

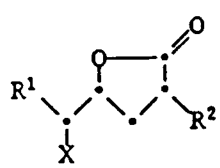 (VI),

in which X represents azide, the lactone ring is opened with a compound that introduces the radical $R^3$.

**Revendications**

**Revendications pour les Etats contractants suivants : BE, CH, DE, FR, GB, IT, LI, NL, SE**

**1.** Procédé pour la préparation de composés de formule

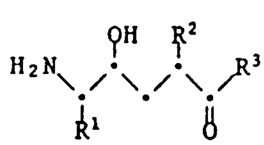 (I),

dans laquelle $R^1$ représente un atome d'hydrogène, un groupe alkyle, cycloalkyle, cycloalkyl-alkyle inférieur, aryle, aryl-alkyle inférieur, éventuellement substitués, ou le reste d'un aminoacide naturel, $R^2$ représente un atome d'hydrogène, un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, et $R^3$ représente un groupe hydroxy ou amino éventuellement substitué, et des sels de ces composés, caractérisé en ce que l'on estérifie un alcool allylique de formule

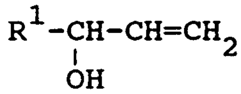

(II)

avec un acide ou ester de formule

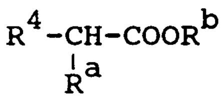

(III),

dans laquelle $R^a$ représente un atome d'hydrogène ou un groupe $COOR^b$, et $R^b$ représente un atome d'hydrogène, un radical hydrocarboné ou un radical silyle, et $R^4$ a les significations de $R^2$ ou est un atome d'halogène, ou avec un dérivé de celui-ci, en présence d'un catalyseur, le composé obtenu est transposé par chauffage et éventuellement décarboxylé in situ et/ou après traitement par une base et un agent de silylation, le composé obtenu, de formule

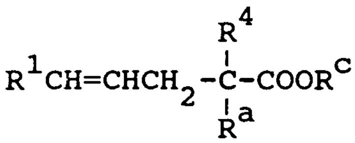

(IV),

dans laquelle $R^c$ représente un atome d'hydrogène, un radical hydrocarboné ou un radical silyle, est hydrolysé dans le cas où $R^c$ est différent d'un atome d'hydrogène et/ou $R^a$ représente un groupe $COOR^b$ et $R^b$ est différent d'un atome d'hydrogène, et décarboxylé dans le cas où $R^a$ représente un groupe $COOR^b$, et, lorsque $R^4$ représente un atome d'halogène, on remplace l'atome d'halogène par le groupe amino, hydroxy ou mercapto,

on soumet à une halogénolactonisation à l'aide d'un agent d'halogénation un composé obtenu de formule

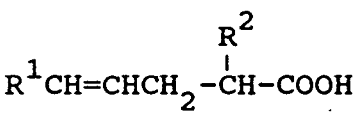

(V),

si on le désire après séparation des énantiomères et/ou conversion du groupe carboxy en une fonction carboxamide ou en un groupe ester d'acide hydroxamique,

dans un composé obtenu de formule

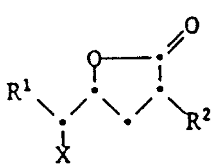

(VI),

dans laquelle X représente un atome d'halogène, on remplace l'atome d'halogène par un radical lié par un atome d'azote, et, en un ordre quelconque, on ouvre le cycle lactone avec un composé introduisant le radical $R^3$, et on convertit le radical azoté X en un groupe amino, et on protège éventuellement celuici,

et, dans un composé obtenu, on élimine les groupes protecteurs éventuellement présents et/ou, si on le désire, on convertit un composé de formule I obtenu en un de ses sels ou on convertit un sel obtenu en le composé libre, et/ou, si on le désire, on scinde des mélanges d'isomères obtenus.

**2.** Procédé selon la revendication 1, pour la préparation de composés de formule I dans lesquels $R^1$ représente un groupe alkyle inférieur, hydroxy-alkyle inférieur, cycloalkyle, cycloalkyl-alkyle inférieur, aryle ou aryl-alkyle inférieur, $R^2$ représente un groupe alkyle inférieur, hydroxy-alkyle inférieur, cycloalkyle, cycloalkyl-alkyle inférieur, aryle, aryl-alkyle inférieur, un groupe amino éventuellement substitué, un groupe hydroxy éventuellement substitué, un groupe mercapto éventuellement substitué, un groupe sulfinyle substitué ou un groupe sulfonyle substitué, et $R^3$ représente un groupe amino éventuellement substitué, ainsi que des sels de ces composés.

**3.** Procédé selon la revendication 1, pour la préparation de composés de formule 1 dans lesquels $R^1$ représente un groupe alkyle inférieur, cycloalkyle, cycloalkyl-alkyle inférieur, phényle ou phényl-alkyle inférieur, $R^2$ représente un groupe alkyle inférieur, hydroxy-alkyle inférieur, cycloalkyle, cycloalkyl-alkyle inférieur, phényle, phényl-alkyle inférieur, amino, (alkyl inférieur)-amino, di(alkyl inférieur)-amino, (alcanoyl inférieur)-amino, un groupe amino substitué faisant partie d'un cycle, le groupe hydroxy, un groupe alcoxy inférieur, alcanoyloxy inférieur, mercapto, (alkyl inférieur)-thio ou (alkyl inférieur)-sulfonyle, et $R^3$ représente un groupe amino substitué dans lequel le substituant est un radical alkyle éventuellement substitué ayant jusqu'à 12 atomes de carbone, hydroxyalkyle inférieur, carboxyalkyle inférieur, un radical carbamoyl-alkyle inférieur éventuellement substitué, cycloalkyle, cycloalkyl-alkyle inférieur, alcényle inférieur, alcynyle inférieur, aryle, aryl-alkyle inférieur, hétéroaryle ou hétéroaryl-alkyle inférieur contenant un hétérocycle mono- ou bicyclique comportant 1 ou 2 atomes d'azote, en outre un groupe di(alkyl inférieur)-amino ou amino substitué faisant partie d'un cycle, ainsi que des sels de ces composés.

**4.** Procédé selon la revendication 1, pour la préparation de composés de formule 1 dans lesquels $R^1$ représente un groupe alkyle inférieur, cycloalkyl-alkyle inférieur ou phényl-alkyle inférieur, $R^2$ représente un groupe alkyle inférieur, cycloalkyle, phényle, di(alkyl inférieur)-amino, un groupe amino substitué faisant partie d'un cycle à 5 ou 6 chaînons, alcoxy inférieur, (alkyl inférieur)-thio ou (alkyl inférieur)-sulfonyle, et $R^3$ représente un groupe (alkyl inférieur)-amino, hydroxy-(alkyl inférieur)-amino, carboxy-(alkyl inférieur)-amino, carbamoyl-(alkyl inférieur)-amino, un groupe cycloalkyl-(alkyl inférieur)-amino, aryl-(alkyl inférieur)-amino, hétéroaryl-(alkyl inférieur)-amino contenant un hétérocycle mont ou bicyclique comportant un ou deux atomes d'azote, un groupe di(alkyl inférieur)-amino ou amino substitué faisant partie d'un cycle à 5 ou 6 chaînons, et les atomes de carbone portant les radicaux $R^1$ et OH présentent tous les deux la configuration R ou tous les deux la configuration S, et l'atome de carbone portant le radical $R^2$ présente la configuration R ou S, ainsi que des sels de ces composés.

**5.** Procédé selon la revendication 1, pour la préparation de composés de formule I dans lesquels $R^1$ représente un groupe alkyle inférieur ou cycloalkyl-alkyle inférieur, $R^2$ représente un groupe alkyle inférieur, di(alkyl inférieur)-amino ou alcoxy inférieur, et $R^3$ représente un groupe alkylamino inférieur, et les atomes de carbone portant les radicaux $R^1$ et OH présentent la configuration S, et l'atome de carbone portant le radical $R^2$ présente la configuration R ou S, ainsi que des sels de ces composés.

**6.** Procédé selon la revendication 1, pour la préparation du composé de formule I dans lequel $R^1$ représente le groupe cyclohexylméthyle, $R^2$ représente le groupe isopropyle et $R^3$ représente le groupe n-butylamino, et les atomes de carbone portant les radicaux $R^1$, $R^2$ et OH présentent la configuration S.

**7.** Procédé pour la préparation de composés de formule

$$R^1CH{=}CHCH_2-\overset{\displaystyle R^4}{\underset{\displaystyle R^a}{\overset{|}{\underset{|}{C}}}}-COOR^c \qquad (IV),$$

dans laquelle $R^1$ a les significations données sous la formule I dans la revendication 1, $R^4$ a les significations données pour $R^2$ sous la formule I dans la revendication 1, ou est un atome d'halogène,

et $R^a$ représente un atome d'hydrogène et $R^c$ représente un atome d'hydrogène ou un groupe alkyle inférieur, et des sels de ces composés, caractérisé en ce que l'on fait réagir un alcool allylique de formule

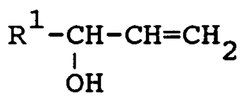

(II)

avec un ester malonique de formule

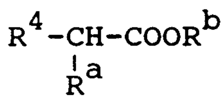

(III),

dans laquelle $R^a$ représente un groupe $COOR^b$ et $R^b$ représente un groupe alkyle inférieur, en présence d'un dérivé tétraalcoxy de titane ou de zirconium, à des températures comprises entre 150 et 250 °C, et, si on le désire, on hydrolyse avec une base aqueuse le composé de formule IV obtenu, dans lequel $R^a$ représente un atome d'hydrogène et $R^c$ représente un groupe alkyle inférieur.

**8.** Procédé selon la revendication 7, caractérisé en ce que, en tant que dérivé tétraalcoxy, on utilise le tétraéthylate de titane.

**9.** Procédé pour la préparation de composés de formule

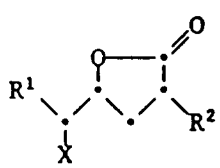

(VI),

dans laquelle $R^1$ et $R^2$ ont les significations données sous la formule I dans la revendication 1, et dans laquelle X représente le chlore, le brome ou l'iode, caractérisé en ce que l'on fait réagir un composé de formule

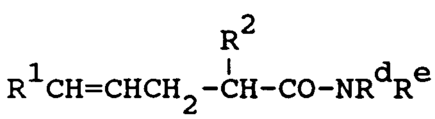

(VIII).

dans laquelle $R^1$ et $R^2$ ont les significations données plus haut, $R^d$ et $R^e$ représentent, indépendamment l'un de l'autre, un groupe alkyle inférieur, cycloalkyle, aryle-alkyle inférieur ou forment ensemble un groupe alkylène inférieur, oxa-alkylène inférieur ou (alkyl inférieur)-aza-alkylène inférieur ayant de 4 à 6 atomes dans la chaîne, la fonction aza n'étant pas en position 1, ou $R^d$ représente un groupe alkyle inférieur et $R^e$ représente un groupe alcoxy inférieur, avec un agent d'halogénation et, lorsque $R^e$ est différent d'un groupe alcoxy inférieur, et $R^d$ et $R^e$ ensemble sont différents d'un groupe 1-oxa-alkylène inférieur, en présence d'un acide faible, dans un solvant organique inerte ou dans un mélange de solvants organiques ou aqueuxorganiques, à des températures comprises entre -20 et +80 °C.

**10.** Procédé selon la revendication 9, caractérisé en ce que, en tant qu'agent d'halogénation, on utilise le N-bromosuccinimide.

**11.** Composés de formule

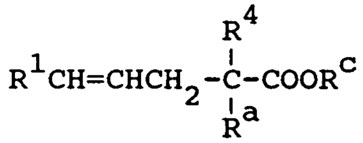

(IV),

dans laquelle $R^1$ représente un groupe cycloalkyl-alkyle inférieur, $R^4$ représente un groupe alkyle, cycloalkyle, cycloalky-alkyle inférieur, aryl-alkyle inférieur, amino, hydroxy, mercapto, sulfinyle, sulfonyle, éventuellement substitués, un atome d'halogène ou le reste d'un aminoacide naturel à l'exception de la glycine, $R^a$ représente un atome d'hydrogène et $R^c$ représente un atome d'hydrogène, un radical hydrocarboné ou le radical silyle, et sels de tels composés avec des groupes salifiants.

**12.** Composés selon la revendication 11, de formule IV, dans lesquels $R^c$ représente un atome d'hydrogène, ainsi que leurs sels, énantiomères purs et sels d'énantiomères purs.

**13.** Composés de formule

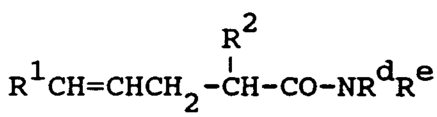

(VIII)

dans laquelle $R^1$ représente un groupe alkyle ayant 2 atomes de carbone ou plus, cycloalkyle, cycloalkyl-alkyle inférieur, aryle, aryl-alkyle inférieur, éventuellement substitués, ou le reste d'un aminoacide naturel, à l'exception de la glycine et de l'alanine, $R^2$ représente un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, à l'exception de la glycine, et $R^d$ et $R^e$ représentent, indépendamment l'un de l'autre, un groupe alkyle inférieur, cycloalkyle ou aryl-alkyle inférieur, $R^d$ et $R^e$ représentent ensemble un groupe alkylène inférieur, oxa-alkylène inférieur ou (alkyl inférieur)-aza-alkylène inférieur ayant de 4 à 6 atomes de carbone dans la chaîne, la fonction aza n'étant pas en position 1, ou $R^d$ représente un groupe alkyle inférieur et $R^e$ représente un groupe alcoxy inférieur, et sels des composés dans lesquels $R^2$ représente un groupe amino éventuellement substitué.

**14.** Composés selon la revendication 13, de formule VIII dans laquelle $R^d$ représente un groupe alkyle inférieur et $R^e$ représente un groupe alkyle inférieur ou alcoxy inférieur, ou $R^d$ et $R^e$ représentent un groupe alkylène inférieur ou oxa-alkylène inférieur, ayant de 4 à 6 atomes de carbone dans la chaîne, et énantiomères purs de ceux-ci.

**15.** Composés de formule

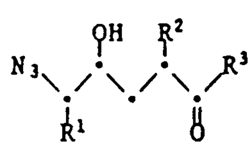

(IX)

dans laquelle $R^1$ représente un atome d'hydrogène, un groupe alkyle, cycloalkyle, cycloalkyl-alkyle inférieur, aryle, aryl-alkyle inférieur, éventuellement substitués, ou le reste d'un aminoacide naturel, $R^2$ représente un atome d'hydrogène, un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, et $R^3$ représente un groupe amino éventuellement substitué, et sels de tels composés avec des groupes salifiants.

**Revendications pour les Etats contractants suivants : AT, ES**

**1.** Procédé pour la préparation de composés de formule

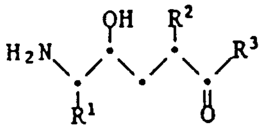

(I),

dans laquelle $R^1$ représente un atome d'hydrogène, un groupe alkyle, cycloalkyle, cycloalkyl-alkyle inférieur, aryle, aryl-alkyle inférieur, éventuellement substitués, ou le reste d'un aminoacide naturel, $R^2$ représente un atome d'hydrogène, un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, et $R^3$ représente un groupe hydroxy ou amino éventuellement substitué, et des sels de ces composés, caractérisé en ce que l'on estérifie un alcool allylique de formule

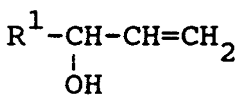

(II)

avec un acide ou ester de formule

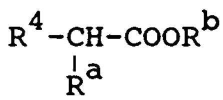

(III),

dans laquelle $R^a$ représente un atome d'hydrogène ou un groupe $COOR^b$, et $R^b$ représente un atome d'hydrogène, un radical hydrocarboné ou un radical silyle, et $R^4$ a les significations de $R^2$ ou est un atome d'halogène, ou avec un dérivé de celui-ci, en présence d'un catalyseur, le composé obtenu est transposé par chauffage et éventuellement décarboxylé in situ et/ou après traitement par une base et un agent de silylation, le composé obtenu, de formule

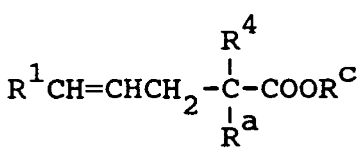

(IV),

dans laquelle $R^c$ représente un atome d'hydrogène, un radical hydrocarboné ou un radical silyle, est hydrolysé dans le cas où $R^c$ est différent d'un atome d'hydrogène et/ou $R^a$ représente un groupe $COOR^b$ et $R^b$ est différent d'un atome d'hydrogène, et décarboxylé dans le cas où $R^a$ représente un groupe $COOR^b$, et, lorsque $R^4$ représente un atome d'halogène, on remplace l'atome d'halogène par le groupe amino, hydroxy ou mercapto,
on soumet à une halogénolactonisation à l'aide d'un agent d'halogénation un composé obtenu de formule

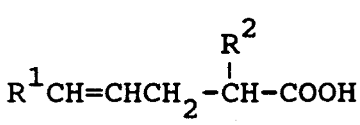

(V),

si on le désire après séparation des énantiomères et/ou conversion du groupe carboxy en une fonction carboxamide ou en un groupe ester d'acide hydroxamique,
dans un composé obtenu de formule

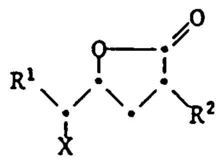

(VI),

dans laquelle X représente un atome d'halogène, on remplace l'atome d'halogène par un radical lié par un atome d'azote, et, en un ordre quelconque, on ouvre le cycle lactone avec un composé introduisant le radical $R^3$, et on convertit le radical azoté X en un groupe amino, et on protège éventuellement celui-ci,
et, dans un composé obtenu, on élimine les groupes protecteurs éventuellement présents et/ou, si on le désire, on convertit un composé de formule I obtenu en un de ses sels ou on convertit un sel obtenu en le composé libre, et/ou, si on le désire, on scinde des mélanges d'isomères obtenus.

**2.** Procédé selon la revendication 1, pour la préparation de composés de formule I dans lesquels $R^1$ représente un groupe alkyle inférieur, hydroxy-alkyle inférieur, cycloalkyle, cycloalkyl-alkyle inférieur, aryle ou aryl-alkyle inférieur, $R^2$ représente un groupe alkyle inférieur, hydroxy-alkyle inférieur, cycloalkyle, cycloalkyl-alkyle inférieur, aryle, aryl-alkyle inférieur, un groupe amino éventuellement substitué, un groupe hydroxy éventuellement substitué, un groupe mercapto éventuellement substitué, un groupe sulfinyle substitué ou un groupe sulfonyle substitué, et $R^3$ représente un groupe amino éventuellement substitué, ainsi que des sels de ces composés.

**3.** Procédé selon la revendication 1, pour la préparation de composés de formule 1 dans lesquels $R^1$ représente un groupe alkyle inférieur, cycloalkyle, cycloalkyl-alkyle inférieur, phényle ou phényl-alkyle inférieur, $R^2$ représente un groupe alkyle inférieur, hydroxy-alkyle inférieur, cycloalkyle, cycloalkyl-alkyle inférieur, phényle, phényl-alkyle inférieur, amino, (alkyl inférieur)-amino, di(alkyl inférieur)-amino, (alcanoyl inférieur)-amino, un groupe amino substitué faisant partie d'un cycle, le groupe hydroxy, un groupe alcoxy inférieur, alcanoyloxy inférieur, mercapto, (alkyl inférieur)-thio ou (alkyl inférieur)-sulfonyle, et $R^3$ représente un groupe amino substitué dans lequel le substituant est un radical alkyle éventuellement substitué ayant jusqu'à 12 atomes de carbone, hydroxyalkyle inférieur, carboxyalkyle inférieur, un radical carbamoyl-alkyle inférieur éventuellement substitué, cycloalkyle, cycloalkyl-alkyle inférieur, alcényle inférieur, alcynyle inférieur, aryle, aryl-alkyle inférieur, hétéroaryle ou hétéroaryl-alkyle inférieur contenant un hétérocycle mono- ou bicyclique comportant 1 ou 2 atomes d'azote, en outre un groupe di(alkyl inférieur)-amino ou amino substitué faisant partie d'un cycle, ainsi que des sels de ces composés.

**4.** Procédé selon la revendication 1, pour la préparation de composés de formule 1 dans lesquels $R^1$ représente un groupe alkyle inférieur, cycloalkyl-alkyle inférieur ou phényl-alkyle inférieur, $R^2$ représente un groupe alkyle inférieur, cycloalkyle, phényle, di(alkyl inférieur)-amino, un groupe amino substitué faisant partie d'un cycle à 5 ou 6 chaînons, alcoxy inférieur, (alkyl inférieur)-thio ou (alkyl inférieur)-sulfonyle, et $R^3$ représente un groupe (alkyl inférieur)-amino, hydroxy-(alkyl inférieur)-amino, carboxy-(alkyl inférieur)-amino, carbamoyl-(alkyl inférieur)-amino, un groupe cycloalkyl-(alkyl inférieur)-amino, aryl-(alkyl inférieur)-amino, hétéroaryl-(alkyl inférieur)-amino contenant un hétérocycle mont ou bicyclique comportant un ou deux atomes d'azote, un groupe di(alkyl inférieur)-amino ou amino substitué faisant partie d'un cycle à 5 ou 6 chaînons, et les atomes de carbone portant les radicaux $R^1$ et OH présentent tous les deux la configuration R ou tous les deux la configuration S, et l'atome de carbone portant le radical $R^2$ présente la configuration R ou S, ainsi que des sels de ces composés.

**5.** Procédé selon la revendication 1, pour la préparation de composés de formule I dans lesquels $R^1$ représente un groupe alkyle inférieur ou cycloalkyl-alkyle inférieur, $R^2$ représente un groupe alkyle inférieur, di(alkyl inférieur)-amino ou alcoxy inférieur, et $R^3$ représente un groupe alkylamino inférieur, et les atomes de carbone portant les radicaux $R^1$ et OH présentent la configuration S, et l'atome de carbone portant le radical $R^2$ présente la configuration R ou S, ainsi que des sels de ces composés.

**6.** Procédé selon la revendication 1, pour la préparation du composé de formule I dans lequel $R^1$ représente le groupe cyclohexylméthyle, $R^2$ représente le groupe isopropyle et $R^3$ représente le groupe n-butylamino, et les atomes de carbone portant les radicaux $R^1$, $R^2$ et OH présentent la configuration S.

**7.** Procédé pour la préparation de composés de formule

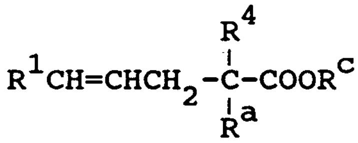

(IV),

dans laquelle $R^1$ a les significations données sous la formule I dans la revendication 1, $R^4$ a les significations données pour $R^2$ sous la formule I dans la revendication 1, ou est un atome d'halogène, et $R^a$ représente un atome d'hydrogène et $R^c$ représente un atome d'hydrogène, un radical hydrocarboné ou le radical silyle, et de sels de tels composés, caractérisé en ce que l'on estérifie un alcool allylique de formule

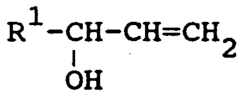

(II)

avec un acide ou ester de formule

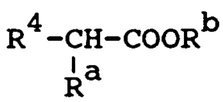

(III),

dans laquelle $R^a$ représente un atome d'hydrogène ou un groupe $COOR^b$ et $R^b$ représente un atome d'hydrogène, un radical hydrocarboné ou le radical silyle, ou avec un dérivé de celui-ci, en présence d'un catalyseur, le composé obtenu est transposé et éventuellement décarboxylé par chauffage in situ et/ou après traitement par une base et un agent de silylation, et si on le désire le composé de formule IV obtenu est hydrolysé et décarboxylé.

**8.** Procédé selon la revendication 7, pour la préparation de composés de formule IV dans lesquels $R^1$, $R^4$ et $R^a$ ont les significations données dans la revendication 7, et $R^c$ représente un atome d'hydrogène ou un groupe alkyle inférieur, et de sels de ces composés, caractérisé en ce que l'on fait réagir un alcool allylique de formule II avec un ester malonique de formule III, dans lequel $R^a$ représente un groupe $COOR^b$ et $R^b$ représente un groupe alkyle inférieur, en présence d'un dérivé tétraalcoxy de titane ou de zirconium, à des températures comprises entre 150 et 250 ° C, et si on le désire, on hydrolyse à l'aide d'une base aqueuse le composé de formule IV obtenu, dans lequel $R^a$ représente un atome d'hydrogène et $R^c$ représente un groupe alkyle inférieur.

**9.** Procédé selon la revendication 8, caractérisé en ce que, en tant que dérivé tétraalcoxy, on utilise le tétraéthylate de titane.

**10.** Procédé selon la revendication 7, 8 ou 9, pour la préparation de composés de formule lu dans lesquels $R^1$ représente un groupe cycloalkyl-alkyle inférieur, $R^4$ représente un groupe alkyle, cycloalkyle, cycloalkyl-alkyle inférieur, aryle, aryl-alkyle inférieur, amino, hydroxy, mercapto, sulfinyle, sulfonyle, éventuellement substitués, un atome d'halogène ou le reste d'un aminoacide naturel, à l'exception de la glycine, $R^a$ représente un atome d'hydrogène et $R^c$ représente un atome d'hydrogène, un radical hydrocarboné ou le radical silyle, et de sels de tels composés avec des groupes salifiants.

**11.** Procédé selon la revendication 7, 8 ou 9, pour la préparation de composés de formule IV dans lesquels $R^1$, $R^4$ et $R^a$ ont les significations données dans la revendication 10, et $R^c$ représente un atome d'hydrogène, ainsi que de sels, d'énantiomères purs et de sels d'énantiomères purs de tels composés.

**12.** Procédé pour la préparation de composés de formule

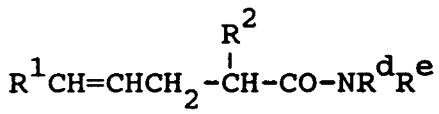

(VIII)

dans laquelle $R^1$ représente un groupe alkyle ayant 2 atomes de carbone ou plus, cycloalkyle, cycloalkyl-alkyle inférieur, aryle, aryl-alkyle inférieur, éventuellement substitués, ou le reste d'un aminoacide naturel, à l'exception de la glycine et de l'alanine, $R^2$ représente un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, à l'exception de la glycine, et $R^d$ et $R^e$ représentent, indépendamment l'un de l'autre, un groupe alkyle inférieur, cycloalkyle ou aryl-alkyle inférieur, $R^d$ et $R^e$ représentent ensemble un groupe alkylène inférieur, oxa-alkylène inférieur ou (alkyl inférieur)-aza-alkylène inférieur ayant de 4 à 6 atomes de carbone dans la chaîne, la fonction aza n'étant pas en position 1, ou $R^d$ représente un groupe alkyle inférieur et $R^e$ représente un groupe alcoxy inférieur, et des sels des composés dans lesquels $R^2$ représente un groupe amino éventuellement substitué, caractérisé en ce que l'on convertit un composé de formule

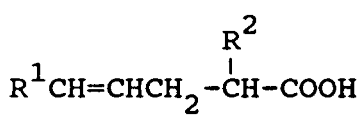

(V),

dans laquelle $R^1$ et $R^2$ ont les significations indiquées, à l'aide d'un agent d'halogénation, en l'halogénure d'acide correspondant, et on fait réagir celui-ci avec une amine ou hydroxylamine introduisant le radical $-NR^dR^e$.

**13.** Procédé selon la revendication 12, pour la préparation de composés de formule VIII dans lesquels $R^d$ représente un groupe alkyle inférieur et $R^e$ représente un groupe alkyle inférieur ou alcoxy inférieur, ou $R^d$ et $R^e$ représentent ensemble un groupe alkylène inférieur ou oxa-alkylène inférieur ayant de 4 à 6 atomes de carbone dans la chaîne, et d'énantiomères purs de ces composés.

**14.** Procédé pour la préparation de composés de formule

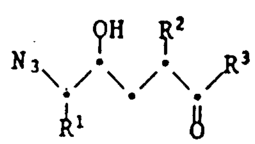

(IX)

dans laquelle $R^1$ représente un atome d'hydrogène, un groupe alkyle, cycloalkyle, cycloalkyl-alkyle inférieur, aryle, aryl-alkyle inférieur, éventuellement substitués, ou le reste d'un aminoacide naturel, $R^2$ représente un atome d'hydrogène, un groupe alkyle, cycloalkyle, cycloalkyl-alkyle inférieur, aryle, aryl-alkyle inférieur, amino, hydroxy, mercapto, sulfinyle, sulfonyle, éventuellement substitués, ou le reste d'un aminoacide naturel, et $R^3$ représente un groupe amino éventuellement substitué, et de sels de tels composés avec des groupes salifiants, caractérisé en ce que l'on ouvre le cycle lactone, à l'aide d'un composé introduisant le radical $R^3$, dans un composé de formule

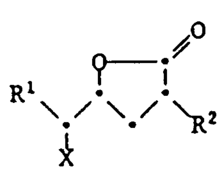

(VI)

dans laquelle X représente le groupe azido.